# EUROPÄISCHE PATENTSCHRIFT

(11) **EP 3 227 287 B1**
(45) Veröffentlichungstag und Bekanntmachung des Hinweises auf die Patenterteilung: **07.08.2019**
(21) Anmeldenummer: 15801455.5
(22) Anmeldetag: 30.11.2015
(51) Int. Cl.: C07D 471/04, A61K 31/437, A61P 7/02, A61P 9/02, A61P 9/04, A61P 9/10, A61P 9/12

(54) **HETEROARYL-SUBSTITUIERTE IMIDAZO[1,2-A]PYRIDINE UND IHRE VERWENDUNG**
HETEROARYL-SUBSTITUTED IMIDAZO[1,2-A]PYRIDINES AND THEIR USE
IMIDAZO[1,2-A]PYRIDINES À SUBSTITUTION HÉTÉROARYLE ET LEUR UTILISATION

(30) Priorität: 02.12.2014 EP 14195902
(43) Veröffentlichungstag der Anmeldung: 11.10.2017
(73) Patentinhaber: Bayer Pharma Aktiengesellschaft, 13353 Berlin (DE)
(72) Erfinder: VAKALOPOULOS, Alexandros, 40721 Hilden (DE); BROCKSCHNIEDER, Damian, 42781 Haan (DE); WUNDER, Frank, 42117 Wuppertal (DE); STASCH, Johannes-Peter, 00046 Grottaferrata (RM) (IT); MARQUARDT, Tobias, 42115 Wuppertal (DE); DIETZ, Lisa, 42111 Wuoppertal (DE); LI, Volkhart Min-Jian, 42553 Velbert (DE)
(74) Vertreter: BIP Patents
(86) Internationale Anmeldenummer: PCT/EP2015/078009
(87) Internationale Veröffentlichungsnummer: WO 2016/087343

(56) Entgegenhaltungen:
- WO-A1-2014/068099
- WO-A1-2014/195333

## Beschreibung

Die vorliegende Anmeldung betrifft neue Heteroaryl-substituierte Imidazo[1,2-a]pyridine, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von kardiovaskulären Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, an das Eisen-Zentralatom des Häms zu binden, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion, der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Myokardinfarkt, Thrombosen, Schlaganfall und sexueller Dysfunktion führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriff am Eisen-Zentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol [YC-1; Wu et al., Blood 84 (1994), 4226; Mülsch et al., Brit. J. Pharmacol. 120 (1997), 681], Fettsäuren [Goldberg et al., J. Biol. Chem. 252 (1977), 1279], Diphenyliodonium-hexafluorphosphat [Pettibone et al., Eur. J. Pharmacol. 116 (1985), 307], Isoliquiritigenin [Yu et al., Brit. J. Pharmacol. 114 (1995), 1587] sowie verschiedene substituierte Pyrazol-Derivate (WO 98/16223).

Unter anderem in EP 0 266 890-A1, WO 89/03833-A1, JP 01258674-A [vgl. Chem. Abstr. 112:178986], WO 96/34866-A1, EP 1 277 754-A1, WO 2001/096335, WO 2006/015737-A1, WO 2006/135667, WO 2008/008539-A2, WO 2008/082490-A2, WO 2008/134553-A1, WO 2010 /030538-A2, WO 2011/113606-A1 und WO 2012/165399-A1 sind verschiedene Imidazo[1,2-a]pyridin-Derivate beschrieben, die zur Behandlung von Erkrankungen verwendet werden können.

Aufgabe der vorliegenden Erfindung war die Bereitstellung neuer Substanzen, die als Stimulatoren der löslichen Guanylatcyclase wirken, und als solche zur Behandlung und/oder Prophylaxe von Krankheiten geeignet sind.

Gegenstand der vorliegenden Erfindung sind Verbindungen der allgemeinen Formel (I) 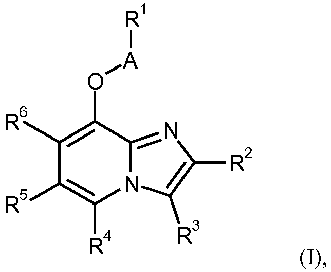 in welcher
- A: für CH₂ steht,
- R¹: für Cyclohexyl steht,
oder
für eine Phenyl-Gruppe der Formel 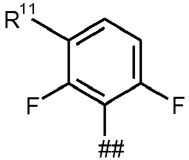 steht, wobei
## für die Anknüpfstelle an A steht, und
R¹¹ für Wasserstoff oder Fluor steht, oder
für eine Pyridyl-Gruppe der Formel 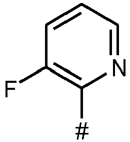 steht, wobei
# für die Anknüpfstelle an A steht,
- R²: für Methyl steht,
- R³: für 3-Pyridyl, 4-Pyridyl, 2-Pyrimidyl, 5-Pyrimidyl oder 1,3,5-Triazinyl steht,
wobei 3-Pyridyl in 4-Position mit -NR⁹R¹⁰ substituiert ist,
wobei 4-Pyridyl in 3-Position mit -NR⁹R¹⁰ substituiert ist,
wobei 2-Pyrimidyl in 4-Position mit -NR⁹R¹⁰ substituiert ist,
wobei 5-Pyrimidyl in 2-Position mit -NR⁹R¹⁰ substituiert ist,
worin jeweils
R⁹ für Wasserstoff oder Methyl steht,
R¹⁰ für (C₁-C₆)-Alkyl oder Phenyl steht,
worin (C₁-C₆)-Alkyl mit 1 oder 2 Substituenten unabhängig voneinander ausgewählt aus der Gruppe (C₁-C₄)-Alkoxy, Amino und Phenyl, sowie bis zu dreifach mit Fluor substituiert sein kann, oder
R⁹ und R¹⁰ zusammen mit dem Stickstoffatom, an das sie gebunden sind, einen Pyrrolidinyl-, Morpholinyl- oder Piperazinyl-Ring bilden, worin der Pyrrolidinyl-, Morpholinyl- oder Piperazinyl-Ring mit 1 oder 2 Substituenten Methyl substituiert sein kann, wobei 4-Pyridyl in 2-Position mit Fluor substituiert sein kann,
wobei 1,3,5-Triazinyl bis zu zweifach mit -NR^{9A}R^{10A} substituiert ist,
worin
R^{9A} für Wasserstoff oder Methyl steht,
R^{10A} für Wasserstoff, (C₁-C₆)-Alkyl oder Phenyl steht,
worin (C₁-C₆)-Alkyl mit Amino sowie bis zu dreifach mit Fluor substituiert sein kann, wobei 1,3,5-Triazinyl mit Chlor substituiert sein kann,
- R⁴: für Wasserstoff steht,
- R⁵: für Wasserstoff, Chlor oder Methyl steht,
- R⁶: für Wasserstoff steht,
sowie ihre *N*-Oxide, Salze, Solvate, Salze der *N*-Oxide und Solvate der *N*-Oxide und Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Außerdem beschreibt die vorliegende Offenbarung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit der jeweils angegebenen Anzahl an Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl, n-Pentyl, iso-Pentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 3,3-Dimethylbutyl, 1-Ethylbutyl, 2-Ethylbutyl.

Cycloalkyl bzw. Carbocyclus steht in Rahmen der Erfindung für einen monocyclischen, gesättigten Alkylrest mit der jeweils angegebenen Anzahl an Ring-Kohlenstoffatomen. Beispielhaft und vorzugsweise seien genannt: Cyclopropyl, Cyclobutyl, Cyclopentyl, Cyclohexyl und Cycloheptyl.

Alkoxy steht im Rahmen der Erfindung für einen linearen oder verzweigten Alkoxyrest mit 1 bis 4 Kohlenstoffatomen. Beispielhaft und vorzugsweise seien genannt: Methoxy, Ethoxy, n-Propoxy, Isopropoxy, 1-Methylpropoxy, n-Butoxy, iso-Butoxy und tert.-Butoxy.

Alkoxycarbonyl stehen im Rahmen der Erfindung für einen linearen oder verzweigten Alkoxyrest mit 1 bis 4 Kohlenstoffatomen und einer am Sauerstoffatom angebundenen Carbonylgruppe. Beispielhaft und vorzugsweise seien genannt: Methoxycarbonyl, Ethoxycarbonyl, n-Propoxycarbonyl, Isopropoxycarbonyl und tert.-Butoxycarbonyl.

Heterocyclus bzw Heterocyclyl steht im Rahmen der Erfindung für einen monocyclischen, gesättigten Heterocyclus mit insgesamt 4 bis 7 Ringatomen, der ein oder zwei Ring-Heteroatome aus der Reihe N, O, S, SO und/oder SO₂ enthält und über ein Ring-Kohlenstoffatom oder gegebenenfalls ein Ring-Stickstoffatom verknüpft ist. Beispielhaft seien genannt: Azetidinyl, Oxetanyl, Pyrrolidinyl, Pyrazolidinyl, Tetrahydrofuranyl, Thiolanyl, Piperidinyl, Piperazinyl, Tetrahydropyranyl, Tetrahydrothiopyranyl, Morpholinyl, Thiomorpholinyl, Hexahydroazepinyl und Hexahydro-1,4-diazepinyl. Bevorzugt sind Azetidinyl, Oxetanyl, Pyrrolidinyl, Tetrahydrofuranyl, Piperidinyl, Piperazinyl, Tetrahydropyranyl und Morpholinyl.

Heteroaryl steht im Rahmen der Erfindung für einen monocyclischen aromatischen Heterocyclus (Heteroaromaten) mit insgesamt 5 bis 10 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft und vorzugsweise seien genannt: Furyl, Pyrrolyl, Thienyl, 1H-Pyrazol-4-yl, 1H-Pyrazol-5-yl, Imidazolyl, 1,3-Thiazol-5-yl, 1,3-Thiazol-2-yl, 1,3-Oxazol-5-yl, 1,3-Oxazol-2-yl, Isoxazolyl, Isothiazolyl, Triazolyl, 1,3,4-Oxadiazol-2-yl, 1,2,4-Oxadiazol-3-yl, 1,2,4-Oxadiazol-5-yl, 1,3,4-Thiadiazol-2-yl, 1,2,4-Thiadiazol-3-yl, 1,2,4-Thiadiazol-5-yl, Pyridyl, Pyrimidinyl, Pyridazinyl, Pyrazinyl und Triazinyl.

Halogen schließt im Rahmen der Erfindung Fluor, Chlor, Brom und Iod ein. Bevorzugt sind Chlor oder Fluor.

In der Formel der Gruppe, für die R¹ stehen kann, steht der Endpunkt der Linie, an dem die Zeichen # oder ## stehen, nicht für ein Kohlenstoffatom beziehungsweise eine CH₂-Gruppe, sondern ist Bestandteil der Bindung zu dem jeweils bezeichneten Atom, an das R¹ gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Die vorliegende Erfindung offenbart auch Verbindungen der Formel (I), in welcher
- A: für CH₂ steht,
- R¹: für Cyclohexyl, Phenyl oder Pyridyl steht,
wobei Phenyl mit 1 bis 4 Substituenten unabhängig voneinander ausgewählt aus der Gruppe Fluor, Chlor, Cyano oder Methyl substituiert ist,
und
wobei Pyridyl mit 1 oder 2 Substituenten Fluor substituiert ist,
- R²: für Methyl, Cyclopropyl oder Trifluormethyl steht,
- R³: für Phenyl, 3-Pyridyl, 4-Pyridyl, 2-Pyrimidyl, 5-Pyrimidyl oder 1,3,5-Triazinyl steht,
wobei Phenyl in 3-Position mit -NR⁷R⁸ substituiert ist,
worin
R⁷ für Wasserstoff steht,
R⁸ für Methyl, Ethyl, Methylcarbonyl oder Ethylcarbonyl steht, oder
R⁷ und R⁸ zusammen mit dem Stickstoffatom, an das sie gebunden sind, einen 5-oder 6-gliedrigen Heterocyclus bilden, wobei Phenyl mit 1 oder 2 Substituenten unabhängig voneinander ausgewählt aus der Gruppe Fluor oder Chlor substituiert sein kann,
wobei 3-Pyridyl, 4-Pyridyl, 2-Pyrimidyl und 5-Pyrimidyl mit -NR⁹R¹⁰ substituiert sind,
worin
R⁹ für Wasserstoff oder Methyl steht,
R¹⁰ für (C₁-C₆)-Alkyl oder Phenyl steht,
worin (C₁-C₆)-Alkyl mit 1 oder 2 Substituenten unabhängig voneinander ausgewählt aus der Gruppe (C₁-C₄)-Alkoxy, Amino und Phenyl, sowie bis zu dreifach mit Fluor substituiert sein kann, oder
R⁹ und R¹⁰ zusammen mit dem Stickstoffatom, an das sie gebunden sind, einen 5- oder 6-gliedrigen Heterocyclus bilden,
worin der 5- oder 6-gliedrige Heterocyclus mit 1 oder 2 Substituenten Methyl substituiert sein kann, wobei 3-Pyridyl, 4-Pyridyl, 2-Pyrimidyl und 5-Pyrimidyl mit 1 oder 2 Substituenten unabhängig voneinander ausgewählt aus der Gruppe Fluor, Chlor oder Methyl substituiert sein können,
wobei 1,3,5-Triazinyl bis zu zweifach mit -NR^{9A}R^{10A} substituiert ist,
worin
R^{9A} für Wasserstoff oder Methyl steht,
R^{10A} für Wasserstoff, (C₁-C₆)-Alkyl oder Phenyl steht,
worin (C₁-C₆)-Alkyl mit Amino sowie bis zu dreifach mit Fluor substituiert sein kann, oder
R^{9A} und R^{10A} zusammen mit dem Stickstoffatom, an das sie gebunden sind, einen 5- oder 6-gliedrigen Heterocyclus bilden,
worin der 5- oder 6-gliedrige Heterocyclus mit 1 oder 2 Substituenten Methyl substituiert sein kann, wobei 1,3,5-Triazinyl mit Chlor substituiert sein kann,
- R⁴: für Wasserstoff steht,
- R⁵: für Wasserstoff, Chlor, Methyl oder Cyclopropyl steht,
- R⁶: für Wasserstoff steht,
sowie ihre *N*-Oxide, Salze, Solvate, Salze der *N*-Oxide und Solvate der *N*-Oxide und Salze.

Mit Ausnahme der Verbindungen
*N*-(3-{8-[(2,6-Difluorbenzyl)oxy]-2-methylimidazo[1,2-a]pyridin-3-yl}phenyl)acetamid,
8-[(2,6-Difluorbenzyl)oxy]-2-methyl-3-[3-(piperidin-1-yl)phenyl]imidazo[1,2-a]pyridin.

Die vorliegende Erfindung offenbart auchVerbindungen der Formel (I), in welcher
- A: für CH₂ steht,
- R¹: für Cyclohexyl, Phenyl oder Pyridyl steht,
wobei Phenyl mit 1 bis 3 Substituenten Fluor substituiert ist,
wobei Pyridyl mit 1 Substituenten Fluor substituiert ist,
- R²: für Methyl steht,
- R³: für Phenyl, 3-Pyridyl, 4-Pyridyl, 2-Pyrimidyl, 5-Pyrimidyl oder 1,3,5-Triazinyl steht, wobei Phenyl in 3-Position mit -NR⁷R⁸ substituiert ist,
worin
R⁷ für Wasserstoff steht,
R⁸ für Methyl, Ethyl, Methylcarbonyl oder Ethylcarbonyl steht, oder
R⁷ und R⁸ zusammen mit dem Stickstoffatom, an das sie gebunden sind, einen 5-oder 6-gliedrigen Heterocyclus bilden, wobei 3-Pyridyl, 4-Pyridyl, 2-Pyrimidyl und 5-Pyrimidyl mit -NR⁹R¹⁰ substituiert sind,
worin
R⁹ für Wasserstoff oder Methyl steht,
R¹⁰ für (C₁-C₆)-Alkyl oder Phenyl steht,
worin (C₁-C₆)-Alkyl mit 1 oder 2 Substituenten unabhängig voneinander ausgewählt aus der Gruppe (C₁-C₄)-Alkoxy, Amino und Phenyl, sowie bis zu dreifach mit Fluor substituiert sein kann, oder
R⁹ und R¹⁰ zusammen mit dem Stickstoffatom, an das sie gebunden sind, einen 5- oder 6-gliedrigen Heterocyclus bilden,
worin der 5- oder 6-gliedrige Heterocyclus mit 1 oder 2 Substituenten Methyl substituiert sein kann, wobei 3-Pyridyl, 4-Pyridyl, 2-Pyrimidyl und 5-Pyrimidyl mit 1 oder 2 Substituenten unabhängig voneinander ausgewählt aus der Gruppe Fluor, Chlor oder Methyl substituiert sein können,
wobei 1,3,5-Triazinyl bis zu zweifach mit -NR^{9A}R^{10A} substituiert ist,
worin
R^{9A} für Wasserstoff oder Methyl steht,
R^{10A} für Wasserstoff, (C₁-C₆)-Alkyl oder Phenyl steht,
worin (C₁-C₆)-Alkyl mit Amino sowie bis zu dreifach mit Fluor substituiert sein kann, wobei 1,3,5-Triazinyl mit Chlor substituiert sein kann,
- R⁴: für Wasserstoff steht,
- R⁵: für Wasserstoff, Chlor, Methyl oder Cyclopropyl steht,
- R⁶: für Wasserstoff steht,
sowie ihre *N*-Oxide, Salze, Solvate, Salze der *N*-Oxide und Solvate der *N*-Oxide und Salze.

Mit Ausnahme der Verbindungen

Die vorliegende Erfindung offenbart auchVerbindungen der Formel (I), in welcher
- A: für CH₂ steht,
- R¹: für Cyclohexyl, Phenyl oder Pyridyl steht,
wobei Phenyl mit 1 bis 3 Substituenten Fluor substituiert ist,
- R²: für Methyl steht,
- R³: für Phenyl, 3-Pyridyl, 4-Pyridyl, 2-Pyrimidyl, 5-Pyrimidyl oder 1,3,5-Triazinyl steht,
wobei Phenyl in 3-Position mit -NR⁷R⁸ substituiert ist,
worin
R⁷ für Wasserstoff steht,
R⁸ für Methyl, Ethyl, Methylcarbonyl oder Ethylcarbonyl steht, oder
R⁷ und R⁸ zusammen mit dem Stickstoffatom, an das sie gebunden sind, einen 5-oder 6-gliedrigen Heterocyclus bilden, wobei 3-Pyridyl, 4-Pyridyl, 2-Pyrimidyl und 5-Pyrimidyl mit -NR⁹R¹⁰ substituiert sind,
worin
R⁹ für Wasserstoff oder Methyl steht,
R¹⁰ für (C₁-C₆)-Alkyl oder Phenyl steht, worin (C₁-C₆)-Alkyl mit 1 oder 2 Substituenten unabhängig voneinander ausgewählt aus der Gruppe (C₁-C₄)-Alkoxy, Amino und Phenyl, sowie bis zu dreifach mit Fluor substituiert sein kann,
oder
R⁹ und R¹⁰ zusammen mit dem Stickstoffatom, an das sie gebunden sind, einen 5- oder 6-gliedrigen Heterocyclus bilden,
worin der 5- oder 6-gliedrige Heterocyclus mit 1 oder 2 Substituenten Methyl substituiert sein kann, wobei 3-Pyridyl, 4-Pyridyl, 2-Pyrimidyl und 5-Pyrimidyl mit 1 oder 2 Substituenten unabhängig voneinander ausgewählt aus der Gruppe Fluor, Chlor oder Methyl substituiert sein können,
wobei 1,3,5-Triazinyl bis zu zweifach mit -NR^{9A}R^{10A} substituiert ist,
worin
R^{9A} für Wasserstoff oder Methyl steht,
R^{10A} für Wasserstoff, (C₁-C₆)-Alkyl oder Phenyl steht,
worin (C₁-C₆)-Alkyl mit Amino sowie bis zu dreifach mit Fluor substituiert sein kann, wobei 1,3,5-Triazinyl mit Chlor substituiert sein kann,
- R⁴: für Wasserstoff steht,
- R⁵: für Wasserstoff, Chlor, Methyl oder Cyclopropyl steht,
- R⁶: für Wasserstoff steht,
sowie ihre *N*-Oxide, Salze, Solvate, Salze der *N*-Oxide und Solvate der *N*-Oxide und Salze.

Die vorliegende Erfindung offenbart auchVerbindungen der Formel (I), in welcher
- A: für CH₂ steht,
- R¹: für Cyclohexyl steht, oder
für eine Phenyl-Gruppe der Formel 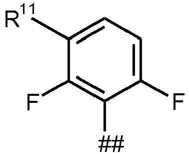 steht, wobei
## für die Anknüpfstelle an A steht, und
R¹¹ für Wasserstoff oder Fluor steht, oder
für eine Pyridyl-Gruppe der Formel 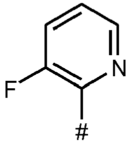 steht, wobei
# für die Anknüpfstelle an A steht,
R² für Methyl steht,
R³ für 3-Pyridyl, 4-Pyridyl, 2-Pyrimidyl, 5-Pyrimidyl oder 1,3,5-Triazinyl steht,
wobei 3-Pyridyl in 4-Position mit -NR⁹R¹⁰ substituiert ist,
wobei 4-Pyridyl in 3-Position mit -NR⁹R¹⁰ substituiert ist,
wobei 2-Pyrimidyl in 4-Position mit -NR⁹R¹⁰ substituiert ist,
wobei 5-Pyrimidyl in 2-Position mit -NR⁹R¹⁰ substituiert ist,
worin jeweils
R⁹ für Wasserstoff oder Methyl steht,
R¹⁰ für (C₁-C₆)-Alkyl oder Phenyl steht,
worin (C₁-C₆)-Alkyl mit 1 oder 2 Substituenten unabhängig voneinander ausgewählt aus der Gruppe (C₁-C₄)-Alkoxy, Amino und Phenyl, sowie bis zu dreifach mit Fluor substituiert sein kann, oder
R⁹ und R¹⁰ zusammen mit dem Stickstoffatom, an das sie gebunden sind, einen Pyrrolidinyl-, Morpholinyl- oder Piperazinyl-Ring bilden,
worin der Pyrrolidinyl-, Morpholinyl- oder Piperazinyl-Ring mit 1 oder 2 Substituenten Methyl substituiert sein kann, wobei 4-Pyridyl in 2-Position mit Fluor substituiert sein kann,
wobei 1,3,5-Triazinyl bis zu zweifach mit -NR^{9A}R^{10A} substituiert ist,
worin
R^{9A} für Wasserstoff oder Methyl steht,
R^{10A} für Wasserstoff, (C₁-C₆)-Alkyl oder Phenyl steht,
worin (C₁-C₆)-Alkyl mit Amino sowie bis zu dreifach mit Fluor substituiert sein kann,
wobei 1,3,5-Triazinyl mit Chlor substituiert sein kann,
- R⁴: für Wasserstoff steht,
- R⁵: für Wasserstoff, Chlor oder Methyl steht,
- R⁶: für Wasserstoff steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R¹: für eine Phenyl-Gruppe der Formel 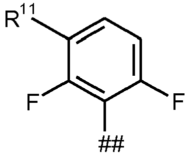 steht, wobei
## für die Anknüpfstelle an A steht, und
R¹¹ für Wasserstoff oder Fluor steht, sowie ihre *N*-Oxide, Salze, Solvate, Salze der *N*-Oxide und Solvate der *N*-Oxide und Salze. Bevorzugt sind im Rahmen der vorliegenden Erfindung auch Verbindungen der Formel (I), in welcher
- R¹: für eine Pyridyl-Gruppe der Formel 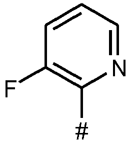 steht, wobei
# für die Anknüpfstelle an A steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R²: für Methyl steht,
sowie ihre *N*-Oxide, Salze, Solvate, Salze der *N*-Oxide und Solvate der *N*-Oxide und Salze.

Die vorliegende Erfindung offenbart auch Verbindungen der Formel (I), in
welcher
- R³: für Phenyl steht,
wobei Phenyl in 3-Position mit -NR⁷R⁸ substituiert ist,
worin
R⁷ für Wasserstoff steht,
R⁸ für Methylcarbonyl steht,
oder
R⁷ und R⁸ zusammen mit dem Stickstoffatom, an das sie gebunden sind, einen Pyrrolidinyl-Ring bilden,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in
welcher
- R³: für 3-Pyridyl, 4-Pyridyl, 2-Pyrimidyl, 5-Pyrimidyl oder 1,3,5-Triazinyl steht,
wobei 3-Pyridyl in 4-Position mit -NR⁹R¹⁰ substituiert ist,
wobei 4-Pyridyl in 3-Position mit -NR⁹R¹⁰ substituiert ist,
wobei 2-Pyrimidyl in 4-Position mit -NR⁹R¹⁰ substituiert ist,
wobei 5-Pyrimidyl in 2-Position mit -NR⁹R¹⁰ substituiert ist,
worin jeweils
R⁹ für Wasserstoff oder Methyl steht,
R¹⁰ für (C₁-C₆)-Alkyl oder Phenyl steht,
worin (C₁-C₆)-Alkyl mit 1 oder 2 Substituenten unabhängig voneinander ausgewählt aus der Gruppe (C₁-C₄)-Alkoxy, Amino und Phenyl, sowie bis zu dreifach mit Fluor substituiert sein kann,
oder
R⁹ und R¹⁰ zusammen mit dem Stickstoffatom, an das sie gebunden sind, einen Pyrrolidinyl-, Morpholinyl- oder Piperazinyl-Ring bilden,
worin der Pyrrolidinyl-, Morpholinyl- oder Piperazinyl-Ring mit 1 oder 2 Substituenten Methyl substituiert sein kann,
wobei 4-Pyridyl in 2-Position mit Fluor substituiert sein kann,
wobei 1,3,5-Triazinyl bis zu zweifach mit -NR^{9A}R^{10A} substituiert ist,
worin
R^{9A} für Wasserstoff oder Methyl steht,
R^{10A} für Wasserstoff, (C₁-C₆)-Alkyl oder Phenyl steht,
worin (C₁-C₆)-Alkyl mit Amino sowie bis zu dreifach mit Fluor substituiert sein kann, wobei 1,3,5-Triazinyl mit Chlor substituiert sein kann,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R³: für 3-Pyridyl steht,
wobei 3-Pyridyl in 4-Position mit -NR⁹R¹⁰ substituiert ist,
worin
R⁹ für Wasserstoff oder Methyl steht,
R¹⁰ für (C₁-C₆)-Alkyl oder Phenyl steht,
worin (C₁-C₆)-Alkyl mit 1 oder 2 Substituenten unabhängig voneinander ausgewählt aus der Gruppe (C₁-C₄)-Alkoxy, Amino und Phenyl, sowie bis zu dreifach mit Fluor substituiert sein kann,
oder
R⁹ und R¹⁰ zusammen mit dem Stickstoffatom, an das sie gebunden sind, einen Pyrrolidinyl-, Morpholinyl- oder Piperazinyl-Ring bilden,
worin der Pyrrolidinyl-, Morpholinyl- oder Piperazinyl-Ring mit 1 oder 2 Substituenten Methyl substituiert sein kann,
sowie ihre *N*-Oxide, Salze, Solvate, Salze der *N*-Oxide und Solvate der *N*-Oxide und Salze.

Besonders bevorzugt sind im Rahmen der vorliegenden Erfindung auch Verbindungen der Formel (I), in
welcher
- R³: für 3-Pyridyl steht,
wobei 3-Pyridyl in 4-Position mit -NR⁹R¹⁰ substituiert ist,
worin
R⁹ für Wasserstoff oder Methyl steht,
R¹⁰ für Methyl, Ethyl oder Phenyl steht,
worin Methyl und Ethyl mit (C₁-C₄)-Alkoxy, Amino und Phenyl substituiert sein können,
oder
R⁹ und R¹⁰ zusammen mit dem Stickstoffatom, an das sie gebunden sind, einen Morpholinyl- oder Piperazinyl-Ring bilden,
worin der Morpholinyl- oder Piperazinyl-Ring mit 1 oder 2 Substituenten Methyl substituiert sein kann,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R³: für 4-Pyridyl steht,
wobei 4-Pyridyl in 3-Position mit -NR⁹R¹⁰ substituiert ist,
worin
R⁹ für Wasserstoff oder Methyl steht,
R¹⁰ für (C₁-C₆)-Alkyl oder Phenyl steht,
worin (C₁-C₆)-Alkyl mit 1 oder 2 Substituenten unabhängig voneinander ausgewählt aus der Gruppe (C₁-C₄)-Alkoxy, Amino und Phenyl, sowie bis zu dreifach mit Fluor substituiert sein kann,
oder
- R⁹ und R¹⁰: zusammen mit dem Stickstoffatom, an das sie gebunden sind, einen Pyrrolidinyl-, Morpholinyl- oder Piperazinyl-Ring bilden,
worin der Pyrrolidinyl-, Morpholinyl- oder Piperazinyl-Ring mit 1 oder 2 Substituenten Methyl substituiert sein kann,
wobei 4-Pyridyl in 2-Position mit Fluor substituiert sein kann,
sowie ihre *N*-Oxide, Salze, Solvate, Salze der *N*-Oxide und Solvate der *N*-Oxide und Salze.

Besonders bevorzugt sind im Rahmen der vorliegenden Erfindung auch Verbindungen der Formel (I), in
welcher
- R³: für 4-Pyridyl steht,
wobei 4-Pyridyl in 3-Position mit -NR⁹R¹⁰ substituiert ist,
worin
R⁹ für Wasserstoff steht,
R¹⁰ für (C₁-C₆)-Alkyl steht,
worin (C₁-C₆)-Alkyl mit Amino substituiert ist,
oder
- R⁹ und R¹⁰: zusammen mit dem Stickstoffatom, an das sie gebunden sind, einen Piperazinyl-Ring bilden,
wobei 4-Pyridyl in 2-Position mit Fluor substituiert sein kann,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R³: für 2-Pyrimidyl steht,
wobei 2-Pyrimidyl in 4-Position mit -NR⁹R¹⁰ substituiert ist,
worin
R⁹ für Wasserstoff oder Methyl steht,
R¹⁰ für (C₁-C₆)-Alkyl oder Phenyl steht,
worin (C₁-C₆)-Alkyl mit 1 oder 2 Substituenten unabhängig voneinander ausgewählt aus der Gruppe (C₁-C₄)-Alkoxy, Amino und Phenyl, sowie bis zu dreifach mit Fluor substituiert sein kann,
oder
R⁹ und R¹⁰ zusammen mit dem Stickstoffatom, an das sie gebunden sind, einen Pyrrolidinyl-, Morpholinyl- oder Piperazinyl-Ring bilden,
worin der Pyrrolidinyl-, Morpholinyl- oder Piperazinyl-Ring mit 1 oder 2 Substituenten Methyl substituiert sein kann,
sowie ihre *N*-Oxide, Salze, Solvate, Salze der *N*-Oxide und Solvate der *N*-Oxide und Salze.

Besonders bevorzugt sind im Rahmen der vorliegenden Erfindung auch Verbindungen der Formel (I), in
welcher
- R³: für 2-Pyrimidyl steht,
wobei 2-Pyrimidyl in 4-Position mit -NR⁹R¹⁰ substituiert ist,
worin
R⁹ für Wasserstoff oder Methyl steht,
R¹⁰ für Methyl oder Phenyl steht,
worin Methyl mit Phenyl substituiert sein kann,
oder
R⁹ und R¹⁰ zusammen mit dem Stickstoffatom, an das sie gebunden sind, einen Pyrrolidinyl-Ring bilden,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R³: für 1,3,5-Triazinyl steht,
wobei 1,3,5-Triazinyl bis zu zweifach mit -NR^{9A}R^{10A} substituiert ist,
worin
R^{9A} für Wasserstoff oder Methyl steht,
R^{10A} für Wasserstoff, (C₁-C₆)-Alkyl oder Phenyl steht,
worin (C₁-C₆)-Alkyl mit Amino sowie bis zu dreifach mit Fluor substituiert sein kann,
wobei 1,3,5-Triazinyl mit Chlor substituiert sein kann,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R⁵: für Wasserstoff, Chlor oder Methyl steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- R⁵: für Chlor steht,
sowie ihre *N*-Oxide, Salze, Solvate, Salze der *N*-Oxide und Solvate der *N*-Oxide und Salze.

Besonders bevorzugt sind im Rahmen der vorliegenden Erfindung auch Verbindungen der Formel (I), in
welcher
- R⁵: für Wasserstoff oder Methyl steht,
sowie ihre *N*-Oxide, Salze, Solvate, Salze der *N*-Oxide und Solvate der *N*-Oxide und Salze.

Besonders bevorzugt sind im Rahmen der vorliegenden Erfindung auch Verbindungen der Formel (I), in
welcher
- R⁵: für Wasserstoff steht,
sowie ihre *N*-Oxide, Salze, Solvate, Salze der *N*-Oxide und Solvate der *N*-Oxide und Salze.

Besonders bevorzugt sind im Rahmen der vorliegenden Erfindung auch Verbindungen der Formel (I), in
welcher
- R⁵: für Methyl steht,
sowie ihre *N*-Oxide, Salze, Solvate, Salze der *N*-Oxide und Solvate der *N*-Oxide und Salze.

Die in den jeweiligen Kombinationen bzw. bevorzugten Kombinationen von Resten im Einzelnen angegebenen Reste-Definitionen werden unabhängig von den jeweiligen angegebenen Kombinationen der Reste beliebig auch durch Reste-Definitionen anderer Kombinationen ersetzt.

Besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I) dadurch gekennzeichnet, dass man
[A] eine Verbindung der Formel (II) 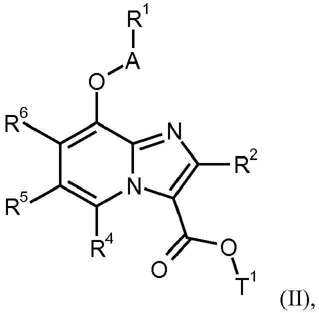 in welcher A, R¹, R², R⁴, R⁵ und R⁶ jeweils die oben angegebenen Bedeutungen haben und
   - T¹: für (C₁-C₄)-Alkyl oder Benzyl steht,
   in einem inerten Lösungsmittel in Gegenwart einer geeigneten Base oder Säure zu einer Carbonsäure der Formel (III) 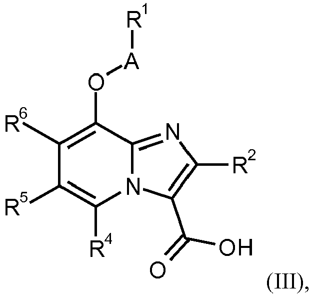 in welcher A, R¹, R², R⁴, R⁵ und R⁶ jeweils die oben angegebenen Bedeutungen haben,
   umsetzt,
   und diese in der Folge in Gegenwart einer geeigneten Säure zu einem Imidazo[1,2-a]-pyridin der Formel (IV) 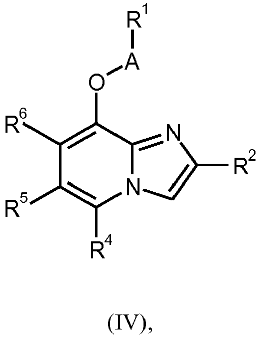 in welcher A, R¹, R², R⁴, R⁵ und R⁶ jeweils die oben angegebenen Bedeutungen haben,
   umsetzt, und dieses dann mit einem Halogen-Äquivalent in eine Verbindung der Formel (V) 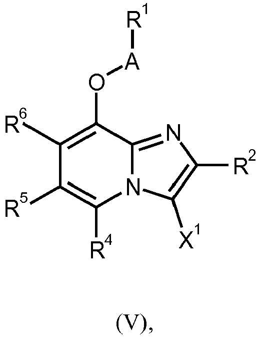 in welcher A, R¹, R², R⁴, R⁵ und R⁶ jeweils die oben angegebenen Bedeutungen haben und
   - X¹: für Chlor, Brom oder Iod steht,
   überführt, und diese im Anschluß in einem inerten Lösungsmittel in Gegenwart eines geeigneten Übergangsmetallkatalysators mit einer Verbindung der Formel (VI), 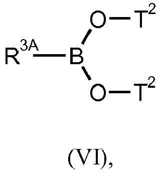 in welcher
   R^{3A} die oben für R³ angegebenen Bedeutungen hat und
   - T²: für Wasserstoff oder (C₁-C₄)-Alkyl steht, oder beide Reste T² zusammen eine -C(CH₃)₂-C(CH₃)₂-Brücke bilden,
umsetzt, anschliessend gegebenenfalls vorhandene Schutzgruppen abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt,

Die beschriebenen Herstellverfahren können durch das folgende Syntheseschema (Schema 1) beispielhaft verdeutlicht werden: 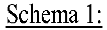 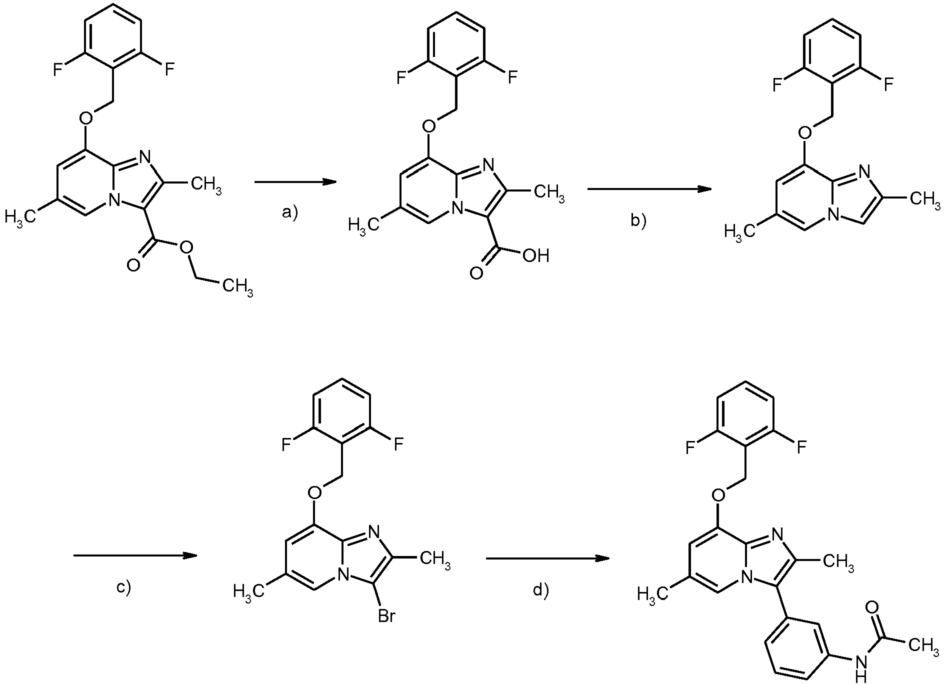
[a): Lithiumhydroxid, THF/Methanol/ H₂O, RT; b): 6 N Salzsäure, 100°C; c): *N*-Bromsuccinimid, Ethanol, RT; d): (3-Acetamidophenyl)borsäure, Bis(tri-tert.-butyl-phosphin)palladium(0), K₃PO₄, Ethanol/Wasser/Toluol, 120°C].

Die Verbindungen der Formeln (VI), (VIII) und (X) sind kommerziell erhältlich, literaturbekannt oder können in Analogie zu literaturbekannten Verfahren hergestellt werden.

Die Hydrolyse der Ester-Gruppe T¹ der Verbindungen der Formel (II) erfolgt nach üblichen Methoden, indem man die Ester in inerten Lösungsmitteln mit Säuren oder Basen behandelt, wobei bei letzterem die zunächst entstehenden Salze durch Behandeln mit Säure in die freien Carbonsäuren überführt werden. Im Falle der tert.-Butylester erfolgt die Esterspaltung bevorzugt mit Säuren. Im Falle der Benzylester erfolgt die Esterspaltung bevorzugt hydrogenolytisch mit Palladium auf Aktivkohle oder Raney-Nickel. Als inerte Lösungsmittel eignen sich für diese Reaktion Wasser oder die für eine Esterspaltung üblichen organischen Lösungsmittel. Hierzu gehören bevorzugt Alkohole wie Methanol, Ethanol, n-Propanol, Isopropanol, n-Butanol oder tert.-Butanol, oder Ether wie Diethylether, Tetrahydrofuran, 2-Methyltetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemische der genannten Lösungsmittel einzusetzen.

Im Falle einer basischen Ester-Hydrolyse werden bevorzugt Gemische von Wasser mit Dioxan, Tetrahydrofuran, Methanol und/oder Ethanol eingesetzt.

Als Basen für die Ester-Hydrolyse sind die üblichen anorganischen Basen geeignet. Hierzu gehören bevorzugt Alkali- oder Erdalkalihydroxide wie beispielsweise Natrium-, Lithium-, Kalium- oder Bariumhydroxid, oder Alkali- oder Erdalkalicarbonate wie Natrium-, Kalium- oder Calciumcarbonat. Besonders bevorzugt sind Natrium- oder Lithiumhydroxid.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der tert.-Butylester und Salzsäure im Falle der Methylester.

Die Esterspaltung erfolgt im Allgemeinen in einem Temperaturbereich von 0°C bis +100°C, bevorzugt bei +0°C bis +50°C.

Die genannten Umsetzungen können bei normalem, erhöhtem oder bei erniedrigtem Druck durchgeführt werden (z.B. von 0.5 bis 5 bar). Im Allgemeinen arbeitet man jeweils bei Normaldruck.

Geeignetes Lösungsmittel für den Verfahrensschritt (III) → (IV) ist Wasser.

Geeignete Säuren für den Verfahrensschritt (III) → (IV) sind Chlorwasserstoff/ Salzsäure, Bromwasserstoff/Bromwasserstoffsäure, Schwefelsäure, Essigsäure oder deren Gemische gegebenenfalls unter Zusatz von Wasser. Bevorzugt wird Salzsäure verwendet.

Die Decarboxylierung (III) → (IV) wird im Allgemeinen in einem Temperaturbereich von +20°C bis +100°C, bevorzugt bei 75°C bis +100°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Als Lösungsmittel eignen sich für den Verfahrensschritt (IV) → (V) Alkohole wie Methanol, Ethanol, n-Propanol, Isopropanol, n-Butanol oder tert.-Butanol, oder Ether wie Diethylether, Tetrahydrofuran, 2-Methyltetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemische der genannten Lösungsmittel einzusetzen. Bevorzugt werden Methanol und/oder Ethanol eingesetzt.

Als Halogen-Quelle bei der Umsetzung (IV) → (V) eignen sich beispielsweise *N*-Bromsuccinimid, *N*-Chlorsuccinimid, *N*-Iod-succinimid, Chlor, Brom oder Iod. Bevorzugt wird *N*-Bromsuccinimid verwendet.

Die Reaktion (IV) → (V) erfolgt im Allgemeinen in einem Temperaturbereich von +20°C bis +100°C, bevorzugt im Bereich von +20°C bis +80°C. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Der Verfahrensschritt (V) + (VI) → (I) erfolgt in einem unter den Reaktionsbedingungen inerten Lösungmittel. Geeignete Lösungsmittel sind beispielsweise Alkohole wie Methanol, Ethanol, n-Propanol, Isopropanol, n-Butanol oder tert.-Butanol, Ether wie Diethylether, Dioxan, Tetrahydrofuran, Glykoldimethylether oder Diethylenglykoldimethylether, oder andere Lösungsmitteln wie 1,2-Dimethoxyethan (DME), Dimethylformamid (DMF), Dimethylsulfoxid (DMSO), *N,N'-*Dimethylpropylenharnstoff (DMPU), *N*-Methylpyrrolidon (NMP), Pyridin, Acetonitril, Toluol oder auch Wasser. Ebenso ist es möglich, Gemische der genannten Lösungsmittel einzusetzen. Bevorzugt sind Methanol, Ethanol, Dioxan, Toluol und Wasser.

Gegebenenfalls kann die Umsetzung (V) + (VI) → (I) in Gegenwart eines geeigneten Palladium- und/oder Kupferkatalysators erfolgen. Als Palladium-Katalysator ist beispielsweise Palladium(II)-acetat, Tetrakis-(triphenylphosphin)-palladium(0), Bis(tri-tert.-butyl-phosphin)palladium(0), Bis-(triphenylphosphin)-palladium(II)-chlorid, Bis-(acetonitril)-palladium(II)-chlorid, [1,1'-Bis(diphenylphosphino)ferrocen]dichlorpalladium(II) und entsprechender Dichlormethan-Komplex, gegebenenfalls in Verbindung mit zusätzlichen Phosphanliganden wie beispielsweise (2-Biphenyl)di-tert. -butylphosphin, 2-Dicyclohexylphosphino-2',6'-dimethoxybiphenyl (SPHOS) Dicyclohexyl[2',4',6'-tris(1-methylethyl)biphenyl-2-yl]phosphan (XPHOS), Bis(2-phenylphosphinophenyl)ether (DPEphos) or 4,5-Bis(diphenylphosphino)-9,9-dimethylxanthen (Xantphos) [vgl. z.B. Hassan J. et al., Chem. Rev. 102, 1359-1469 (2002)] oder Chloro(2-dicyclohexylphosphino-2',4',6'-triisopropyl-1,1'-biphenyl)[2-(2'-amino-1,1'-biphenyl)palladium (II) [CAS: 1310584-14-5] geeignet.

Die Umsetzung (V) + (VI) → (I) erfolgt gegenbenenfalls in Gegenwart einer geeigneten Base. Geeignete Basen für diese Umsetzung sind die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium-, Natrium- oder Kalium-bis(trimethylsilyl)amid oder Lithiumdiisopropylamid, oder organische Amine wie Triethylamin, *N*-Methylmorpholin, *N-*Methylpiperidin, *N,N*-Diisopropylethylamin, Pyridin, 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO®) oder Kaliumphosphat. Bevorzugt wird Kaliumphosphat verwendet.

Die Reaktion (V) + (VI) → (I) wird im Allgemeinen in einem Temperaturbereich von 0°C bis +200°C, bevorzugt bei +60°C bis +120°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Die Verbindungen der Formel (II) sind literaturbekannt oder können hergestellt werden, indem eine Verbindung der Formel (VII) 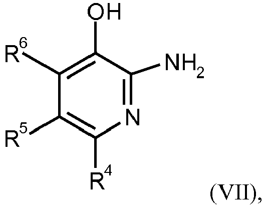 in welcher R⁴, R⁵ und R⁶ die oben angegebene Bedeutung hat,
in einem inerten Lösungsmittel in Gegenwart einer geeigneten Base mit einer Verbindung der Formel (VIII) 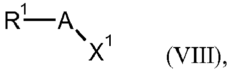 in welcher A und R¹ die oben angegebene Bedeutung hat und
- X¹: für eine geeignete Abgangsgruppe, insbesondere Chlor, Brom, Iod, Mesylat, Triflat oder Tosylat, steht,
zu einer Verbindung der Formel (IX) 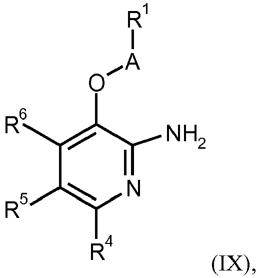 in welcher A, R¹, R⁴, R⁵ und R⁶ jeweils die oben angegebenen Bedeutungen haben,
umgesetzt wird, und diese anschliessend in einem inerten Lösungsmittel mit einer Verbindung der Formel (X) 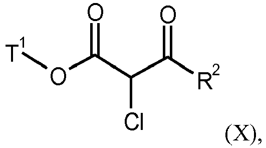 in welcher R² und T¹ jeweils die oben angegebenen Bedeutungen haben,
umgesetzt wird.

Das beschriebene Verfahren wird durch das nachfolgende Schema (Schema 2) beispielhaft verdeutlicht: 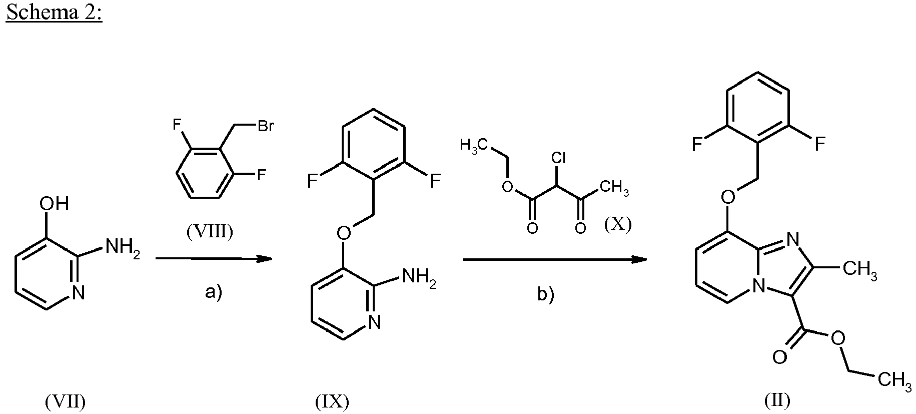
[a): i) NaOMe, MeOH, RT; ii) DMSO, RT; b): EtOH, Molekularsieb, Rückfluss].

Die gezeigte Synthesesequenz kann dahingehend modifiziert werden, dass die jeweiligen Reaktionsschritte in einer veränderten Reihenfolge durchlaufen werden. Ein Beispiel für eine solche modifizierte Synthesesequenz ist in Schema 3 gezeigt. 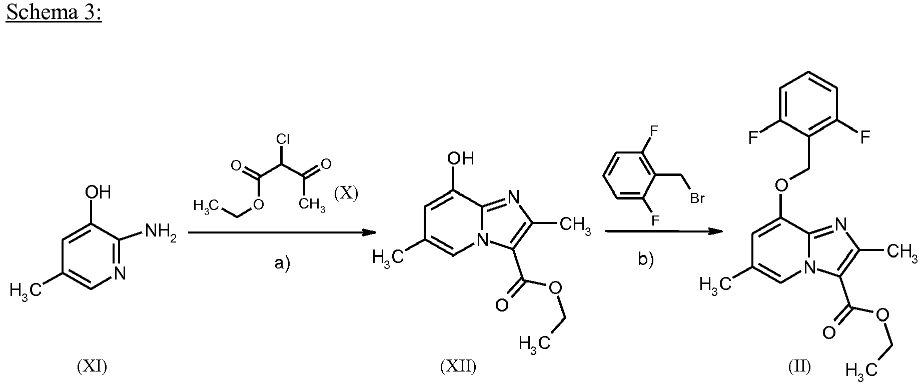
[a): EtOH, Molekularsieb, Rückfluss; b): b) Cs₂CO₃, DMF, 50°C].

Inerte Lösungsmittel für den Verfahrensschritt (VII) + (VIII) → (IX) sind beispielsweise Halogenkohlenwasserstoffe wie Dichlormethan, Trichlormethan, Tetrachlormethan, Trichlorethylen oder Chlorbenzol, Ether wie Diethylether, Dioxan, Tetrahydrofuran, Glykoldimethylether oder Diethylenglykoldimethylether, Kohlenwasserstoffe wie Benzol, Toluol, Xylol, Hexan, Cyclohexan oder Erdölfraktionen, Alkohole wie Methanol, Ethanol, *tert*-Butanol, oder andere Lösungsmittel wie Aceton, Methylethylketon, Essigsäureethylester, Acetonitril, *N,N-*Dimethylformamid, Dimethylsulfoxid, *N,N*'-Dimethylpropylenharnstoff (DMPU), *N*-Methylpyrrolidon (NMP) oder Pyridin. Ebenso ist es möglich, Gemische der genannten Lösungsmittel einzusetzen. Bevorzugt wird Methanol, Dimethylformamid oder Dimethylsulfoxid verwendet.

Als Basen für den Verfahrensschritt (VII) + (VIII) → (IX) eignen sich die üblichen anorganischen oder organischen Basen. Hierzu gehören bevorzugt Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat gegebenenfalls unter Zusatz eines Alkaliiodids wie beispielsweise Natriumiodid oder Kaliumiodid, Alkali-Alkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium- oder Kalium-bis(trimethylsilyl)-amid oder Lithiumdiisopropylamid, oder organische Amine wie Triethylamin, *N*-Methylmorpholin, *N*-Methylpiperidin, *N,N*-Diisopropylethylamin, Pyridin, 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO®). Bevorzugt wird Kaliumcarbonat, Cäsiumcarbonat oder Natriummethanolat verwendet.

Die Reaktion erfolgt im Allgemeinen in einem Temperaturbereich von 0°C bis +120°C, bevorzugt bei +20°C bis +80°C, gegebenenfalls in einer Mikrowelle. Die Umsetzung kann bei normalem, erhöhtem oder bei erniedrigtem Druck durchgeführt werden (z.B. von 0.5 bis 5 bar).

Inerte Lösungsmittel für den Ringschluss zum Imidazo[1,2-a]pyridin-Grundgerüst (IX) + (X) → (II) bzw. (XI) + (X) → (XII) sind die üblichen organischen Lösungsmittel. Hierzu gehören bevorzugt Alkohole wie Methanol, Ethanol, n-Propanol, Isopropanol, n-Butanol, n-Pentanol oder tert.-Butanol, oder Ether wie Diethylether, Tetrahydrofuran, 2-Methyltetrahydrofuran, Dioxan oder Glykoldimethylether, oder andere Lösungsmittel wie Aceton, Dichlormethan, 1,2-Dichlorethan, Acetonitril, Dimethylformamid oder Dimethylsulfoxid. Ebenso ist es möglich, Gemische der genannten Lösungsmittel einzusetzen. Bevorzugt wird Ethanol verwendet.

Der Ringschluss erfolgt im Allgemeinen in einem Temperaturbereich von +50°C bis +150°C, bevorzugt bei +50°C bis +100°C, gegebenenfalls in einer Mikrowelle.

Der Ringschluss (IX) + (X) → (II) bzw. (XI) + (X) → (XII) erfolgt optional in Gegenwart wasserziehender Reaktionszusätze, beispielsweise in Gegenwart von Molekularsieb (4Å Porengröße) oder mittels Wasserabscheider. Die Umsetzung (IX) + (X) → (II) bzw. (XI) + (X) → (XII) erfolgt unter Verwendung eines Überschusses des Reagenzes der Formel (X), beispielsweise mit 1 bis 20 Äquivalenten des Reagenzes (X), gegebenenfalls unter Zusatz von Basen (wie z.B. Natriumhydrogencarbonat) wobei die Zugabe dieses Reagenzes einmalig oder in mehreren Portionen erfolgen kann.

Weitere erfindungsgemäße Verbindungen können gegebenenfalls auch hergestellt werden durch Umwandlungen von funktionellen Gruppen einzelner Substituenten, insbesondere den unter R³ aufgeführten, ausgehend von den nach obigen Verfahren erhaltenen Verbindungen der Formel (I). Diese Umwandlungen werden nach üblichen, dem Fachmann bekannten Methoden durchgeführt und umfassen beispielsweise Reaktionen wie nukleophile und elektrophile Substitutionen, Oxidationen, Reduktionen, Hydrierungen, Übergangsmetall-katalysierte Kupplungsreaktionen, Eliminierungen, Alkylierung, Aminierung, Veresterung, Esterspaltung, Veretherung, Etherspaltung, Bildung von Carbonamiden, sowie Einführung und Entfernung temporärer Schutzgruppen.

Die erfindungsgemäßen Verbindungen besitzen wertvolle pharmakologische Eigenschaften und können zur Vorbeugung und Behandlung von Erkrankungen bei Menschen und Tieren verwendet werden. Die erfindungsgemäßen Verbindungen eröffnen eine weitere Behandlungsalternative und stellen somit eine Bereicherung der Pharmazie dar.

Die erfindungsgemäßen Verbindungen bewirken eine Gefäßrelaxation und eine Hemmung der Thrombozytenaggregation und führen zu einer Blutdrucksenkung sowie zu einer Steigerung des koronaren Blutflusses. Diese Wirkungen sind über eine direkte Stimulation der löslichen Guanylatcyclase und einen intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (endothelium-derived relaxing factor), NO-Donatoren, Protoporphyrin IX, Arachidonsäure oder Phenylhydrazin-Derivate.

Die erfindungsgemäßen Verbindungen eignen sich zur Behandlung und/oder Prophylaxe von kardiovaskulären, pulmonalen, thromboembolischen und fibrotischen Erkrankungen.

Die erfindungsgemäßen Verbindungen können daher in Arzneimitteln zur Behandlung und/oder Prophylaxe von kardiovaskulären Erkrankungen wie beispielsweise Bluthochdruck (Hypertonie), resistente Hypertonie, akute und chronische Herzinsuffizienz, koronare Herzerkrankung, stabile und instabile Angina pectoris, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden Grad I-III (AB-Block I-III), supraventrikuläre Tachyarrhythmie, Vorhofflimmern, Vorhoffflattern, Kammerflimmern, Kammerflattern, ventrikuläre Tachyarrhytmie, Torsade de pointes-Tachykardie, Extrasystolen des Vorhoffs und des Ventrikels, AV-junktionale Extrasystolen, Sick-Sinus Syndrom, Synkopen, AV-Knoten-Reentrytachykardie, Wolff-Parkinson-White-Syndrom, von akutem Koronarsyndrom (ACS), autoimmune Herzerkrankungen (Perikarditis, Endokarditis, Valvolitis, Aortitis, Kardiomyopathien), Schock wie kardiogenem Schock, septischem Schock und anaphylaktischem Schock, Aneurysmen, Boxerkardiomyopathie (premature ventricular contraction (PVC)), zur Behandlung und/oder Prophylaxe von thromboembolischen Erkrankungen und Ischämien wie myokardiale Ischämie, Myokardinfarkt, Hirnschlag, Herzhypertrophie, transistorischen und ischämischen Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, peripheren Durchblutungsstörungen, Reperfusionsschäden, arterielle und venöse Thrombosen, Mikroalbuminurie, Herzmuskelschwäche, endotheliale Dysfunktion, zur Verhinderung von Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädigungen (Vasculitis), erhöhte Spiegel von Fibrinogen und von LDL geringer Dichte sowie erhöhte Konzentrationen von Plasminogenaktivator-Inhibitor 1 (PAI-1), sowie zur Behandlung und/oder Prophylaxe von erektiler Dysfunktion und weiblicher sexueller Dysfunktion eingesetzt werden.

Im Sinne der vorliegenden Erfindung umfasst der Begriff Herzinsuffizienz sowohl akute als auch chronische Erscheinungsformen der Herzinsuffizienz, wie auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz und akute Phasen der Verschlechterung einer bestehenden chronischen Herzinsuffizienz (worsening heart failure).

Darüber hinaus können die erfindungsgemäßen Verbindungen auch zur Behandlung und/oder Prophylaxe von Arteriosklerose, Lipidstoffwechselstörungen, Hypolipoproteinämien, Dyslipidämien, Hypertriglyceridämien, Hyperlipidämien, Hypercholesterolämien, Abetelipoproteinämie, Sitosterolämie, Xanthomatose, Tangier Krankheit, Fettsucht (Adipositas), Fettleibigkeit (Obesitas) und von kombinierten Hyperlipidämien sowie des Metabolischen Syndroms eingesetzt werden.

Außerdem können die erfindungsgemäßen Verbindungen zur Behandlung und/oder Prophylaxe von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden.

Weiterhin eignen sich die erfindungsgemäßen Verbindungen zur Behandlung urologischer Erkrankungen wie beispielsweise benignes Prostata-Syndrom (BPS), benigne Prostata-Hyperplasie (BPH), benigne Prostata Vergrösserung (BPE), Blasenentleerungsstörung (BOO), untere Harnwegssyndrome (LUTS, einschließlich Felines Urologisches Syndrom (FUS)), Erkrankungen des Urogenital-Systems einschliesslich neurogene überaktive Blase (OAB) und (IC), Inkontinenz (UI) wie beispielsweise Misch-, Drang-, Stress-, oder Überlauf-Inkontinenz (MUI, UUI, SUI, OUI), Beckenschmerzen, benigne und maligne Erkrankungen der Organe des männlichen und weiblichen Urogenital-Systems.

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem Nierenversagen. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantatabstoßung, Immunkomplex-induzierte Nierenerkrankungen, durch toxische Substanzen induzierte Nephropathie, Kontrastmittel-induzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignen sich die erfindungsgemäßen Verbindungen auch zur Behandlung und/oder Prophylaxe von asthmatischen Erkrankungen, pulmonaler arterieller Hypertonie (PAH) und anderen Formen der pulmonalen Hypertonie (PH), umfassend mit Linksherzerkrankung, HIV, Sichelzellanämie, Thromboembolien (CTEPH), Sarkoidose, COPD oder Lungenfibrose assoziierte pulmonale Hypertonie, der chronisch-obstruktive Lungenerkrankung (COPD), des akuten Atemwegssyndrom (ARDS), der akuten Lungenschädigung (ALI), der alpha-1-Antitrypsin-Defizienz (AATD), der Lungenfibrose, des Lungenemphysem (z.B. durch Zigarettenrauch induziertes Lungenemphysem) und der zystischen Fibrose (CF).

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Demyelinisation, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung und/oder Prophylaxe von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen und Tinnitus eingesetzt werden.

Zudem besitzen die erfindungsgemäßen Verbindungen antiinflammatorische Wirkung und können daher als entzündungshemmende Mittel zur Behandlung und/oder Prophylaxe von Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankungen, entzündlichen Hauterkrankungen sowie entzündlichen Augenerkrankungen eingesetzt werden.

Des weiteren können die erfindungsgemäßen Verbindungen ebenfalls zur Behandlung und/ oder Prophylaxe von Autoimmunerkrankungen eingesetzt werden.

Weiterhin sind die erfindungsgemäßen Verbindungen zur Behandlung und/oder Prophylaxe fibrotischer Erkrankungen der inneren Organe, wie beispielsweise der Lunge, des Herzens, der Niere, des Knochenmarks und insbesondere der Leber, sowie dermatologischer Fibrosen und fibrotischer Erkrankungen des Auges, geeignet. Im Sinne der vorliegenden Erfindungen umfasst der Begriff fibrotischer Erkrankungen insbesondere die folgenden Begriffe Leberfibrose, Leberzirrhose, Lungenfibrose, Endomyocardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung (auch nach chirurgischen Eingriffen), Naevi, diabetische Retinopathie, proliferative Vitroretinopathie und Erkrankungen des Bindegewebes (z.B. Sarkoidose).

Weiterhin eignen sich die erfindungsgemäßen Verbindungen zur Bekämpfung postoperativer Narbenbildung, z.B. in Folge von Glaukom-Operationen.

Die erfindungsgemäßen Verbindungen können ebenfalls kosmetisch bei alternder und verhornender Haut eingesetzt werden.

Außerdem sind die erfindungsgemäßen Verbindungen zur Behandlung und/ oder Prophylaxe von Hepatitis, Neoplasma, Osteoporose, Glaukom und Gastroparese geeignet.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung sind die erfindungsgemäßen Verbindungen zur Verwendung in einem Verfahren zur Behandlung und/ oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist die Verwendung der erfindungsgemäßen Verbindungen zur Herstellung eines Arzneimittels zur Verwendung zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Verwendung zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Die vorliegende Erfindung offenbart auch ein Verfahren zur Behandlung und/oder Prophylaxe von Erkrankungen, insbesondere der zuvor genannten Erkrankungen, unter Verwendung einer wirksamen Menge von mindestens einer der erfindungsgemäßen Verbindungen.

Die vorliegende Erfindung offenbart auch ein Verfahren zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO; und/oder
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil; und/oder
- antithrombotisch wirkende Mittel, beispielhaft und vorzugsweise aus der Gruppe der Thrombozytenaggregationshemmer, der Antikoagulantien oder der profibrinolytischen Substanzen; und/oder
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten, neutrale Endopeptidase (NEP) Hemmer und Kombinationen aus diesen Gruppen sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten; und/oder
- antifibrotisch wirkende Mittel, beispielhaft und vorzugsweise aus der Gruppe der Kinase-Inhibitoren oder TGF-beta- bzw. TNF-alpha-Modulatoren

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Dabigatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), Edoxaban (DU-176b), Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten, neutrale Endopeptidase (NEP) Hemmer sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan oder einem dualen Angiotensin AII-Antagonisten/NEP-Inhibitor, wie beispielsweise und vorzugsweise LCZ696 (Valsartan/Sacubitril), verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Schleifendiuretikum, wie beispielsweise Furosemid, Torasemid, Bumetanid und Piretanid, mit kaliumsparenden Diuretika wie beispielsweise Amilorid und Triamteren, mit Aldosteronantagonisten, wie beispielsweise Spironolacton, Kaliumcanrenoat und Eplerenon sowie Thiaziddiuretika, wie beispielsweise Hydrochlorothiazid, Chlorthalidon, Xipamid, und Indapamid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Bei einer bevorzugten Ausführungsform der Erfindung werden die erfindungsgemäßen Verbindungen in Kombination mit einem Kinase-Inhibitor, wie beispielhaft und vorzugsweise Nintedanib, verabreicht.

Bei einer bevorzugten Ausführungsform der Erfindung werden die erfindungsgemäßen Verbindungen in Kombination mit einem TGF-beta- bzw. TNF-alpha-Modulator, wie beispielhaft und vorzugsweise Pirfenidon, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.001 bis 2 mg/kg, vorzugsweise etwa 0.001 bis 1 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- abs.: absolut (= getrocknet)
- aq.: wässrige Lösung
- ber.: berechnet
- Boc: *tert*-Butyloxycarbonyl
- br.: Verbreitertes Signal (NMR Kupplungsmuster)
- CAS-Nr.: Chemical Abstracts Service Nummer
- Cbz: Benzyloxycarbonyl
- δ: Verschiebung im NMR Spektrum (Angabe in ppm)
- d: Dublett (NMR-Kupplungsmuster)
- DC: Dünnschichtchromatographie
- DCI: direkte chemische Ionisation (bei MS)
- DMAP: 4-*N,N*-Dimethylaminopyridin
- DMF: Dimethylformamid
- DMSO: Dimethylsulfoxid
- EDCI: *N*-[3-(Dimethylamino)propyl]-N'-ethylcarbodiimid
- d. Th.: der Theorie (bei Ausbeute)
- *ent*: enantiomerenrein
- eq.: Äquivalent(e)
- ESI: Elektrospray-Ionisation (bei MS)
- Et: Ethyl
- gef.: gefunden
- h: Stunde(n)
- HATU: N-[(Dimethylamino)(3H-[1,2,3]triazolo[4,5-b]-pyridin-3-yloxy)methylen]-N-methylmethanaminiumhexafluorophosphat
- HOBT: 1H-Benzotriazol-1-ol
- HPLC: Hochdruck-, Hochleistungsflüssigchromatographie
- HRMS: hochaufgelöste Massenspektrometrie
- ID: Innendurchmesser
- konz.: konzentriert
- LC-MS: Flüssigchromatographie-gekoppelte Massenspektrometrie
- LiHMDS: Lithiumhexamethyldisilazid
- m: Multiplett
- Me: Methyl
- min: Minute(n)
- MS: Massenspektrometrie
- NMR: Kemresonanzspektrometrie
- PDA: Photodiodenarraydetektor
- Pd₂dba₃: Tris-(dibenzylidenaceton)-dipalladium
- Ph: Phenyl
- q: Quartett (NMR Kupplungsmuster)
- quint.: Quintett (NMR Kupplungsmuster)
- *rac*: racemisch
- *rel*: relative Stereochemie
- R_{F}: Retentionsfaktor (bei Dünnschichtchromatographie)
- RT: Raumtemperatur
- Rₜ: Retentionszeit (bei HPLC)
- s: Singulett (NMR Kupplungsmuster)
- t: Triplett (NMR Kupplungsmuster)
- THF: Tetrahydrofuran
- TBTU: (Benzotriazol-1-yloxy)bisdimethylaminomethyliumfluoroborat
- UPLC-MS: Ultradruck-Flüssigchromatographiegekoppelte Massenspektrometrie
- UV: Ultraviolett-Spektrometrie
- v/v: Volumen zu Volumen-Verhältnis (einer Lösung)
- Xantphos: 4,5-Bis(diphenylphosphino)-9,9-dimethylxanthene
- XPHOS: Dicyclohexyl-(2',4',6'-triisopropylbiphenyl-2-yl)-phosphin

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Angaben zu Kopplungsmustern in NMR-Spektren sind beschreibender Natur, Kopplungsmuster höherer Ordnung werden nicht als solche beschrieben.

### LC/MS- und HPLC-Methoden:

### Methode 1 (LC-MS):

Instrument: Waters ACQUITY SQD UPLC System; Säule: Waters Acquity UPLC HSS T3 1,8µ 50 x 1mm; Eluent A: 1 l Wasser + 0.25 ml 99%ige Ameisensäure, Eluent B: 1 l Acetonitril + 0.25 ml 99%ige Ameisensäure; Gradient: 0.0 min 90% A → 1.2 min 5% A → 2.0 min 5% A; Ofen: 50°C; Fluss: 0.40 ml/min; UV-Detektion: 210 - 400 nm.

### Methode 2 (LC-MS):

Instrument: Micromass Quattro Premier mit Waters UPLC Acquity; Säule: Thermo Hypersil GOLD 1.9 µ 50 mm x 1 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Methode 3 (LC-MS):

Gerätetyp MS: Waters Micromass Quattro Micro; Gerätetyp HPLC: Agilent 1100 Serie; Säule: Thermo Hypersil GOLD 3 µ 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 4 (LC-MS):

Instrument MS: Waters SQD; Instrument HPLC: Waters UPLC; Säule: Zorbax SB-Aq (Agilent), 50 mm x 2.1 mm, 1.8 µm; Eluent A: Wasser + 0.025% Ameisensäure, Eluent B: Acetonitril (ULC) + 0.025% Ameisensäure; Gradient: 0.0 min 98% A - 0.9 min 25% A - 1.0 min 5% A - 1.4 min 5% A - 1.41 min 98% A - 1.5 min 98% A; Ofen: 40°C; Fluss: 0.600 ml/min; UV-Detektion: DAD; 210 nm.

### Methode 5 (LC-MS):

Instrument MS: Waters ZQ 2000; Instrument HPLC: Agilent 1100, 2-Säulen-Schaltung, Autosampler: HTC PAL; Säule: YMC-ODS-AQ, 50 mm x 4.6 mm, 3.0 µm; Eluent A: Wasser + 0.1% Ameisensäure, Eluent B: Acetonitril + 0.1% Ameisensäure; Gradient: 0.0 min 100% A - 0.2 min 95% A - 1.8 min 25% A - 1.9 min 10% A - 2.0 min 5% A - 3.2 min 5% A - 3.21 min 100% A - 3.35 min 100% A; Ofen: 40°C; Fluss: 3.0 ml/min; UV-Detektion: 210 nm.

### Methode 6 (präparative HPLC):

Säule: Macherey-Nagel VP 50/21 Nucleosil 100-5 C18 Nautilus. Flußrate: 25 ml/min. Gradient: A = Acetonitril, B= Wasser + 0.1% Ameisensäure, 0 min 10% A; 2.00 min 10% A; 6.00 min 90% A ; 7.00 min 90% A ; 7.10 min 10% A ; 8 min 10% A; UV-Detektion: 220 nm.

### Methode 7 (präparative HPLC):

Säule: Phenomenex Gemini C18; 110A, AXIA, 5 µm, 21.2 X 50 mm 5micron; Gradient: A = Wasser + 0.1 % konz. Ammoniak, B = Acetonitril, 0 min = 10% B, 2 min = 10% B, 6 min = 90% B, 7 min = 90% B, 7.1 min = 10% B, 8 min = 10% B, Flußrate 25 ml/min, UV-Detektion 220 nm.

### Methode 8 (präparative HPLC):

Säule: Axia Gemini 5 µ C18 110 A, 50 x 21.5 mm, P/NO: 00B-4435-P0-AX, S/NO: 35997-2, Gradient: A=Wasser + 0.1 % konz. aq. Ammoniak, B = Acetonitril, 0 min = 30 % B, 2 min = 30% B, 6 min = 100% B, 7 min = 100% B, 7.1 Min = 30% B, 8 Min=30% B, Flußrate 25 ml/min, UV-Detektion 220 nm.

### Methode 9 (präparative HPLC):

Säule: Macherey-Nagel VP 50/21 Nucleosil 100-5 C18 Nautilus. Flussrate: 25 ml/min. Gradient: A = Wasser + 0.1 % Ameisensäure, B = Methanol, 0 min = 30 % B, 2 min = 30% B, 6 min = 100% B, 7 min = 100% B, 7.1 min = 30% B, 8 min = 30% B, Flussrate 25 ml/min, UV-Detektion 220 nm.

### Methode 10 (präparative HPLC):

Säule: Macherey-Nagel VP 50/21 Nucleosil 100-5 C18 Nautilus. Flussrate: 25 ml/min. Gradient: A = Wasser + 0.1 % konz. aq. Ammoniak, B = Methanol, 0 min = 30 % B, 2 min = 30% B, 6 min = 100% B, 7 min = 100% B, 7.1 min = 30% B, 8 min = 30% B, Flussrate 25 ml/min, UV-Detektion 220 nm.

### Methode 11 (präparative HPLC):

Instrument MS: Waters, Instrument HPLC: Waters (Säule Waters X-Bridge C18, 18 mm x 50 mm, 5 µm, Eluent A: Wasser + 0.05% Triethylamin, Eluent B: Acetonitril (ULC) + 0.05% Triethylamin, Gradient: 0.0 min 95%A - 0.15 min 95%A - 8.0 min 5%A - 9.0 min 5%A; Fluss: 40 ml/min; UV-Detektion: DAD; 210 - 400 nm).

### bzw.:

Instrument MS: Waters, Instrument HPLC: Waters (Säule Phenomenex Luna 5µ C18(2) 100A, AXIA Tech. 50 x 21.2 mm, Eluent A: Wasser + 0.05% Ameisensäure, Eluent B: Acetonitril (ULC) + 0.05% Ameisensäure, Gradient: 0.0 min 95%A - 0.15 min 95%A - 8.0 min 5%A - 9.0 min 5%A; Fluss: 40 ml/min; UV-Detektion: DAD; 210 - 400 nm).

### Methode 12 (LC-MS):

Instrument MS: Waters SQD; Instrument HPLC: Waters UPLC; Säule: Zorbax SB-Aq (Agilent), 50 mm x 2.1 mm, 1.8 µm; Eluent A: Wasser + 0.025% Ameisensäure, Eluent B: Acetonitril (ULC) + 0.025% Ameisensaeure; Gradient: 0.0 min 98%A - 0.9 min 25%A - 1.0 min 5%A - 1.4 min 5%A - 1.41 min 98%A - 1.5 min 98%A; Ofen: 40°C; Fluss: 0.600 ml/min; UV-Detektion: DAD; 210 nm.

### Methode 13 (DCI-MS):

Gerät: DSQ II; Thermo Fisher-Scientific; DCI mit NH₃, Fluss: 1.1 ml/min; Quellentemeperatur: 200°C; Ionisierungsenergie 70 eV; DCI-Heizfaden bis 800°C aufheizen; Mass-Range 80-900.

### Methode 14 (GC-MS):

Instrument: Micromass GCT, GC6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### Methode 15 (MS):

Gerät: Waters ZQ; Ionisierungsart: ESI (+); Laufmittel; Acetonitril/Wasser.

### Methode 16 (LCMS):

Instrument: Waters ACQUITY SQD UPLC System; Säule: Waters Acquity UPLC HSS T3 1.8 µ 30 x 2 mm; Eluent A: 1 l Wasser + 0.25 ml 99%ige Ameisensäure, Eluent B: 1 l Acetonitril + 0.25 ml 99%ige Ameisensäure; Gradient: 0.0 min 90% A → 1.2 min 5% A → 2.0 min 5% A Ofen: 50°C; Fluss: 0.60 ml/min; UV-Detektion: 208 - 400 nm.

### Methode 17 (LC-MS):

Instrument: Micromass Quattro Premier mit Waters UPLC Acquity; Säule: Thermo Hypersil GOLD 1.9 µ 50 x 1 mm; Eluent A: 1 l Wasser + 0.5 ml 50%ige Ameisensäure, Eluent B: 1 l Acetonitril + 0.5 ml 50%ige Ameisensäure; Gradient: 0.0 min 97% A → 0.5 min 97% A → 3.2 min 5% A → 4.0 min 5% A Ofen: 50°C; Fluss: 0.3 ml/min; UV-Detektion: 210 nm.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

Die in den folgenden Paragraphen angegebenen Multiplizitäten von Protonensignalen in ¹H-NMR-Spektren geben die jeweils beobachtete Signalform wieder und berücksichtigen keine Signalphänomene höherer Ordnung. Alle Angaben in ¹H-NMR-Spektren geben die Chemischen Verschiebungen δ in ppm an.

Zusätzlich können die Ausgangsverbindungen, Intermediate und Ausführungsbeispiele als Hydrate vorliegen. Eine quantitative Bestimmung des Wassergehaltes erfolgte nicht. Die Hydrate können unter Umständen einen Einfluss auf das ¹H-NMR-Spektrum haben und ggf. das Wasser-Signal in ¹H-NMR verschieben und/oder stark verbreitern.

Bei Aufreinigungen von erfindungsgemäßen Verbindungen per präparativer HPLC nach den oben beschriebenen Methoden, in denen die Elutionsmittel Zusatzstoffe wie beispielsweise Trifluoressigsäure, Ameisensäure oder Ammoniak enthalten, können die erfindungsgemäßen Verbindungen in Salz-Form, beispielsweise als Trifluoracetat, Formiat oder Ammonium-Salz anfallen, sofern die erfindungsgemäßen Verbindungen eine ausreichend basische bzw. saure Funktionalität enthalten. Ein solches Salz kann durch verschiedene dem Fachmann bekannte Methoden in die entsprechende freie Base bzw. Säure überführt werden.

Wenn bei den im Folgenden beschriebenen Synthese-Intermediaten und Ausführungsbeispielen der Erfindung eine Verbindung in der Form eines Salzes der korrespondierenden Base bzw. Säure aufgeführt ist, so ist die exakte stöchiometrische Zusammensetzung eines solchen Salzes, wie es nach dem jeweiligen Herstell- und/oder Reinigungsverfahren erhalten wurde, in der Regel nicht bekannt. Sofern nicht genauer spezifiziert, sind daher Namens- und Strukturformel-Zusätze wie beispielsweise "Hydrochlorid", "Trifluoracetat", "Natrium-Salz" bzw. "x HCl", "x CF₃CO₂H", "x Na⁺" bei solchen Salzen nicht stöchiometrisch zu verstehen, sondern haben allein deskriptiven Charakter bezüglich der enthaltenen salzbildenden Komponenten.

Sinngemäß gleiches gilt für den Fall, dass Synthese-Intermediate oder Ausführungsbeispiele oder Salze hiervon nach den beschriebenen Herstell- und/oder Reinigungsverfahren in Form von Solvaten, wie beispielsweise Hydraten, erhalten wurden, deren stöchiometrische Zusammensetzung (sofern definierter Art) nicht bekannt ist.

### Ausgangsverbindungen und Intermediate:

### Beispiel 1A

### 3-[(2,6-Difluorbenzyl)oxy]pyridin-2-amin

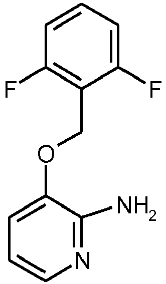

51 g Natriummethanolat (953 mmol, 1.05 Äquivalente) wurden in 1000 ml Methanol bei RT vorgelegt, mit 100 g 2-Amino-3-hydroxypyridin (908 mmol, 1 Äquivalent) versetzt und 15 min bei RT gerührt. Die Reaktionsmischung wurde am Vakuum eingeengt, der Rückstand in 2500 ml DMSO aufgenommen und mit 197 g 2,6-Difluorbenzylbromid (953 mmol, 1.05 Äquivalente) versetzt. Nach 4 h bei RT wurde das Reaktionsgemisch auf 20 L Wasser gegeben, für 15 min nachgerührt und der Feststoff abfiltriert. Der Feststoff wurde mit 1 L Wasser sowie 100 ml IsoPropanol und 500 ml Petrolether gewaschen und im Hochvakuum getrocknet. Es wurden 171 g der Titelverbindung (78% d. Th) erhalten.
¹H-NMR (400 MHz, DMSO-d₆): δ = 5.10 (s, 2 H), 5.52 (br. s, 2 H), 6.52 (dd, 1 H), 7.16 - 7.21 (m, 3 H), 7.49 - 7.56 (m, 2 H).

### Beispiel 2A

### Ethyl-8-[(2,6-difluorbenzyl)oxy]-2-methylimidazo[1,2-a]pyridin-3-carboxylat

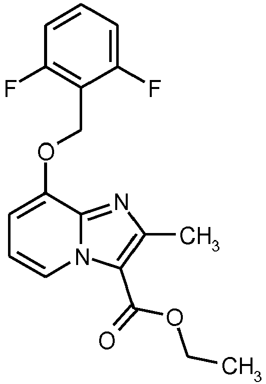

170 g 3-[(2,6-Difluorbenzyl)oxy]pyridin-2-amin (Beispiel 1A; 719 mmol, 1 Äquivalent) wurden in 3800 ml Ethanol vorgelegt, mit 151 g gepulvertem Molekularsieb 3Å und 623 g Ethyl-2-chloracetoacetat (3.6 mol, 5 Äquivalente) versetzt. Die Reaktionsmischung wurde für 24 h zum Rückfluß erhitzt, anschließend über Kieselgel abfiltriert und am Vakuum aufkonzentriert. Es wurde 48 h bei RT belassen und der entstandene Feststoff filtriert. Dann wurde dreimal mit wenig Iso-Propanol gerührt und anschließend abfiltriert und mit Diethylether gewaschen. Es wurden 60.8 g (23% d. Th.) der Titelverbindung erhalten. Die vereinigten Filtrate der Filtrationsschritte wurden eingeengt und der Rückstand an Kieselgel mit Cyclohexan/Diethylether als Eluent chromatographiert. Man erhielt weitere 46.5 g (18% d. Th.; Gesamtausbeute: 41% d. Th.) der Titelverbindung.
LC-MS (Methode 1): Rₜ = 1.01 min
MS (ESpos): m/z = 347 (M+H)⁺
¹H-NMR (400 MHz, DMSO-d₆): δ = 1.36 (t, 3 H), 2.54 (s, 3 H; verdeckt durch DMSO-Signal), 4.36 (q, 2 H), 5.33 (s, 2 H), 7.11 (t, 1 H), 7.18 - 7.27 (m, 3 H),7.59 (quint, 1 H), 8.88 (d, 1 H).

### Beispiel 3A

### 8-[(2,6-Difluorbenzyl)oxy]-2-methylimidazo[1,2-a]pyridin-3-carbonsäure

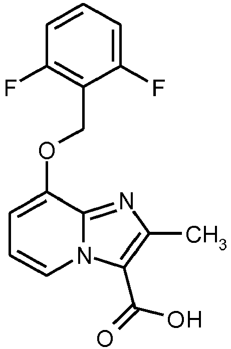

107 g Ethyl-8-[(2,6-difluorbenzyl)oxy]-2-methylimidazo[1,2-a]pyridin-3-carboxylat (Beispiel 2A; 300 mmol, 1 Äquivalent) wurden in 2.8 L THF/Methanol (1:1) gelöst, mit 1.5 L 1 N wässriger Lithiumhydroxid-Lösung (1.5 mol, 5 Äquivalente) versetzt und 16 h bei RT gerührt. Die organischen Lösemittel wurden am Vakuum entfernt und die resultierende wässrige Lösung im Eisbad mit 1 N wässriger Salzsäure auf pH 3-4 eingestellt. Der resultierende Feststoff wurde abfiltriert, mit Wasser und Iso-Propanol nachgewaschen und im Vakuum getrocknet. Es wurden 92 g (95% d. Th.) der Titelverbindung erhalten.
LC-MS (Methode 1): Rₜ = 0.62 min
MS (ESpos): m/z = 319.1 (M+H)⁺
¹H-NMR (400 MHz, DMSO-d₆): δ = 2.55 (s, 3 H; überlagert durch DMSO-Signal), 5.32 (s, 2 H); 7.01 (t, 1 H), 7.09 (d, 1 H), 7.23 (t, 2 H), 7.59 (quint, 1 H), 9.01 (d, 1 H).

### Beispiel 4A

### 3-(Cyclohexylmethoxy)pyridin-2-amin

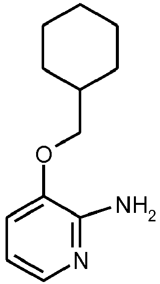

96 g Natriumhydroxid 45%ig in Wasser (1081 mmol, 1 Äquivalent) wurden in 1170 ml Methanol bei RT vorgelegt, mit 119 g 2-Amino-3-hydroxypyridin (1080 mmol, 1 Äquivalent) versetzt und 10 min bei RT weitergerührt. Die Reaktionsmischung wurde am Vakuum eingeengt, der Rückstand in 2900 ml DMSO aufgenommen und mit 101 g Cyclohexylmethylbromid (1135 mmol, 1.05 Äquivalente) versetzt. Nach 16 h bei RT wurde das Reaktionsgemisch langsam zu 6 L Wasser gegeben und die wässrige Lösung zweimal mit je 2 L Essigsäureethylester extrahiert. Die vereinigten organischen Phasen wurden mit je 1 L gesättigter, wässriger Natriumhydrogencarbonat-Lösung und Wasser gewaschen, getrocknet, filtriert und eingeengt. Der Rückstand wurde mit 500 ml n-Pentan gerührt, filtriert und am Vakuum getrocknet. Es wurden 130 g (58% d. Th.) der Titelverbindung erhalten.
LC-MS (Methode 3): Rₜ = 1.41 min
MS (ESpos): m/z = 207.1 (M+H)⁺

### Beispiel 5A

### Ethyl-8-(cyclohexylmethoxy)-2-methylimidazo[1,2-a]pyridin-3-carboxylat

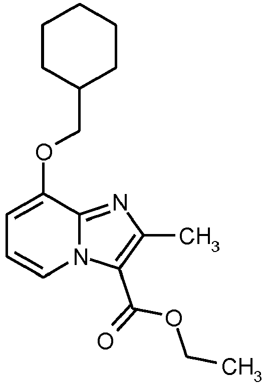

130 g 3-(Cyclohexylmethoxy)pyridin-2-amin (Beispiel 4A; 630 mmol, 1 Äquivalent) wurden in 3950 ml Ethanol vorgelegt und mit 436 ml Ethyl-2-chloracetoacetat (3.2 mol, 5 Äquivalente) versetzt. Es wurde 24 h am Rückfluß erhitzt und anschließend im Vakuum eingeengt. Das so erhaltene Rohprodukt wurde an Kieselgel mit Cyclohexan/Diethylether als Eluent chromatographiert und lieferte 66.2 g (33% d. Th.) der Titelverbindung.
LC-MS (Methode 1): Rₜ = 1.17 min
MS (ESpos): m/z = 317.1 (M+H)⁺
¹H-NMR (400 MHz, DMSO-d₆): δ = 1.02-1.31 (m, 5 H), 1.36 (t, 3 H), 1.64 - 1.77 (m, 3 H), 1.79 - 1.90 (m, 3 H), 2.60 (s, 3 H),3.97 (d, 2 H), 4.35 (q, 2 H), 6.95 (d, 1 H), 7.03 (t, 1 H), 8.81 (d, 1 H).

### Beispiel 6A

### 8-(Cyclohexylmethoxy)-2-methylimidazo[1,2-a]pyridin-3-carbonsäure

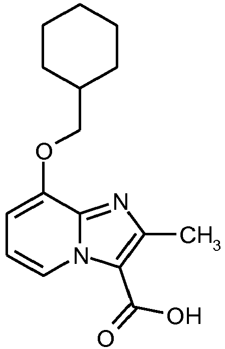

50 g Ethyl-8-(cyclohexylmethoxy)-2-methylimidazo[1,2-a]pyridin-3-carboxylat (Beispiel 5A; 158 mmol, 1 Äquivalent) wurde in 600 ml 1,4-Dioxan gelöst, mit 790 ml 2 N wässriger Natronlauge (1.58 mol, 10 Äquivalente) versetzt und 16 h bei RT gerührt. Es wurde mit 316 ml 6 N wässriger Salzsäure versetzt und auf ca. 1/5 des Gesamtvolumens eingeengt. Der resultierende Feststoff wurde abfiltriert, mit Wasser und tert.-Butylmethylether nachgewaschen und im Vakuum getrocknet. Es wurden 35 g (74% d. Th.) der Titelverbindung erhalten.

LC-MS (Methode 1): Rₜ = 0.81 min
MS (ESpos): m/z = 289.0 (M+H)⁺
¹H-NMR (400 MHz, DMSO-d₆): δ = 1.03-1.44 (m, 5 H), 1.64 - 1.78 (m, 3 H), 1.81 - 1.92 (m, 3 H), 2.69 (s, 3 H), 4.07 (d, 2 H), 7.30 - 7.36 (m, 2 H), 9.01 (d, 1 H).

### Beispiel 7A

### 5-Brom-3-[(2,6-difluorbenzyl)oxy]pyridin-2-amin

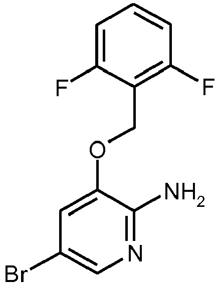

32.6 g 3-[(2,6-Difluorbenzyl)oxy]pyridin-2-amin (Beispiel 1A; 138 mmol, 1 Äquivalent) wurden in 552 ml 10%iger Schwefelsäure suspendiert und auf 0°C gekühlt. 8.5 ml Brom (165 mmol, 1.2 Äquivalente) wurde in 85 ml Essigsäure gelöst und dann innerhalb von 90 min zur eisgekühlten, Reaktionslösung getropft. Nach erfolgter Zugabe wurde 90 min bei 0°C -gerührt, anschließend mit 600 ml Essigsäureethylester verdünnt und die wässrige Phase abgetrennt. Die wässrige Phase wurde mit Essigsäureethylester extrahiert. Die organischen Phasen wurden vereinigt, mit gesättigter, wässriger Natriumhydrogencarbonat-Lösung gewaschen, getrocknet und eingeengt. Der Rückstand wurde in Dichlormethan gelöst und an Kieselgel chromatographiert (Petrolether/Essigsäureethylester Gradient als Eluent). Es wurden 24 g (55% d. Th.) der Titelverbindung erhalten.
LC-MS (Methode 1): Rₜ = 0.96 min
MS (ESpos): m/z = 315.1/317.1 (M+H)⁺
¹H-NMR (400 MHz, DMSO-d₆): δ = 5.14 (s, 2 H), 5.83 (br. s, 2 H), 7.20 (t, 2 H), 7.42 (d, 1 H), 7.54 (q, 1 H), 7.62 (d, 1 H).

### Beispiel 8A

### Ethyl-6-brom-8-[(2,6-difluorbenzyl)oxy]-2-methylimidazo[1,2-a]pyridin-3-carboxylat

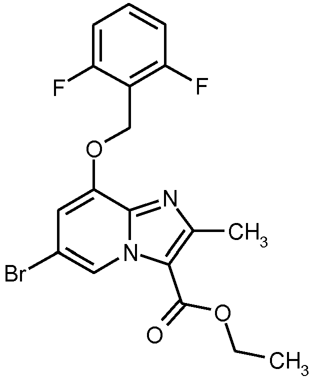

24 g 5-Brom-3-[(2,6-difluorbenzyl)oxy]pyridin-2-amin (Beispiel 7A; 76.2 mmol; 1 Äquivalent) in 400 ml Ethanol wurden mit 16 g gepulvertem Molekularsieb 3Å und 52.7 ml Ethyl-2-chloracetoacetat (380.8 mmol; 5 Äquivalente) versetzt und über Nacht zum Rückfluß erhitzt. Es wurden 8 g Molekularsieb zugegeben und für weitere 24 h zum Rückfluß erhitzt. Das Reaktionsgemisch wurde im Vakuum eingeengt, der Rückstand in Dichlormethan aufgenommen und an Kieselgel chromatographiert (Dichlormethan/Methanol 20:1 als Eluent). Die produkthaltigen Fraktionen wurden eingeengt und der Rückstand mit 100 ml Diethylether 30 min gerührt. Dann wurde abfiltriert, mit wenig Diethylether gewaschen und getrocknet. Es wurden 15 g (45% d. Th.) der Titelverbindung erhalten.
LC-MS (Methode 2): Rₜ = 1.43 min
MS (ESpos): m/z = 414.9/416.8 (M+H)⁺
¹H-NMR (400 MHz, DMSO-d₆): δ = 1.36 (t, 3 H), 2.54 (s, 3 H; verdeckt durch DMSO-Signal), 4.37 (q, 2 H), 5.36 (s, 2 H), 7.25 (t, 2 H), 7.42 (d, 1 H), 7.61 (q, 1 H), 9.00 (d, 1 H).

### Beispiel 9A

### 6-Brom-8-[(2,6-difluorbenzyl)oxy]-2-methylimidazo[1,2-a]pyridin-3-carbonsäure

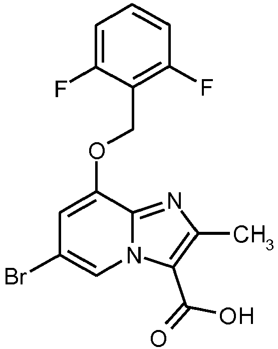

1.5 g Ethyl-6-brom-8-[(2,6-difluorbenzyl)oxy]-2-methylimidazo[1,2-a]pyridin-3-carboxylat (Beispiel 8A; 3.5 mmol, 1 Äquivalent) wurden in 72 ml THF/Methanol 5:1 gelöst, mit 17.6 ml IN wässriger Lithiumhydroxid-Lösung (17.6 mmol, 5 Äquivalente) versetzt, auf 40°C erwärmt und für 6 h bei dieser Temperatur gerührt. Dann wurde mit 6 N wässriger Salzsäure auf pH 4 gestellt und im Vakuum eingeengt. Der entstandene Feststoff wurde mit Wasser versetzt, -gerührt, abfiltriert, mit Wasser nachgewaschen und im Vakuum getrocknet. Es wurden 1.24 g der Titelverbindung (88% d. Th.) erhalten.
LC-MS (Methode 1): Rₜ = 0.93 min
MS (ESpos): m/z = 397.0/399.1 (M+H)⁺
¹H-NMR (400 MHz, DMSO-d₆): δ = 2.54 (s, 3 H; überlagert von DMSO-Signal); 5.36 (s, 2 H); 7.25 (t, 2 H); 7.40 (d, 1 H); 7.61 (q, 1 H); 9.06 (d, 1 H); 13.35 (br. s, 1 H).

### Beispiel 10A

### Ethyl-8-[(2,6-difluorbenzyl)oxy]-2,6-dimethylimidazo[1,2-a]pyridin-3-carboxylat

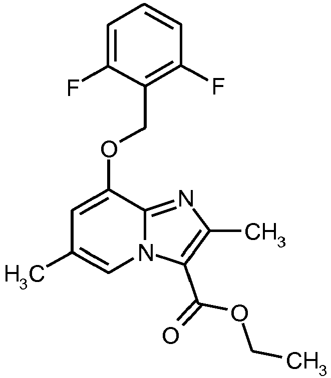

### Methode 1:

600 mg Ethyl-6-brom-8-[(2,6-difluorbenzyl)oxy]-2-methylimidazo[1,2-a]pyridin-3-carboxylat (Beispiel 8A; 1.4 mmol, 1 Äquivalent) und 230 mg 1,1'-Bis(diphenylphosphino)ferrocene-palladium(II)dichlorid dichloromethan Komplex (0.282 mmol, 20 mol%) wurden in 25 ml THF gelöst und mit 0.88 ml (1.76 mmol, 1.2 Äquivalente) einer 2 M Lösung von Methylzinkchlorid in THF versetzt. Die Reaktionsmischung wurde in der Mikrowelle für 40 min auf 100°C erhitzt. Die Reaktionsmischung wurde über Celite filtriert und anschließend im Vakuum eingeengt. Der Rückstand wurde chromatographiert (Biotage Isolera Four; Cyclohexan:Essigsäurethylester). Es wurden 225 mg (38% d. Th.) der Titelverbindung erhalten.

### Methode 2:

20.00 g (85.38 mmol) Ethyl-8-hydroxy-2,6-dimethylimidazo[1,2-a]pyridin-3-carboxylat aus Beispiel 15A, 19.44 g (93.91 mmol), 2,6-Difluorbenzylbromid und 61.20 g (187.83 mmol) Cäsiumcarbonat in 1.18 L DMF wurden 5 h bei 60°C gerührt. Dann wurde das Reaktionsgemisch auf 6.4 L 10%ige wässrige Natriumchlorid-Lösung gegeben und anschließend zweimal mit Essigsäureethylester extrahiert. Die vereinigten organischen Phasen wurden mit 854 ml 10%iger wässriger Natriumchlorid-Lösung gewaschen, getrocknet, eingeengt und über Nacht im Hochvakuum bei RT getrocknet. Es wurden 28.2 g (92% d. Th.; Reinheit 90%) der Titelverbindung erhalten.
LC-MS (Methode 1): Rₜ = 1.05 min
MS (ESpos): m/z = 361.1 (M+H)⁺
¹H-NMR (400 MHz, DMSO-d₆): δ = 1.38 (t, 3 H), 2.36 (s, 3 H), 4.35 (q, 2 H), 5.30 (s, 2 H), 7.10 (s, 1 H), 7.23 (t, 2 H), 7.59 (q, 1 H), 8.70 (s, 1 H).

### Beispiel 11A

### 8-[(2,6-Difluorbenzyl)oxy]-2,6-dimethylimidazo[1,2-a]pyridin-3-carbonsäure

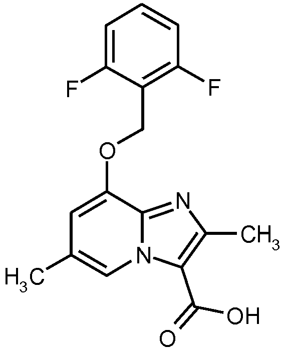

220 mg Ethyl-8-[(2,6-difluorbenzyl)oxy]-2,6-dimethylimidazo[1,2-a]pyridin-3-carboxylat (Beispiel 10A; 0.524 mmol, 1 Äquivalent) wurden in 7 ml THF/Methanol 1:1 gelöst, mit 2.6 ml 1 N wässriger Lithiumhydroxid-Lösung (2.6 mmol, 5 Äquivalente) versetzt und für 16 h bei RT gerührt. Es wurde im Vakuum eingeengt, der Rückstand mit IN wässriger Salzsäure sauer gestellt und 15 min gerührt. Der Feststoff wurde abfiltriert, mit Wasser nachgewaschen und im Vakuum getrocknet. Es wurden 120 mg der Titelverbindung (60% d. Th.) erhalten.
LC-MS (Methode 1): Rₜ = 0.68 min
MS (ESpos): m/z = 333.1 (M+H)⁺
¹H-NMR (400 MHz, DMSO-d₆): δ = 2.34 (s, 3 H), 5.28 (s, 2 H), 7.09 (s, 1 H), 7.23 (t, 2 H), 7.58 (q, 1 H), 8.76 (s, 1 H), 13.1 (br. s, 1 H).

### Beispiel 12A

### 3-(Benzyloxy)-5-brompyridin-2-amin

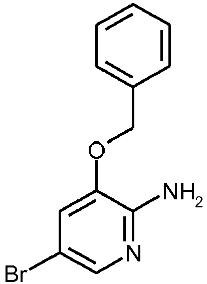

Die Zielverbindung ist literaturbekannt und beschrieben:
1) Palmer, A.M. et al. J Med. Chem. 2007, 50, 6240-6264.
2) ALTANA WO2005/58325
3) ALTANA WO2005/90358
4) Cui, J.T. et al. J Med. Chem. 2011, 54, 6342-6363

### Weitere Herstellungsmethode:

200 g (1 mol) 2-Amino-3-benzyloxypyridin wurden in 41 Dichlormethan vorgelegt und bei 0°C innerhalb von 30 min mit einer Lösung aus 62 ml (1.2 mol) Brom in 620 ml Dichlormethan versetzt. Nach beendeter Zugabe wurde die Reaktionslösung 60 min bei 0°C gerührt. Dann wurde das Gemisch mit ca. 41 gesättigter, wässriger Natriumhydrogencarbonat-Lösung versetzt. Die organische Phase wurde abgetrennt und eingeengt. Der Rückstand wurde mittels Kieselgelsäulechromatographie (Petrolether:Essigsäurethylester 6:4) gereinigt und die Produktfraktionen wurden eingeengt. Man erhielt 214 g (77% d. Th.) der Titelverbindung.
LC-MS (Methode 1): Rₜ = 0.92 min
MS (ESpos): m/z = 279 (M+H)⁺
¹H-NMR (400 MHz, DMSO-d₆): δ = 5.16 (s, 2H), 5.94 - 6.00 (m, 2H), 7.26 - 7.29 (m, 1H), 7.31 - 7.36 (m, 1H), 7.37 - 7.43 (m, 2H), 7.47-7.52 (m, 2H), 7.57 - 7.59 (m, 1H).

### Beispiel 13A

### Ethyl-8-(benzyloxy)-6-brom-2-methylimidazo[1,2-a]pyridin-3-carboxylat

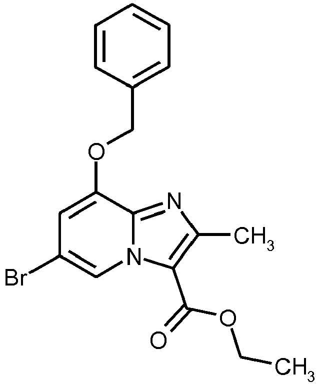

Unter Argon wurden 200 g (0.72 mol) 3-(Benzyloxy)-5-brompyridin-2-amin aus Beispiel 12A, 590 g (3.58 mol) Ethyl-2-chloracetoacetat und 436 g 3A Molekularsieb in 61 Ethanol suspendiert und 72 h bei Rückfluß gerührt. Die Reaktionsmischung wurde über Kieselgel abfiltriert und eingeengt. Der Rückstand wurde mittels Kieselgelchromatographie (Petrolether:Essigsäureethylester 9:1, anschließend 6:4) gereinigt und die Produktfraktionen wurden eingeengt. Man erhielt 221 g (79% d. Th.) der Zielverbindung.
LC-MS (Methode 16): Rₜ = 1.31 min
MS (ESpos): m/z = 389 (M+H)⁺
¹H-NMR (400 MHz, DMSO-d₆): δ = 1.36 (t, 3 H), 2.58 (s, 3 H), 4.32 - 4.41 (m, 2 H), 5.33 (s, 2 H), 7.28 - 7.32 (m, 1 H), 7.36 - 7.47 (m, 3 H), 7.49 - 7.54 (m, 2 H), 8.98 (d, 1 H).

### Beispiel 14A

### Ethyl-8-(benzyloxy)-2,6-dimethylimidazo[1,2-a]pyridin-3-carboxylat

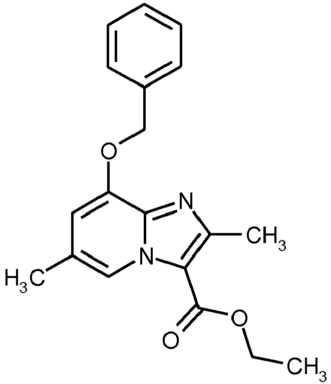

105 g (270 mmol) Ethyl-8-(benzyloxy)-6-brom-2-methylimidazo[1,2-a]pyridin-3-carboxylat aus Beispiel 13A wurden unter Argon in 4.2 11,4-Dioxan suspendiert und nacheinander mit 135.4 g (539 mmol, Reinheit 50%) Trimethylboroxin, 31.2 g (27 mmol) Tetrakis-(triphenylphosphin)palladium(0) sowie 78.3 g (566 mmol) Kaliumcarbonat versetzt und 8 h unter Rückfluss gerührt. Die auf RT abgekühlte Reaktionsmischung wurde über Kieselgel vom Niederschlag abfiltriert und das Filtrat wurde eingeengt. Der Rückstand wurde in Dichlormethan gelöst und mittels Kieselgelchromatographie (Dichlormethan:Essigsäureethylester = 9:1) gereinigt. Man erhielt 74 g (84.6% d. Th.) der Zielverbindung.
LC-MS (Methode 16): Rₜ = 1.06 min
MS (ESpos): m/z = 325 (M+H)⁺
¹H-NMR (400 MHz, DMSO-d₆): δ = 1.35 (t, 3 H), 2.34 (br. s, 3 H), 2.56 (s, 3 H), 4.31 - 4.38 (m, 2 H), 5.28 (br. s, 2 H), 6.99 - 7.01 (m, 1 H), 7.35 - 7.47 (m, 3 H), 7.49 - 7.54 (m, 2 H), 8.68 - 8.70 (m, 1 H).

### Beispiel 15A

### Ethyl-8-hydroxy-2,6-dimethylimidazo[1,2-a]pyridin-3-carboxylat

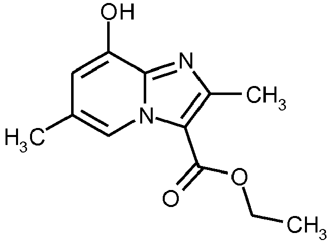

74 g (228 mmol) Ethyl-8-(benzyloxy)-2,6-dimethylimidazo[1,2-a]pyridin-3-carboxylat aus Beispiel 14A wurden in 1254 ml Dichlormethan und 251 ml Ethanol vorgelegt und unter Argon mit 20.1 g 10%igem Palladium auf Aktivkohle (wasserfeucht 50%) versetzt. Das Reaktionsgemisch wurde über Nacht bei RT und Normaldruck hydriert. Die Reaktionsmischung wurde über Kieselgel abfiltriert und eingeengt. Der Rohprodukt wurde mittels Kieselgel-chromatographie (Dichlormethan:Methanol = 95:5) gereinigt. Man erhielt 50.4 g (94% d. Th.) der Zielverbindung.
DCI-MS: (Methode 13) (ESpos): m/z = 235.2 (M+H)⁺
¹H-NMR (400 MHz, DMSO-d₆): δ = 1.35 (t, 3 H), 2.27 (s, 3 H), 2.58 (s, 3 H), 4.30 - 4.38 (m, 2 H), 6.65 (d, 1 H), 8.59 (s, 1 H), 10.57 (br. s, 1H).

### Beispiel 16A

### Ethyl-2,6-dimethyl-8-[(2,3,6-trifluorbenzyl)oxy]imidazo[1,2-a]pyridin-3-carboxylat

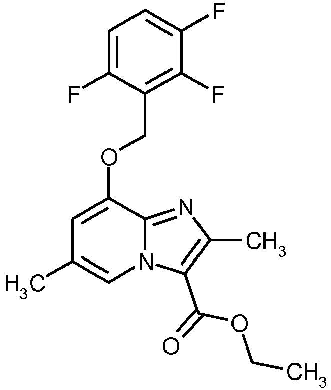

3.00 g (12.81 mmol) Ethyl-8-hydroxy-2,6-dimethylimidazo[1,2-a]pyridin-3-carboxylat Beispiel 15A, 3.27 g (14.1 mmol) 2-(Brommethyl)-1,3,4-trifluorbenzol und 9.18 g (28.17 mmol) Cäsiumcarbonat wurden in 183 ml trockenem DMF vorgelegt und für 30 min in einem auf 60°C erwärmten Ölbad erhitzt. Dann wurde mit ca. 1.8 l Wasser versetzt und 30 min gerührt. Der Feststoff wurde abfiltriert, mit Wasser gewaschen und im Vakuum getrocknet. Es wurden 5.07 g der Titelverbindung (99% d. Th.) erhalten.
LC-MS (Methode 1): Rₜ = 1.14 min
MS (ESpos): m/z = 379 (M+H)⁺
¹H-NMR (400 MHz, DMSO-d₆): δ = 1.35 (t, 3 H), 2.36 (s, 3 H); 2.55 (s, 3 H; überlagert durch DMSO-Signal), 4.36 (q, 2 H), 5.35 (s, 2 H), 7.09 (s, 1 H), 7.22 - 7.32 (m, 1 H), 7.60 - 7.73 (m, 1 H), 8.72 (s, 1 H).

### Beispiel 17A

### 2,6-Dimethyl-8-[(2,3,6-trifluorbenzyl)oxy]imidazo[1,2-a]pyridin-3-carbonsäure

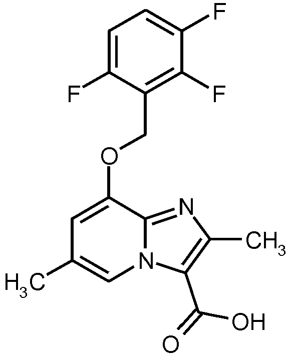

5.07 g (12.87 mmol) Ethyl-2,6-dimethyl-8-[(2,3,6-trifluorbenzyl)oxy]imidazo[1,2-a]pyridin-3-carboxylat Beispiel 16A wurden in 275 ml THF/Methanol (5/1) gelöst, mit 64.4 ml 1 N wässriger Lithiumhydroxid-Lösung versetzt und 3.5 h bei 40°C gerührt. Es wurde bei 0°C mit 6 N wässriger Salzsäure auf ca. pH 4 gebracht und dann eingeengt. Der erhaltene Feststoff wurde abfiltriert, mit Wasser nachgewaschen und im Vakuum getrocknet. Es wurden 4.77 g (98% d. Th.; Reinheit ca. 93%) der Titelverbindung erhalten.
LC-MS (Methode 1): Rₜ = 0.72 min
MS (ESpos): m/z = 351 (M+H)⁺
¹H-NMR (400 MHz, DMSO-d₆): δ = 2.37 (s, 3 H), 2.54 (s, 3 H; überlagert durch DMSO-Signal), 5.36 (s, 2 H), 7.11 (s, 1 H), 7.25 - 7.33 (m, 1 H), 7.61 - 7.73 (m, 1 H), 8.78 (s, 1 H), 13.10 (br. s, 1 H).

### Beispiel 18A

### 8-[(2,6-Difluorbenzyl)oxy]-2-methylimidazo[1,2-a]pyridin

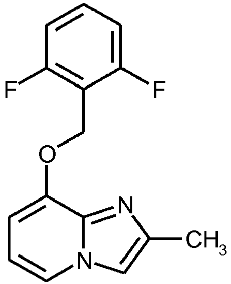

12 g 3-[(2,6-Difluorbenzyl)oxy]pyridin-2-amin (Beispiel 1A, 50.8 mmol, 1 Äquivalent) und 8 g 1-Chloraceton (86.4 mmol, 1.7 Äquivalente) in 90 ml Ethanol wurden über Nacht bei 80°C gerührt. Das Reaktionsgemisch wurde mit Kieselgel versetzt und eingeengt. Den Rückstand wurde mittels Kieselgelchromatographie (Laufmittelgemisch Dichlormethan/Ethanol = 50:1) gereinigt. Das erhaltene Produktgemisch wurde anschließend mittels Kieselgelchromatographie (Laufmittelgemisch Dichlormethan/Ethanol/Diethylamin = 50:1:0.1, 40:1:0.5, 30:1:0.5) gereinigt. Man erhielt 6.3 g (45% d. Th.) der Titelverbindung.
LC-MS (Methode 1): Rₜ = 0.58 min
MS (ESpos): m/z = 274 (M+H)⁺
¹H-NMR (400 MHz, DMSO-d₆): δ = 2.27 (s, 3 H), 5.27 (s, 2 H), 6.69 - 6.80 (m, 2 H), 7.23 (s, 2 H), 7.51 - 7.62 (m, 1 H), 7.65 (s, 1 H), 8.03 - 8.12 (m, 1 H).

### Beispiel 19A

### 3-Brom-8-[(2,6-difluorbenzyl)oxy]-2-methylimidazo[1,2-a]pyridin

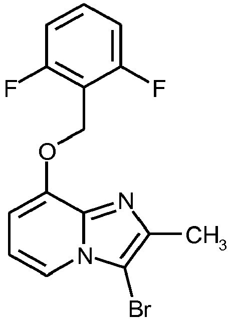

193 g 8-[(2,6-Difluorbenzyl)oxy]-2-methylimidazo[1,2-a]pyridin (Beispiel 18A, 0.7 mmol, 1 Äquivalent) wurden in 2.2 L Ethanol vorgelegt und mit 150.3 g N-Bromsuccinimid (0.8 mmol, 1.2 Äquivalente) versetzt. Nach 1.5 h bei RT wurde bei RT im Vakuum eingeengt. Der Rückstand wurde mit Essigsäureethylester verdünnt, die organische Phase wurde mit gesättigter wässriger Natriumhydrogencarbonat-Lösung gewaschen, über Natriumsulfat getrocknet, filtriert und einrotiert. Den Rückstand wurde mittels Kieselgelchromatographie (Laufmittelgemisch Cyclohexan/Essigsäureethylester = 98:2, 96:4, 92:8, 9:1, 8:2 und 7:3) gereinigt. Das erhaltene Produkt wurde mit 600 ml Essigsäureethylester gerührt und es wurde abdekantiert. Der Rückstand wurde im Vakuum getrocknet. Es wurden 23.4 g (9% d. Th.) der Titelverbindung erhalten.

Das Filtrat wurde im Vakuum eingeengt und der Rückstand mit 100 ml Essigsäureethylester gerührt. Die Essigsäureethylester Phase wurde abdekantiert und der Rückstand im Vakuum getrocknet. Es wurden weitere 6.1g (2.3% d. Th.) der Titelverbindung erhalten.
LC-MS (Methode 1): Rₜ = 0.90 min
MS (ESpos): m/z = 353 (M+H)⁺
¹H-NMR (400 MHz, DMSO-d₆): δ = 2.27 (s, 3 H), 5.27 (s, 2 H), 6.70 - 6.80 (m, 2 H), 7.23 (t, 2 H), 7.52 - 7.62 (m, 1 H), 7.65 (s, 1 H), 8.09 (d, 1 H).

### Beispiel 20A

### 8-[(2,6-Difluorbenzyl)oxy]-2,6-dimethylimidazo[1,2-a]pyridin

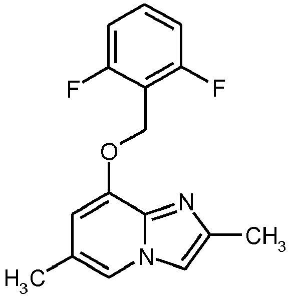

10.0 g (30.09 mmol) 8-[(2,6-Difluorbenzyl)oxy]-2,6-dimethylimidazo[1,2-a]pyridin-3-carbonsäure aus Beispiel 11A wurden in 228 ml Dioxan vorgelegt, mit 25.1 ml 6 N wässriger Salzsäure-Lösung versetzt und 2 h bei 100°C gerührt. Nach Abkühlen wurde Dioxan im Vakuum entfernt und der wässrige Rückstand mit 2 N wässriger Natronlauge auf pH 8 gebracht. Der erhaltene Feststoff wurde abfiltriert, mit Wasser gewaschen und im Hochvakuum getrocknet. Es wurden 8.97 g der Zielverbindung (97% d. Th., Reinheit 94%) erhalten.
LC-MS (Methode 1): Rₜ = 0.70 min
MS (ESpos): m/z = 289 (M+H)⁺
¹H-NMR (400 MHz, DMSO-d₆): δ = 2.22 - 2.30 (m, 6 H); 5.27 (s, 2 H); 6.67 (s, 1 H); 7.21 (t, 2 H); 7.53 - 7.63 (m, 2 H); 7.89 (s, 1 H).

### Beispiel 21A

### 3-Brom-8-[(2,6-difluorbenzyl)oxy]-2,6-dimethylimidazo[1,2-a]pyridin

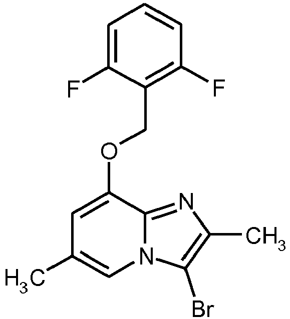

3.865 g (13.41 mmol) 8-[(2,6-Difluorbenzyl)oxy]-2,6-dimethylimidazo[1,2-a]pyridin aus Beispiel 20A wurden unter Argon und Lichtausschluss in 42 ml Ethanol vorgelegt, mit 2.625 g (14.75 mmol) *N*-Bromsuccinimid versetzt und 4 h bei Raumtemperatur gerührt. Das Reaktionsgemisch wurde eingeengt. Der Rückstand wurde mit ca. 100 ml Wasser gerührt und die entstandene Suspension anschließend 30 min bei RT gerührt. Der entstandene Niederschlag wurde abfiltriert, mit Wasser gewaschen und im Hochvakuum getrocknet. Es wurden 4.48 g der Zielverbindung (91% d. Th.) erhalten.
LC-MS (Methode 1): Rₜ = 0.93 min
MS (ESpos): m/z = 267 (M+H)⁺
¹H-NMR (400 MHz, DMSO-d₆): δ = 2.28 (s, 3H), 2.33 (s, 3 H); 5.30 (s, 2 H); 6.89 (s, 1 H); 7.22 (t, 2 H); 7.53-7.63 (m, 1 H); 7.75 (s, 1 H).

### Beispiel 22A

### 2,6-Dimethyl-8-[(2,3,6-trifluorbenzyl)oxy]imidazo[1,2-a]pyridin

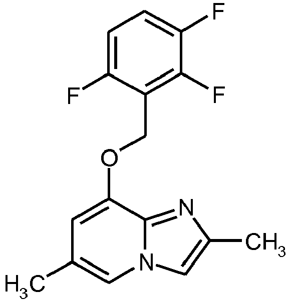

6.48 g (18.50 mmol) 2,6-Dimethyl-8-[(2,3,6-trifluorbenzyl)oxy]imidazo[1,2-a]pyridin-3-carbonsäure aus Beispiel 17A wurden in 140 ml Dioxan vorgelegt, mit 15.4 ml 6 N wässriger Salzsäure-Lösung versetzt und 4 h bei 100°C gerührt. Nach Abkühlen wurde Dioxan im Vakuum entfernt und der wässrige Rückstand mit 1 N Natronlauge auf pH 8 gebracht. Der entstandene Feststoff wurde abfiltriert, mit Wasser gewaschen und im Hochvakuum getrocknet. Es wurden 5.57g der Zielverbindung (96% d. Th.) erhalten.
LC-MS (Methode 1): Rₜ = 0.65 min
MS (ESpos): m/z = 307 (M+H)⁺
¹H-NMR (400 MHz, DMSO-d₆): δ = 2.20 - 2.29 (m, 6 H), 5.29 (s, 2 H), 6.69 (s, 1 H), 7.23 - 7.33 (m, 1 H), 7.57 (s, 1 H), 7.60-7.73 (m, 1 H), 7.91 (s, 1 H).

### Beispiel 23A

### 3-Brom-2,6-dimethyl-8-[(2,3,6-trifluorbenzyl)oxy]imidazo[1,2-a]pyridin

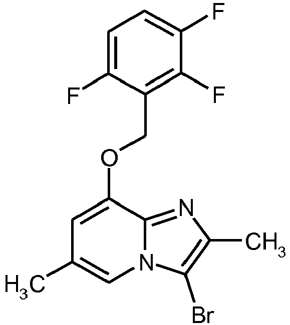

2.28 g (7.45 mmol) 2,6-Dimethyl-8-[(2,3,6-trifluorbenzyl)oxy]imidazo[1,2-a]pyridin aus Beispiel 22A wurden unter Argon und Lichtausschluss in 23.4 ml Ethanol vorgelegt, mit 1.46 g (8.20 mmol) *N*-Bromsuccinimid versetzt und 1.5 h bei Raumtemperatur gerührt. Das Reaktionsgemisch wurde im Vakuum eingeengt. Der Rückstand wurde mit 200 ml Wasser gerührt und die entstandene Suspension anschließend 2 h bei RT gerührt. Der entstandene Niederschlag wurde abfilrtiert, mit Wasser gewaschen und im Hochvakuum getrocknet. Es wurden 2.47 g der Zielverbindung (86% d. Th.) erhalten.
LC-MS (Methode 1): Rₜ = 0.97 min
MS (ESpos): m/z = 385 (M+H)⁺
¹H-NMR (400 MHz, DMSO-d₆): δ = 2.28 (s, 3 H), 2.33 (s, 3 H); 5.32 (s, 2 H); 6.87 (s, 1 H); 7.24 - 7.33 (m, 1 H); 7.62-7.73 (m, 1 H); 7.76 (s, 1 H).

### Beispiel 24A

### 8-[(2,6-Difluorbenzyl)oxy]-2-methylimidazo[1,2-a]pyridin-3-carboxamid

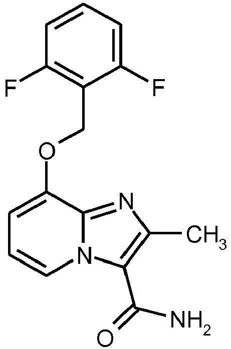

Unter Argon wurden 5 g 8-[(2,6-Difluorbenzyl)oxy]-2-methylimidazo[1,2-a]pyridin-3-carbonsäure (Beispiel 3A, 15.7 mmol, 1 Äquivalent) in 300 ml Dichlormethan vorgelegt und bei RT nacheinander mit 4.5 g 1-(3-Dimethylaminopropyl)-3-ethylcarbodiimidhydrochlorid (23.6 mmol, 1.5 Äquivalente) sowie 3.6 g 1-Hydroxy-1H-benzotriazol Hydrat (HOBT, 23.6 mmol, 1.5 Äquivalente) versetzt und 10 min bei RT gerührt. Dann wurden 4.2 g Ammoniumchlorid (78.5 mmol, 5 Äquivalente) und 19.2 ml *N,N-*Diisopropylethylamin (109.9 mmol, 7 Äquivalente) zugegeben und über Nacht bei RT gerührt. Es wurde einrotiert, der Rückstand mit Dichlormethan versetzt, filtriert, mit Dichlormethan nachgewaschen und über Nacht im Vakuum getrocknet. Es wurden 5.38 g (108% d. Th.) der Titelverbindung erhalten und ohne Reinigung weiter umgesetzt.
LC-MS (Methode 1): Rₜ = 0.65 min
MS (ESpos): m/z = 318.2 (M+H)⁺

### Beispiel 25A

### 8-[(2,6-Difluorbenzyl)oxy]-2-methylimidazo[1,2-a]pyridin-3-carbonitril

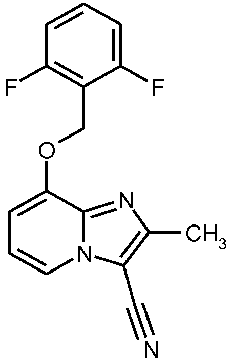

912 mg 8-[(2,6-Difluorbenzyl)oxy]-2-methylimidazo[1,2-a]pyridin-3-carboxamid (Beispiel 24A, 2.9 mmol, 1 Äquivalent) wurde in 13 ml THF vorgelegt und mit 0.6 ml Pyridin (7.4 mmol, 2.56 Äquivalente) versetzt. Dann wurden 1.04 ml Trifluoressigsäureanhydrid (7.4 mmol, 2.56 Äquivalente) zugetropft und der Ansatz über Nacht bei RT gerührt. Anschließend wurde auf Wasser gegeben und dreimal mit Essigsäureethylester extrahiert. Die vereinigten organischen Phasen wurden einmal mit gesättigter, wässriger Natriumhydrogencarbonat-Lösung, einmal mit 1 N wässriger Salzsäure und einmal mit gesättigter Natriumchlorid-Lösung gewaschen, über Natriumsulfat getrocknet und einrotiert. Der Rückstand wurde über Nacht im Vakuum getrocknet. Es wurden 787 mg (91% d. Th.) der Titelverbindung erhalten.
LC-MS (Methode 1): Rₜ = 0.97 min
MS (ESpos): m/z = 300.1 (M+H)⁺
¹H-NMR (400 MHz, DMSO-d₆): δ = 2.44 (s, 3 H), 5.33 (s, 2 H), 7.10 - 7.16 (m, 1 H), 7.18 - 7.28 (m, 3 H), 7.54 - 7.64 (m, 1 H), 8.22 (d, 1 H).

### Beispiel 26A

### 8-[(2,6-Difluorbenzyl)oxy]-2-methylimidazo[1,2-a]pyridin-3-carboximidamid

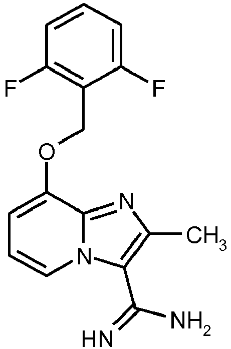

135 mg Ammoniumchlorid (2.5 mmol, 2.52 Äquivalente) wurden unter Argon in 3.9 ml Toluol vorgelegt und auf 0°C abgekühlt. Bei dieser Temperatur wurden 1.26 ml 2 M Trimethylaluminium in Toluol (2.5 mmol, 2.52 Äquivalente) zugegeben und die Lösung wurde 2 h bei RT gerührt. In einem anderen Kolben wurden 300 mg 8-[(2,6-Difluorbenzyl)oxy]-2-methylimidazo[1,2-a]pyridin-3-carbonitril (Beispiel 25A, 1.0 mmol, 1 Äquivalent) in 3.3 ml Toluol vorgelegt, bei RT mit 2 ml der zuvor hergestellten Lösung versetzt und 1 h bei 110°C gerührt. Dieser Vorgang wurde viermal wiederholt. Dann wurde der Ansatz abgekühlt, bei RT mit Kieselgel und einem 1:1 Gemisch aus Dichlormethan/Methanol versetzt und 30 min bei RT gerührt. Das Kieselgel wurde über eine Fritte abfiltriert. Es wurde mit Methanol gewaschen und das Filtrat wurde im Vakuum eingeengt. Der Rückstand wurde mittels Kieselgel-chromatographie (Eluent: Dichlormethan; Dichlormethan:Methanol = 10:2) gereinigt. Es wurden 137.5 mg (43% d. Th.) der Titelverbindung erhalten.
LC-MS (Methode 1): Rₜ = 0.51 min
MS (ESpos): m/z = 317.1 (M+H)⁺
¹H-NMR (400 MHz, DMSO-d₆): δ = 2.46 (s, 3 H), 5.32 (s, 2 H), 7.04 (t, 1 H), 7.14 (d, 1 H), 7.24 (t, 2 H), 7.53 - 7.66 (m, 1 H), 8.17 (d, 1 H), 9.31 (d, 3 H).

### Beispiel 27A

### 8-[(2,6-Difluorbenzyl)oxy]-N-hydroxy-2-methylimidazo[1,2-a]pyridin-3-carboximidamid

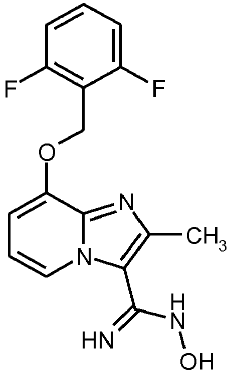

50.0 g (148.9 mmol) 8-[(2,6-Difluorbenzyl)oxy]-2-methylimidazo[1,2-a]pyridin-3-carbonitril aus Beispiel 25A wurden in Ethanol (1.5 l) suspendiert, mit 51.75 g (744.6 mmol) Hydroxylamin Hydrochlorid sowie 103.0 ml (744.6 mmol) Triethylamin versetzt und über Nacht bei RT gerührt. Anschliessend wurde im Vakuum eingeengt, mit Wasser (2.0 l) und Ethanol (100 ml) versetzt und 1 h gerührt. Der entstandene Feststoff wurde abfiltriert, mit Wasser gewaschen und über Nacht am Hochvakuum getrocknet. Es wurden 38.5 g (78 % d. Th.) der Titelverbindung erhalten.
LC-MS (Methode 1): Rₜ = 0.56 min
MS (ESpos): m/z = 333.2 (M+H)⁺

### Beispiel 28A

### 8-[(2,6-Difluorbenzyl)oxy]-2-methylimidazo[1,2-a]pyridin-3-carboximidamid Hydrochlorid

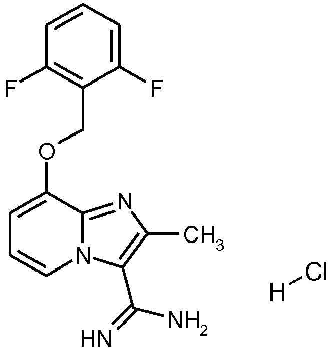

37.5 g (98.4 mmol, 87 % Reinheit) 8-[(2,6-Difluorbenzyl)oxy]-N-hydroxy-2-methylimidazo[1,2-a]pyridin-3-carboximidamid aus Beispiel 27A wurden in Essigsäure (1.0 l) vorgelegt und mit 11.14 ml (118.08 mmol) Essigsäureanhydrid versetzt. Danach wurden 7.5 g Palladium/Kohle (10%ig, feucht) zugegeben und es wurde 16 h bei Normaldruck hydriert. Es wurde über Kieselgur filtriert und mit Ethanol gewaschen. Nach Einengen wurde der Rückstand dreimal mit je 500 ml Toluol versetzt und im Vakuum eingeengt. Der Rückstand wurde mit 200 ml Essigsäureethylester gerührt, filtriert und im Hochvakuum getrocknet. Es wurden 22.0 g (59% d. Th.) der Titelverbindung erhalten.
LC-MS (Methode 1): Rₜ = 0.51 min
MS (ESpos): m/z = 317.2 (M+H)⁺
¹H-NMR (400 MHz, DMSO-d₆): δ = 1.82 (s, 3H), 2.46 (s, 3 H), 5.31 (s, 2 H), 6.93 (t, 1 H), 7.01 (d, 1 H), 7.21-7.25 (m, 2 H), 7.55 - 7.63 (m, 1 H), 8.55 (br d, 1 H).

### Beispiel 29A

### 8-[(2,6-Difluorbenzyl)oxy]-2,6-dimethylimidazo[1,2-a]pyridin-3-carboxamid

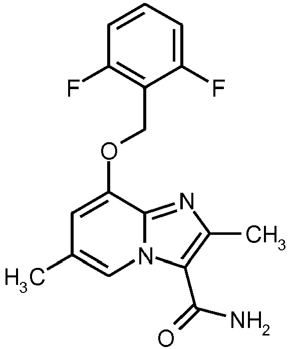

7.0 g (21.07 mmol) 8-[(2,6-Difluorbenzyl)oxy]-2,6-dimethylimidazo[1,2-a]pyridin-3-carbonsäure aus Beispiel 11A wurden in 403 ml Dichlormethan vorgelegt, mit 6.06 g (31.60 mmol) 1-(3-Dimethylaminopropyl)-3-ethylcarbodiimidhydrochlorid und 4.27 g (31.60 mmol) 1-Hydroxy-1H-benzotriazol-Hydrat versetzt und 10 min bei Raumtemperatur gerührt. Anschließend wurden 5.63 g (105.32 mmol) Ammoniumchlorid und 25.68 ml (147.5 mmol) *N,N*-Diisopropylethylamin zugegeben und über Nacht bei Raumtemperatur gerührt. Das Reaktionsgemisch wurde mit Wasser versetzt, der enthaltene Feststoff wurde abfiltriert, anschließend mit Wasser 30 min bei 50°C verrührt, erneut abfiltriert und mit Wasser gewaschen. Es wurden 4.59 g (65% d. Th.) der Titelverbindung erhalten. Von den vereinten Filtratfraktionen (Dichlormethan/Wasser) wurden die Phasen getrennt. Die Dichlormethanphase wurde je einmal mit gesättigter, wässriger Natriumhydrogencarbonatlösung und mit gesättigter, wässriger Natriumchloridlösung gewaschen. Die organische Phase wurde über Natriumsulfat getrocknet, filtriert und im Vakuum eingeengt Der Rückstand wurde mit wenig Acetonitril gerührt und abfiltriert. Es wurden weitere 1.29 g (17% d. Th.; Reinheit 93%) der Titelverbindung erhalten.
LC-MS (Methode 1): Rₜ = 0.64 min
MS (ESpos): m/z = 332 (M+H)⁺
¹H-NMR (400 MHz, DMSO-d₆): δ = 2.31 (s, 3H), 2.50 (s, 3 H; verborgen unter DMSO-Signal), 5.28 (s, 2 H), 6.92 (s, 1 H), 7.22 (t, 2 H), 7.35 (br. s, 2 H), 7.53-7.63 (m, 1 H); 8.62 (s, 1 H).

### Beispiel 30A

### 8-[(2,6-Difluorbenzyl)oxy]-2,6-dimethylimidazo[1,2-a]pyridin-3-carbonitril

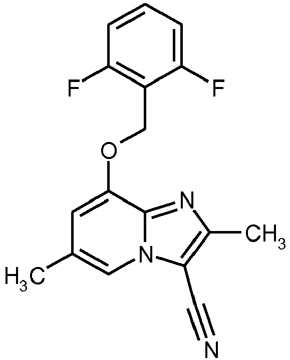

5.7 g (17.20 mmol) 8-[(2,6-Difluorbenzyl)oxy]-2,6-dimethylimidazo[1,2-a]pyridin-3-carboxamid Beispiel 29A wurden in 77 ml THF vorgelegt und mit 3.56 ml (44.0 mmol) Pyridin versetzt. Dann wurden bei RT 6.22 ml (44.0 mmol) Trifluoressigsäureanhydrid zugetropft und die Reaktionsmischung rührte 3 h bei RT. Nach beendeter Reaktionszeit wurde auf Wasser gegeben und dreimal mit Essigsäureethylester extrahiert. Die vereinigten organischen Phasen wurden einmal mit gesättigter, wässriger Natriumhydrogencarbonat-Lösung, einmal mit 1 N wässriger Salzsäure sowie einmal mit gesättigter Natriumchlorid-Lösung gewaschen, über Natriumsulfat getrocknet und im Vakuum eingeengt. Der Rückstand wurde über Nacht im Vakuum getrocknet. Es wurden 5.47 g (90% d. Th.) der Titelverbindung erhalten.
LC-MS (Methode 1): Rₜ = 1.12 min
MS (ESpos): m/z = 314 (M+H)⁺
¹H-NMR (400 MHz, DMSO-d₆): δ = 2.37 (s, 3 H), 2.41 (s, 3 H), 5.31 (s, 2 H), 7.12 (s, 1 H), 7.23 (t, 2 H), 7.54 - 7.63 (m, 1 H), 8.09 (s, 1 H).

### Beispiel 31A

### 8-[(2,6-Difluorbenzyl)oxy]-2,6-dimethylimidazo[1,2-a]pyridin-3-carboximidamid

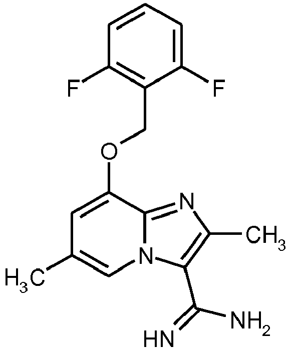

5.47 g (17.46 mmol) 8-[(2,6-Difluorbenzyl)oxy]-2,6-dimethylimidazo[1,2-a]pyridin-3-carbonitril aus Beispiel 30A wurden in Analogie zu Beispiel 26A umgesetzt. Es wurden 1.28 g (22 % d. Th.) der Titelverbindung erhalten.
LC-MS (Methode 1): Rₜ = 0.60 min
MS (ESpos): m/z = 331.3 (M+H)⁺
¹H-NMR (400 MHz, DMSO-d₆): δ = 2.35 (s, 3 H), 2.43 (s, 3 H), 5.31 (s, 2 H), 7.06 (s, 1 H), 7.24 (t, 2 H), 7.54 - 7.65 (m, 1 H), 8.02 (s, 1 H), 9.25 (br. s, 3 H).

### Beispiel 32A

### 2-Methyl-2-nitropropyltrifluormethansulfonat

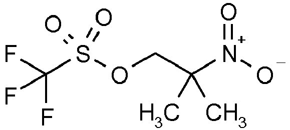

1.0 g (8.40 mmol) 2-Methyl-2-nitropropan-1-ol wurden in 20 ml Dichlormethan vorgelegt, mit 1.0 ml (12.59 mmol) Pyridin versetzt, auf 0°C abgekühlt und langsam mit 1.85 ml (10.91 mmol) Trifluormethansulfonsäureanhydrid versetzt. Anschließend wurde 1 h bei 0°C gerührt. Der Reaktionsverlauf wurde durch DC kontrolliert (Cyclohexan/Essigsäureethylester 7/3, Anfärbereagenz Kaliumpermangantfärbereagenz). Die Reaktionslösung wurde je einmal mit Wasser und gesättigter, wässriger Natriumchloridlösung gewaschen. Die organische Phase wurde über Natriumsulfat getrocknet, filtriert und das Filtrat eingeengt. Es wurden 2.18 g der Zielverbindung (99% d. Th.) erhalten. Die Zielverbindung wurde bei -18°C gelagert und ohne weitere Reinigung eingesetzt.
MS (Methode 13):
MS (ESpos): m/z = 269 (M+NH₄)⁺
¹H NMR (400 MHz, DMSO-*d*₆) δ = 1.64 (s, 6 H), 5.13 (s, 2 H).

### Beispiel 33A

### 8-[(2,6-Difluorbenzyl)oxy]-3-(2,5-difluorpyridin-4-yl)-2,6-dimethylimidazo[1,2-a]pyridin

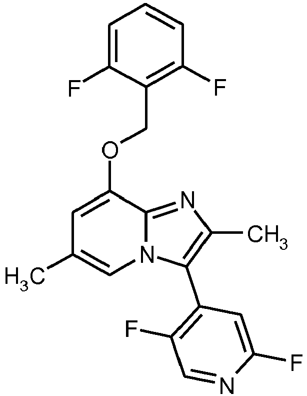

500 mg (1.36 mmol) 3-Brom-8-[(2,6-difluorbenzyl)oxy]-2,6-dimethylimidazo[1,2-a]pyridin (Beispiel 21A) wurden in einem Gemisch aus 9.6 ml Ethanol, 4.8 ml Wasser und 4.8 ml Toluol wurde unter Argon nacheinander mit 541 mg (3.40 mmol) (2,5-Difluorpyridin-4-yl)borsäure, 867 mg (4.09 mmol) Kaliumphosphat und 70 mg (0.14 mmol) Bis(tri-tert-butyl-phosphin)palladium(0) versetzt. Die Suspension wurde mit Argon entgast und dann 30 min bei 120°C gerührt. Nach beendeter Reaktion wurde das Reaktionsgemisch eingeengt, der Rückstand in Essigsäureethylester/Wasser aufgenommen und extrahiert. Die wässrige Phase wurde zweimal mit Essigsäureethylester extrahiert. Die vereinten organischen Phasen wurden über Natriumsulfat getrocknet, filtriert, einrotiert und im Hochvakuum getrocknet. Der Rückstand wurde mittels Kieselgelchromatographie gereinigt (Laufmittel-Gradient: Dichlormethan nach Dichlormethan/Essigester/Methanol = 50/3/1). Es wurden 382 mg der Zielverbindung (68% d. Th.) erhalten.
LC-MS (Methode 1): Rₜ = 0.86 min
MS (ESpos): m/z = 402 (M+H)⁺

### Beispiel 34A

### 6-{8-[(2,6-Difluorbenzyl)oxy]-2-methylimidazo[1,2-a]pyridin-3-yl}-1,3,5-triazin-2,4(1H,3H)-dion

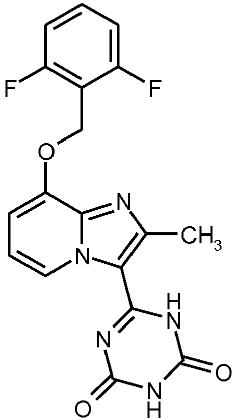

800 mg (2.27 mmol) 8-[(2,6-Difluorbenzyl)oxy]-2-methylimidazo[1,2-a]pyridin-3-carboximidamid Hydrochlorid aus Beispiel 28A wurden in 16 ml Dichlormethan vorgelegt und mit 0.63 ml (4.25 mmol) 1,8-Diazabicyclo(5.4.0)undec-7-en (DBU) und 1.09 g (4.25 mmol) Diphenylimidodicarbonat versetzt. Das Gemisch wurde über Nacht bei RT gerührt. Anschließend wurde die Reaktionslösung mit Dichlormethan verdünnt und zweimal mit Wasser extrahiert. Die vereinigten wässrigen Phasen wurden zweimal mit Dichlormethan extrahiert. Die vereinigten organischen Phasen wurden über Natriumsulfat getrocknet, abfiltriert und einrotiert. Der Rückstand wurde mittels Kieselgelchromatographie gereinigt (Laufmittel-Gradient: Dichlormethan/Methanol = 60/1 nach Dichlormethan/Methanol = 20/1 nach Dichlormethan/Methanol = 10/1). Es wurden 418 mg der Zielverbindung (39% d. Th., Reinheit 81%) erhalten.
LC-MS (Methode 1): Rₜ = 0.65 min
MS (ESpos): m/z = 386 (M+H)⁺

### Beispiel 35A

### 3-(4,6-Dichlor-1,3,5-triazin-2-yl)-8-[(2,6-difluorbenzyl)oxy]-2-methylimidazo[1,2-a]pyridin

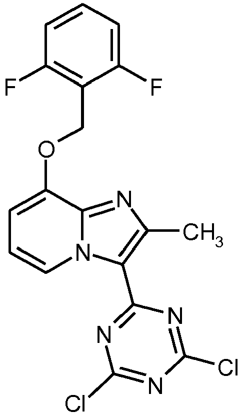

418 mg (0.88 mmol, Reinheit 81%) 6-{8-[(2,6-Difluorbenzyl)oxy]-2-methylimidazo[1,2-a]pyridin-3-yl}-1,3,5-triazin-2,4(1H,3H)-dion aus Beispiel 34A wurden in 3.6 ml (38.24 mmol) Phosphorylchlorid suspendiert und anschließend wurde das Gemisch über Nacht bei 120°C gerührt. Zur Aufarbeitung wurden ca. 50 ml Wasser auf 60°C erwärmt. Die noch warme Reaktionslösung wurde vorsichtig unter starkem Rühren hinzugetropft und 1 h ausgerührt. Anschließend wurde diese Suspension auf RT abgekühlt und mit Dichlormethan zweimal extrahiert. Die vereinigten organischen Phasen wurden über Natriumsulfat getrocknet, filtriert und eingedampft. Der Rückstand wurde in Dichlormethan und wenig Methanol aufgenommen und mittels Kieselgelchromatographie gereinigt (Laufmittel-Gradient: Cyclohexan/Essigester = 10/1 nach Cyclohexan/Essigester = 5/1 nach Cyclohexan/Essigester = 2/1). Es wurden 120 mg der Zielverbindung (30% d. Th.) erhalten.
LC-MS (Methode 1): Rₜ = 1.30 min
MS (ESpos): m/z = 422 (M+H)⁺

### Beispiel 36A

### ent-Benzyl-{1-[(4-chlor-6-{8-[(2,6-difluorbenzyl)oxy]-2-methylimidazo[1,2-a]pyridin-3-yl}-1,3,5-triazin-2-yl)amino]-5,5,5-trifluor-2-methylpentan-2-yl}carbamat Trifluoracetat (Enantiomer B)

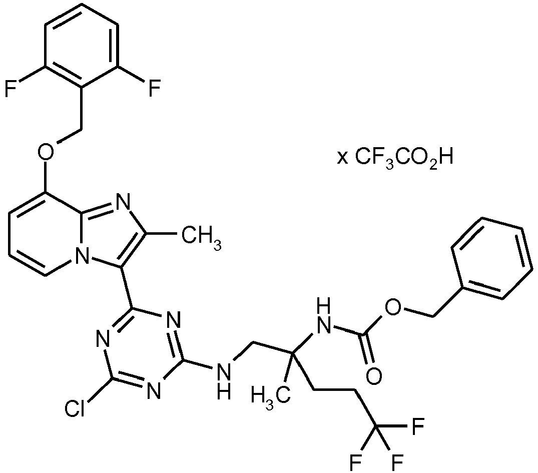

40 mg (0.09 mmol) 3-(4,6-Dichlor-1,3,5-triazin-2-yl)-8-[(2,6-difluorbenzyl)oxy]-2-methylimidazo[1,2-a]pyridin aus Beispiel 35A wurden in 0.35 ml NMP vorgelegt. Bei RT wurden 28 mg (0.09 mmol) *ent*-Benzyl-(1-amino-5,5,5-trifluor-2-methylpentan-2-yl)carbamat (Enantiomer B; Beispiel 409A in WO 2014/068099) zugegeben und das Gemisch wurde 4 h bei Raumtemperatur gerührt. Anschließend wurden 50 µl NMP und 14 mg (0.045 mmol) *ent*-Benzyl-(1-amino-5,5,5-trifluor-2-methylpentan-2-yl)carbamat (Enantiomer B) hinzugegeben und 3.5 h bei Raumtemperatur weiter gerührt. Die Reaktionslösung wurde mit Acetonitril verdünnt, mit Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden vereinigt und einrotiert. Der Rückstand wurde in Dichlormethan und wenig Methanol gelöst und zweimal mit gesättigter, wässriger Natriumhydrogencarbonatlösung gewaschen. Die vereinigten wässrigen Phasen wurden zweimal mit Dichlormethan reextrahiert. Die vereinigten organischen Phasen wurden über Natriumsulfat getrocknet, filtriert und einrotiert. Es wurden 54 mg der Zielverbindung (71% d. Th.) erhalten.
LC-MS (Methode 1): Rₜ = 1.30 min
MS (ESpos): m/z = 690.5 (M+H)⁺

### Beispiel 37A

### 3-Brom-8-(cyclohexylmethoxy)-2-methylimidazo[1,2-a]pyridin

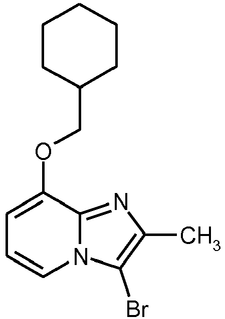

Eine Lösung von 600 mg (2.08 mmol) 8-(Cyclohexylmethoxy)-2-methylimidazo[1,2-a]pyridin-3-carbonsäure aus Beispiel 6A in 8.3 ml DMF wurden mit 524 mg (6.24 mmol) Natriumhydrogencarbonat versetzt. Eine Lösung von 389 mg (2.19 mmol) *N*-Bromsuccinimid in 5.53 ml DMF wurde sehr langsam [2.6 ml/h] mittels Spritzenpumpe bei RT hinzugetropft. Die Reaktionslösung wurde mit Dichlormethan verdünnt und anschließend zweimal mit Wasser gewaschen. Die vereinigten wässrigen Phasen wurden zweimal mit Dichlormethan reextrahiert. Die vereinigten organischen Phasen wurden über Natriumsulfat getrocknet, filtriert und einrotiert. Der Rückstand wurde mit Wasser verrührt, der enthaltene Feststoff wurde abfiltriert und im Hochvakuum getrocknet. Es wurden 515 mg der Titelverbindung (71% d. Th., Reinheit 93%) isoliert.
LC-MS (Methode 1): Rₜ = 0.92 min
MS (ESpos): m/z = 323 (M+H)⁺

### Beispiel 38A

### 5-Chlor-2-nitropyridin-3-ol

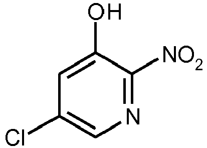

30 g 5-Chlorpyridin-3-ol (232 mmol, 1 Äquivalent) wurden unter Eiskühlung in 228 mL konzentrierter Schwefelsäure gelöst und bei 0 °C langsam mit 24 mL konzentrierter Salpetersäure versetzt. Die Reaktion wurde auf RT erwärmt und über Nacht weitergerührt. Es wurde in ein Eis/Wasser-Gemisch eingerührt und für 30 min nachgerührt. Der Fesstoff wurde abfiltriert, mit kaltem Wasser nachgewaschen und an der Luft getrocknet. Es wurden 33 g (82% d. Th.) der Titelverbindung erhalten und ohne weitere Aufreinigung in die Folgereaktion eingesetzt.
LC-MS (Methode 1): Rₜ = 0.60 min
MS (ESneg): m/z = 172.9/174.9 (M-H)^{- 1}H-NMR (400 MHz, DMSO-d₆): δ = 7.71 (d, 1 H); 8.10 (d, 1 H); 12.14 (br. 1 H).

### Beispiel 39A

### 5-Chlor-3-[(3-fluorpyridin-2-yl)methoxy]-2-nitropyridin

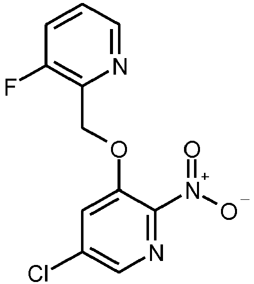

20.0 g (114.6 mmol) 5-Chlor-2-nitropyridin-3-ol aus Beispiel 38A und 56.0 g (171.9 mmol) Cäsiumcarbonat wurden in 319 ml DMF vorgelegt. 17.51 g (120.3 mmol) 2-(Chlormethyl)-3-fluorpyridin (kommerziell erhältlich; zusätzlich beschrieben in: K. Weidmann et al. Journal of Medicinal Chemistry 1992, 35, 438-450; US5593993, 1997; WO2007/2181 A2, 2007) wurden zugegeben und das Reaktionsgemisch wurde über Nacht bei RT gerührt. Es wurden 6.0 g (41.2 mmol) 2-(Chlormethyl)-3-fluorpyridin hinzugegeben und das Gemisch wurde 24 h bei RT gerührt. Anschließend wurden nochmals 6.0 g (41.2 mmol) 2-(Chlormethyl)-3-fluorpyridin und 5.0 g (15.3 mmol) Cäsiumcarbonat hinzugegeben und 12 h bei 60°C gerührt. Das Reaktionsgemisch wurde vorsichtig zu 2.3 l 0.5 M wässriger Salzsäure gegeben. Es wurde dreimal mit je 500 ml Essigsäureethylester extrahiert. Die vereinigten organischen Phasen wurden mit 500 ml gesättigter, wässriger Natriumchloridlösung gewaschen, getrocknet und im Vakuum eingeengt. Das Rohprodukt wurde mittels Kieselgelchromatographie gereinigt (Laufmittel: Cyclohexan/Essigsäureethylester-Gradient: 9/1 bis 7/3). Es wurden 29.8 g (92% d. Th.) der Zielverbindung erhalten.
LC-MS (Methode 1): Rₜ = 0.94 min.
MS (ESIpos): m/z = 284 (M+H)⁺.
¹H-NMR (400 MHz, DMSO-d₆): δ = 5.59 (d, 2H), 7.53 - 7.60 (m, 1H), 7.80 - 7.87 (m, 1H), 8.26 (d, 1H), 8.40 - 8.47 (m, 2H).

### Beispiel 40A

### 5-Chlor-3-[(3-fluorpyridin-2-yl)methoxy]pyridin-2-amin

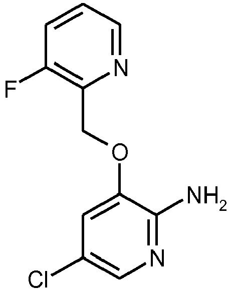

29.8 g (105.1 mmol) 5-Chlor-3-[(3-fluorpyridin-2-yl)methoxy]-2-nitropyridin aus Beispiel 39A wurden unter Argon in 317 ml Ethanol vorgelegt. 18.2 g (325.7 mmol) Eisenpulver wurden zugegeben und das Reaktionsgemisch wurde zum Rückfluss erhitzt. 80.4 ml konz. wässrige Salzsäure wurden langsam zugetropft und das Gemisch wurde weiter 6 h unter Rückfluss erhitzt. Das Reaktionsgemisch wurde mit 33%iger wässriger Ammoniaklösung alkalisch gestellt und anschließend im Vakuum eingeengt. Nach Reinigung durch Kieselgelchromatographie (Laufmittel: Dichlormethan/Methanol-Gradient: 95/5 bis 90/10) wurden 25.0 g (94% d. Th.) der Zielverbindung erhalten.
LC-MS (Methode 1): Rₜ = 0.70 min
MS (ESIpos): m/z = 254 (M+H)⁺
¹H-NMR (400 MHz, DMSO-d₆): δ = 5.27 (d, 2H), 5.87 (br. s, 2H), 7.32 - 7.35 (m, 1H), 7.51 - 7.58 (m, 2H), 7.77 - 7.85 (m, 1H), 7.45 - 7.50 (m, 1H).

### Beispiel 41A

### Ethyl-6-chlor-8-[(3-fluorpyridin-2-yl)methoxy]-2-methylimidazo[1,2-a]pyridin-3-carboxylat

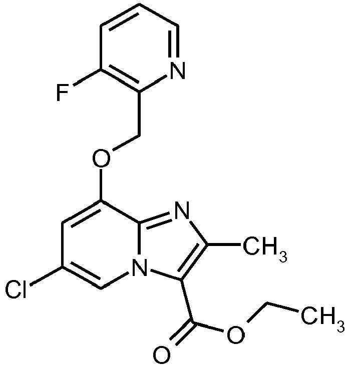

3.00 g (11.83 mmol) 5-Chlor-3-[(3-fluorpyridin-2-yl)methoxy]pyridin-2-amin aus Beispiel 40A und 9.73 g (59.13 mmol) Ethyl-2-chlor-3-oxobutanoat wurden in 72 ml Ethanol gelöst und zusammen mit 4.5 g 3 Å Molsieb unter Rückfluss für 6 Tage gerührt. Das Gemisch wurde abgekühlt, filtriert und das Filtrat im Vakuum eingeengt. Der erhaltene Rückstand wurde mittels Kieselgel-Chromatographie gereinigt (Laufmittel: Cyclohexan/Essigsäureethylester-Gradient = 4/1 nach 2/1). Es wurden 2.0 g (46% d. Th.) der Zielverbindung erhalten.
LC-MS (Methode 1): Rₜ = 1.07 min
MS (ESIpos): m/z = 364 (M+H)⁺
¹H-NMR (400 MHz, DMSO-d₆): δ = 1.36 (t, 3H), 2.56 (s, 3H; überlagert mit Lösungsmittelpeak), 4.37 (q, 2H), 5.48 (d, 2H), 7.36 (d, 1H), 7.57 - 7.63 (m, 1H), 7.83 - 7.90 (m, 1H), 8.50 (d, 1H), 8.92 (d, 1H).

### Beispiel 42A

### 6-Chlor-8-[(3-fluorpyridin-2-yl)methoxy]-2-methylimidazo[1,2-a]pyridin-3-carbonsäure

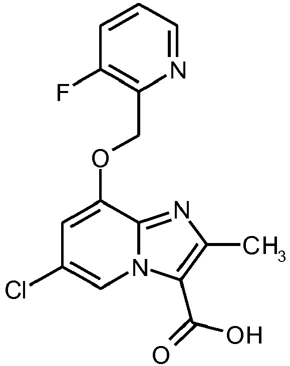

2.0 g (5.62 mmol) Ethyl-6-chlor-8-[(3-fluorpyridin-2-yl)methoxy]-2-methylimidazo[1,2-a]pyridin-3-carboxylat aus Beispiel 41A wurden in 110 ml THF/Methanol (5/1) mit 28.1 ml (28.1 mmol) 1 M wässriger Lithiumhydroxid-Lösung versetzt und 2.5 h bei 40°C gerührt. Das auf RT abgekühlte Reaktionsgemisch wurde mit 6 N wässriger Salzsäure auf ca. pH 4 gestellt, das Lösungsmittel zur Hälfte eingeengt, der ausgefallene Feststoff abgesaugt und im Vakuum getrocknet. Man erhielt 1.97 g (102% d. Th.) der Zielverbindung (möglicherweise anteilig als Hydrochlorid-Salz).
LC-MS (Methode 1): Rₜ = 0.65 min
MS (ESIpos): m/z = 336 (M+H)⁺
¹H-NMR (400 MHz, DMSO-d₆): δ = 5.43 - 5.51 (m, 2H), 7.32 (d, 1H), 7.57 - 7.63 (m, 1H), 7.83 - 7.91 (m, 1H), 8.48 - 8.54 (m, 1H), 8.96 - 9.00 (m, 1H), 13.36 (br. s, 1H), [weiteres Signal unter Lösungsmittelpeak].

### Beispiel 43A

### 3-Brom-6-chlor-8-[(3-fluorpyridin-2-yl)methoxy]-2-methylimidazo[1,2-a]pyridin

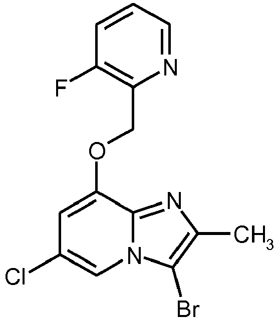

Eine Lösung von 200 mg (0.60 mmol) 6-Chlor-8-[(3-fluorpyridin-2-yl)methoxy]-2-methylimidazo[1,2-a]pyridin-3-carbonsäure aus Beispiel 42A in 2.4 ml DMF wurden mit 150 mg (1.79 mmol) Natriumhydrogencarbonat versetzt. Eine Lösung von 111 mg (0.63 mmol) *N-*Bromsuccinimid in 1.6 ml DMF wurde sehr langsam [2.6 ml/h] mittels Spritzenpumpe bei RT hinzugetropft. Die Reaktionslösung wurde mit Dichlormethan verdünnt und anschließend zweimal mit Wasser gewaschen. Die vereinigten wässrigen Phasen wurden zweimal mit Dichlormethan extrahiert. Die vereinigten organischen Phasen wurden über Natriumsulfat getrocknet, filtriert und einrotiert. Der Rückstand wurde mit Wasser verrührt, der enthaltene Feststoff wurde abfiltriert und im Hochvakuum getrocknet. Es wurden 156 mg der Titelverbindung (71% d. Th.) isoliert.
LC-MS (Methode 1): Rₜ = 0.95 min
MS (ESpos): m/z = 370 (M+H)⁺

### Ausführungsbeispiele:

### Beispiel 1

### N-Benzyl-5-{8-[(2,6-difluorbenzyl)oxy]-2-methylimidazo[1,2-a]pyridin-3-yl}pyridin-2-amin

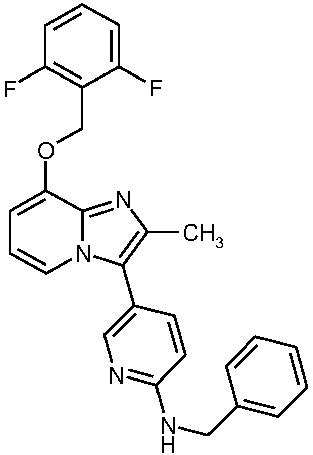

31 mg (0.10 mmol) N-Benzyl-5-(4,4,5,5-tetramethyl-1,3,2-dioxaborolan-2-yl)pyridin-2-amin in 0.6 ml 1,4-Dioxan wurden mit 35 mg (0.10 mmol) 3-Brom-8-[(2,6-difluorbenzyl)oxy]-2-methylimidazo[1,2-a]pyridin (Beispiel 19A), 5.8 mg (0.005 mmol) Tetrakis(triphenylphosphine)palladium(0), 22 mg (0.20 mmol) Natriumcarbonat und 0.2 ml Wasser versetzt und es wurde bei 85°C über Nacht geschüttelt. Nach beendeter Reaktionszeit wurde die Reaktionslösung filtriert, 1,4-Dioxan im Vakuum entfernt, der Rückstand in wenig DMSO gelöst und über präparative HPLC (Methode 11) gereinigt. Es wurden 0.7 mg (2% d. Th.) der Titelverbindung erhalten.
LC-MS (Methode 12): Rₜ = 0.89 min
MS (ESpos): m/z = 457.3 (M+H)⁺

In Analogie zu Beispiel 1 wurden die in Tabelle 1 gezeigten Beispielverbindungen hergestellt, indem 3-Brom-8-[(2,6-difluorbenzyl)oxy]-2-methylimidazo[1,2-a]pyridin (Beispiel 19A) mit den entsprechenden Boronsäuren oder Boronsäureester [4,4,5,5-tetramethyl-1,3,2-dioxaborolanyl = Boronsäure-Pinakolester] umgesetzt wurde.

**Tabelle 1:**

| **Beispiel Nr.** | **IUPAC-Name** | **Analytische Daten** |
|---|---|---|
| | **Struktur** | |
| | **(Ausbeute)** | |
| **2** | 5-{8-[(2,6-Difluorbenzyl)oxy]-2-methylimidazo[1,2-a]pyridin-3-yl}-*N*-(2-methoxyethyl)pyridin-2-amin | LC-MS (Methode 12): Rₜ = 0.75 min |
| | 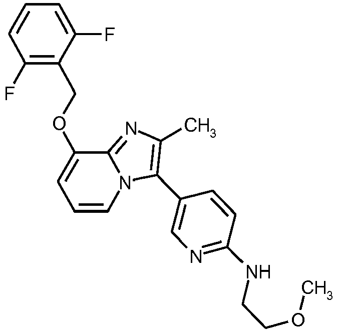 | MS (ESpos): m/z = 425 (M+H)⁺ |
| | Es wurde der Boronsäure-Pinakolester [1]eingesetzt. | |
| | (4% d. Th., Reinheit 100%) | |
| **3** | 5-{8-[(2,6-Difluorbenzyl)oxy]-2-methylimidazo[1,2-a]pyridin-3-yl}-*N*-ethyl-N-methylpyridin-2-amin | LC-MS (Methode 12): Rₜ = 0.80 min |
| | 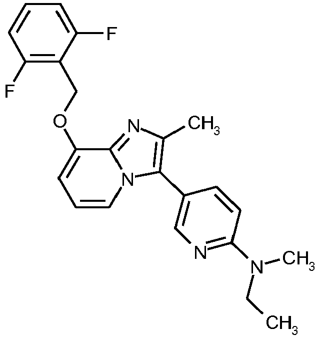 | MS (ESpos): m/z = 409.2 (M+H)⁺ |
| | Es wurde die Boronsäure [2] eingesetzt. | |
| | (19% d. Th., Reinheit 77%) | |
| **4** | 8-[(2,6-Difluorbenzyl)oxy]-3-[6-(2,6-dimethylmorpholin-4-yl)pyridin-3-yl]-2-methylimidazo[1,2-a]pyridin | LC-MS (Methode 12): Rₜ = 0.94 min |
| | 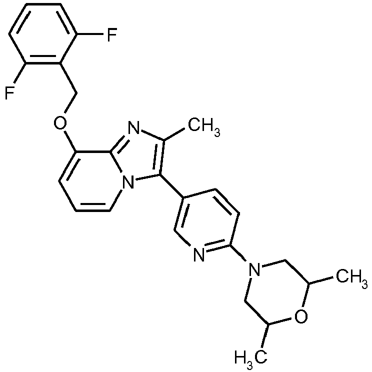 | MS (ESpos): m/z = 465.2 (M+H)⁺ |
| | Es wurde der Boronsäure-Pinakolester [3] eingesetzt. | |
| | (19% d. Th., Reinheit 83%) | |
| **5** | 5-{8-[(2,6-Difluorbenzyl)oxy]-2-methylimidazo[1,2-a]pyridin-3-yl}-*N*-phenylpyridin-2-amin | LC-MS (Methode 12): Rₜ = 0.94 min |
| | 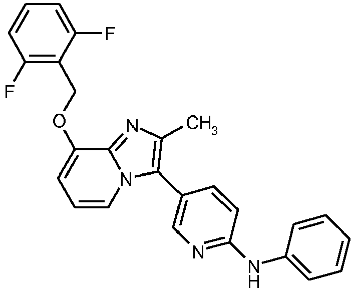 | MS (ESpos): m/z = 443.2 (M+H)⁺ |
| | Es wurde der Boronsäure-Pinakolester [4] eingesetzt. | |
| | (11% .d. Th., Reinheit 82%) | |

[1] Darstellung analog ASTRAZENECA, *Patent:* WO2007186800A1, 2007 aus 5-Brom-N-(2-methoxyethyl)pyridin-2-amin.
[2] Darstellung analog Majo, Vattoly J.; Prabhakaran, Jaya; Mann, J. John; Kumar, J. S. Dileep, Advanced Synthesis and Catalysis, 2003, vol. 345, p. 620 - 624 aus 5-Brom-N-ethyl-N-methylpyridin-2-amin.
[3] Darstellung analog XCOVERY, INC. Patent: WO2008/88881 A1, 2008 aus 4-(5-Brompyridin-2-yl)-2,6-dimethylmorpholin.
[4] Darstellung analog BioMarin IGA, Ltd.; Wren, Stephen Paul; Wynne, Graham Michael; Lecci, Cristina; Wilson, Francis Xavier; Davis, Paul James; Patent: WO2010/57833 A1, 2010 aus 5-Brom-N-phenylpyridin-2-amin.

### Beispiel 6

### 8-[(2,6-Difluorbenzyl)oxy]-2-methyl-3-[2-(4-methylpiperazin-1-yl)pyrimidin-5-yl]imidazo[1,2-a]pyridin

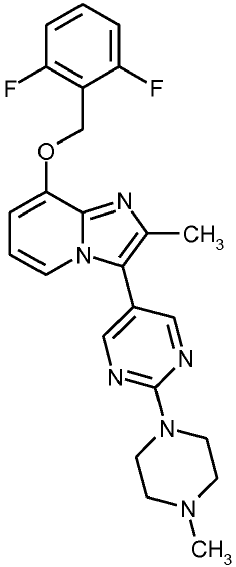

125 mg (0.35 mmol) 3-Brom-8-[(2,6-difluorbenzyl)oxy]-2-methylimidazo[1,2-a]pyridin (Beispiel 19A), 118 mg (0.39 mmol) 2-(4-Methylpiperazin-1-yl)-5-(4,4,5,5-tetramethyl-1,3,2-dioxaborolan-2-yl)pyrimidin und 119 mg (1.42 mmol) Natriumhydrogencarbonat wurden mit 14 mg (0.02 mmol) 1,1'- Bis-(diphenylphosphino)ferrocen-palladium(II)chlorid Dichlormethan-Komplex vorgelegt und mit einer entgasten 3:1 Mischung aus 1,2-Dimethoxyethan und Wasser versetzt. Es wurde über Nacht bei 80°C gerührt. Die Reaktionslösung wurde mit Acetonitril/Wasser verdünnt, über einen Millipore-Filter filtriert und mittels präparativer HPLC gereinigt (RP18 Säule, Laufmittel: Acetonitril/Wasser-Gradient unter Zusatz von 0.1% TFA). Die Produktfraktionen wurden eingedampft, der Rückstand wurde in Dichlormethan aufgenommen und zweimal mit gesättigter, wässriger Natriumhydrogencarbonatlösung gewaschen. Die wässrige Phase wurde noch zweimal mit Dichlormethan extrahiert. Die vereinigten organischen Phasen wurden über Natriumsulfat getrocknet, filtriert, eingeengt und lyophilisiert. Es wurden 77 mg der Zielverbindung (48% d. Th.) erhalten.
LC-MS (Methode 16): Rₜ = 0.46 min
MS (ESpos): m/z = 451 (M+H)⁺

### Beispiel 7

### 6-{8-[(2,6-Difluorbenzyl)oxy]-2,6-dimethylimidazo[1,2-a]pyridin-3-yl}-1,3,5-triazin-2,4-diamin

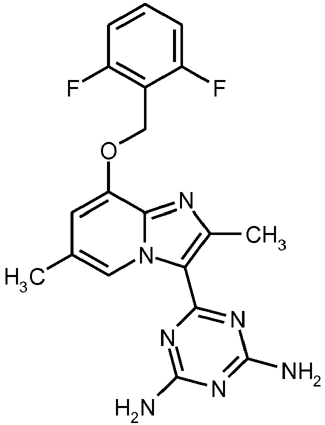

43 mg (0.31 mmol) Imidodikohlenstoffimiddiamid-Hydrochlorid [Biguanid-Hydrochlorid] wurden unter Argon in 0.93 ml abs. Methanol vorgelegt, mit 148 mg (0.16 ml, 0.69 mmol) Natriummethylat (25%ig in Methanol) versetzt und 30 min bei 50°C gerührt. Anschließend wurden 75 mg (0.21 mmol) Ethyl-8-[(2,6-difluorbenzyl)oxy]-2,6-dimethylimidazo[1,2-a]pyridin-3-carboxylat aus Beispiel 10A zugegeben und über Nacht unter Rückfluss gerührt. Nach dem Abkühlen wurde das Reaktionsgemisch auf Wasser gegossen und der entstandene Feststoff wurde abfiltriert und getrocknet. Das Rohprodukt wurde in Acetonitril/Wasser/TFA gelöst und mittels präparativer HPLC gereinigt (RP18 Säule, Laufmittel: Acetonitril/Wasser-Gradient unter Zusatz von 0.1% TFA). Die produkthaltigen Fraktionen wurden eingeengt, in Dichlormethan gelöst und mit gesättigter, wässriger Natriumhydrogencarbonatlösung gewaschen. Die wässrige Phase wurde zweimal mit Dichlormethan unter Zusatz von etwas Methanol extrahiert. Die vereinten organischen Phasen wurden über Natriumsulfat getrocknet, filtriert, das Filtrat eingeengt und der Rückstand mit Wasser ausgerührt. Der Niederschlag wurde abfiltriert und im Hochvakuum getrocknet. Es wurden 6.3 mg der Zielverbindung (7.6% d. Th.) erhalten.
LC-MS (Methode 1): Rₜ = 0.73 min
MS (ESpos): m/z = 398 (M+H)⁺
¹H-NMR (400 MHz, DMSO-*d*₆) δ = 2.38 (s, 3 H), 2.73 (s, 3 H), 5.29 (s, 2 H), 6.72 (br. s, 4 H), 6.92 (s, 1 H), 7.22 (t, 2 H), 7.54 - 7.64 (m, 1 H), 9.60 (s, 1 H).

### Beispiel 8

### 6-{8-[(2,6-Difluorbenzyl)oxy]-2,6-dimethylimidazo[1,2-a]pyridin-3-yl}-N,N-dimethyl-1,3,5-triazin-2,4-diamin

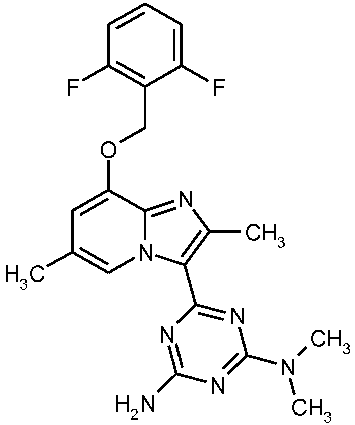

103 mg (0.62 mmol) *N,N*-Dimethylimidodikohlenstoffimiddiamid-Hydrochlorid [Methformin-Hydrochlorid] wurden unter Argon in 1.87 ml abs. Methanol vorgelegt, mit 297 mg (0.31 ml, 1.38 mmol) Natriummethylat (25%ig in Methanol) versetzt und 30 min bei 50°C gerührt. Anschließend wurden 150 mg (0.42 mmol) Ethyl-8-[(2,6-difluorbenzyl)oxy]-2,6-dimethylimidazo[1,2-a]pyridin-3-carboxylat aus Beispiel 10A zugegeben und über Nacht unter Rückfluss gerührt. Nach dem Abkühlen wurde das Reaktionsgemisch auf Wasser gegossen und der entstandene Feststoff wurde abgesaugt und getrocknet. Das Rohprodukt wurde in Acetonitril/Wasser/TFA gelöst und mittels präparativer HPLC gereinigt (RP18 Säule, Laufmittel: Acetonitril/Wasser-Gradient unter Zusatz von 0.1% TFA). Das Rohprodukt wurde nochmals mittels Kieselgelchromatographie gereinigt (Laufmittel: Cyclohexan/Essigsäureethylester: 2/1 dann 1/1). Es wurden 31 mg der Zielverbindung (16% d. Th.; Reinheit 92%) erhalten.
LC-MS (Methode 1): Rₜ = 0.85 min
MS (ESpos): m/z = 426 (M+H)⁺
¹H-NMR (400 MHz, DMSO-*d*₆) δ = 2.37 (s, 3 H), 2.72 (s, 3 H), 3.05 - 3.20 (m, 6 H), 5.29 (s, 2 H), 6.78 - 7.09 (m, 3 H), 7.22 (t, 2 H), 7.54 - 7.64 (m, 1 H), 9.62 (s, 1 H).

### Beispiel 9

### 6-{8-[(2,6-Difluorbenzyl)oxy]-2,6-dimethylimidazo[1,2-a]pyridin-3-yl}-N-propyl-1,3,5-triazin-2,4-diamin

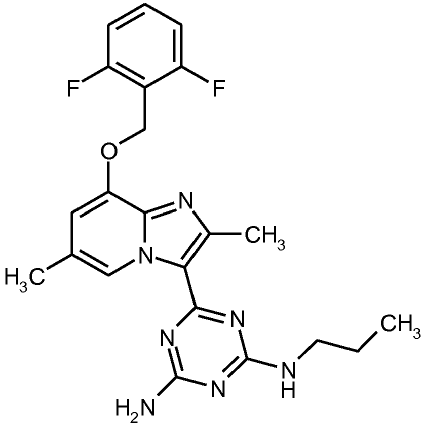

89 mg (0.62 mmol) 1-Amino(propylamino)methylen]guanidin wurden unter Argon in 1.87 ml abs. Methanol vorgelegt, mit 297 mg (0.31 ml, 1.38 mmol) Natriummethylat (25%ig in Methanol) versetzt und 30 min bei 50°C gerührt. Anschließend wurden 150 mg (0.42 mmol) Ethyl-8-[(2,6-difluorbenzyl)oxy]-2,6-dimethylimidazo[1,2-a]pyridin-3-carboxylat aus Beispiel 10A zugegeben und über Nacht unter Rückfluss gerührt. Nach dem Abkühlen wurde das Reaktionsgemisch auf Wasser gegossen und der entstandene Feststoff wurde abgesaugt und getrocknet. Das Rohprodukt wurde in Acetonitril/Wasser/TFA gelöst und mittels präparativer HPLC gereinigt (RP18 Säule, Laufmittel: Acetonitril/Wasser-Gradient unter Zusatz von 0.1% TFA). Das Rohprodukt wurde nochmals mittels Kieselgelchromatographie gereinigt (Laufmittel: Cyclohexan/Essigsäureethylester: 2/1 dann 1/1). Es wurden 36 mg der Zielverbindung (18% d. Th.; Reinheit 93%) erhalten.
LC-MS (Methode 1): Rₜ = 0.87 min
MS (ESpos): m/z = 440 (M+H)⁺
¹H-NMR (400 MHz, DMSO-*d*₆) δ = 0.91 (t, 3 H), 1.49 - 1.63 (2 H), 2.38 (s, 3 H), 2.72 (s, 3 H), 3.20 - 3.32 (m, 2 H), 5.30 (s, 2 H), 6.60 - 6.90 (m, 2 H), 6.91 - 6.98 (m, 1 H), 7.20 - 7.32 (m, 2 H), 7.55 - 7.63 (m, 1 H), 9.58 - 9.64 (m, 1 H).

### Beispiel 10

### N¹-(4-{8-[(2,6-Difluorbenzyl)oxy]-2,6-dimethylimidazo[1,2-a]pyridin-3-yl}-5-fluorpyridin-2-yl)-2-methylpropan-1,2-diamin

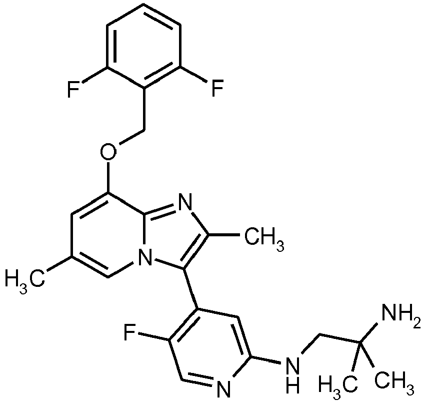

30 mg (0.072 mmol) 8-[(2,6-Difluorbenzyl)oxy]-3-(2,5-difluorpyridin-4-yl)-2,6-dimethylimidazo[1,2-a]pyridin aus Beispiel 33A wurden in 0.27 ml NMP vorgelegt. Bei Raumtemperatur wurden 32 mg (0.36 mmol) 2-Methylpropan-1,2-diamin zugegeben und das Gemisch wurde 2 h bei 150°C in einem geschlossenem Gefäß im Ölbad gerührt. Anschließend wurden nochmals 13 mg (0.15 mmol) 2-Methylpropan-1,2-diamin zugegeben und das Gemisch wurde 3 h bei 150°C in einem geschlossenem Gefäß in der Mikrowelle gerührt. Anschließend wurden nochmals 19 mg (0.22 mmol) 2-Methylpropan-1,2-diamin zugegeben und das Gemisch wurde 3 h bei 150°C in einem geschlossenem Gefäß in der Mikrowelle gerührt. Es wurden 0.27 ml NMP hinzugegeben und 5 h bei 180°C in der Mikrowelle gerührt. Die Reaktionslösung wurde mit Acetonitril/Wasser verdünnt, mit TFA versetzt und mittels präparativer HPLC gereinigt (RP18 Säule, Laufmittel: Acetonitril/Wasser-Gradient unter Zusatz von 0.1% TFA). Die Produktfraktionen wurden eingedampft, in Dichlormethan gelöst und zweimal mit gesättigter, wässriger Natriumhydrogencarbonatlösung gewaschen. Die vereinigten wässriger Phasen wurden zweimal mit Dichlormethan extrahiert. Die vereinigten organischen Phasen wurden über Natriumsulfat getrocknet, filtriert und einrotiert. Es wurden 9 mg der Zielverbindung (27% d. Th.) erhalten.
LC-MS (Methode 1): Rₜ = 0.64 min
MS (ESpos): m/z = 470 (M+H)⁺
¹H-NMR (400 MHz, DMSO-*d*₆) δ = 1.05 (s, 6 H), 1.75 (br. s, 2 H), 2.24 - 2.29 (m, 6 H), 3.18 (d, 2 H), 5.30 (s, 2 H), 6.53 (t, 1 H), 6.68 (d, 1 H), 6.84 (s, 1 H), 7.19 - 7.28 (m, 2 H), 7.55 - 7.65 (m, 2 H), 8.08 (s, 1 H).

### Beispiel 11

### N¹-(4-Chlor-6-{8-[(2,6-difluorbenzyl)oxy]-2-methylimidazo[1,2-a]pyridin-3-yl}-1,3,5-triazin-2-yl)-2-methylpropan-1,2-diamin

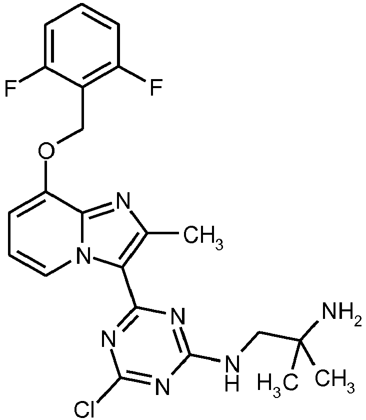

25 mg (0.058 mmol) 3-(4,6-Dichlor-1,3,5-triazin-2-yl)-8-[(2,6-difluorbenzyl)oxy]-2-methylimidazo[1,2-a]pyridin aus Beispiel 35A wurden in 0.22 ml NMP vorgelegt. Bei Raumtemperatur wurden 5.2 mg (0.058 mmol) 2-Methylpropan-1,2-diamin zugegeben und das Gemisch wurde 1.5 h bei RT in einem geschlossenem Gefäß gerührt. Die Reaktionslösung wurde mit Acetonitril/Wasser verdünnt, mit TFA versetzt und mittels präparativer HPLC gereinigt (RP18 Säule, Laufmittel: Acetonitril/Wasser-Gradient unter Zusatz von 0.1% TFA). Die Produktfraktionen wurden eingedampft, in Dichlormethan gelöst und zweimal mit gesättigter, wässriger Natriumhydrogencarbonatlösung gewaschen. Die vereinigten wässriger Phasen wurden zweimal mit Dichlormethan extrahiert. Die vereinigten organischen Phasen wurden über Natriumsulfat getrocknet, filtriert und einrotiert. Es wurden 15 mg der Zielverbindung (55% d. Th.) erhalten.
LC-MS (Methode 1): Rₜ = 0.70 min
MS (ESpos): m/z = 474 (M+H)⁺
¹H-NMR (400 MHz, DMSO-*d*₆) δ = 1.07 (s, 6 H), 1.69 (br. s, 2 H), 2.73 - 2.77 (m, 3 H), 3.23 - 3.40 (m, 2 H; überlagert mit Lösungsmittelpeak), 5.33 (s, 2 H), 7.05 - 7.28 (m, 4 H), 7.55 - 7.64 (m, 1 H), 8.48 (br. s, 1 H), 9.48 und 9.68 (je d, zusammen 1 H).

### Beispiel 12

### ent-N¹-(4-{8-[(2,6-Difluorbenzyl)oxy] -2-methylimidazo[1,2-a]pyridin-3-yl} -1,3,5-triazin-2-yl)-5,5,5-trifluor-2-methylpentan-1,2-diamin (Enantiomer B)

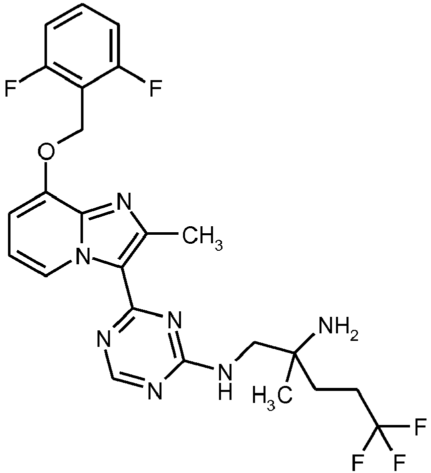

54 mg (0.07 mmol) *ent*-Benzyl-{1-[(4-chlor-6-{8-[(2,6-difluorbenzyl)oxy]-2-methylimidazo[1,2-a]pyridin-3-yl}-1,3,5-triazin-2-yl)amino]-5,5,5-trifluor-2-methylpentan-2-yl}carbamat Trifluoracetat (Enantiomer B) aus Beispiel 36A wurden in 1.7 ml Ethanol gelöst und mit 5 µl (0.07 mmol) Trifluoressigsäure versetzt. Unter Argon wurden 2 mg Palladium/Aktivkohle (10%ig) hinzugegeben. Das Reaktionsgemisch wurde 4.5 h bei RT unter Normaldruck hydriert. Das Reaktionsgemisch wurde über einen Millipore-Filter filtriert, gut mit Ethanol gewaschen und einrotiert. Der Rückstand wurde erneut in 1.7 ml Ethanol gelöst, unter Argon mit 5 µl (0.07 mmol) Trifluoressigsäure und 2 mg Palladium/Aktivkohle (10%ig) versetzt und 1.5 h bei Normaldruck hydriert. Das Reaktionsgemisch wurde über einen Millipore-Filter filtriert, gut mit Ethanol gewaschen und einrotiert. Der Rückstand wurde in Acetonitril aufgenommen, mit Wasser/TFA versetzt mittels präparativer HPLC gereinigt (RP18 Säule, Laufmittel: Acetonitril/Wasser-Gradient unter Zusatz von 0.1% TFA). Die Produktfraktionen wurden vereinigt und einrotiert. Der Rückstand wurde in Dichlormethan gelöst und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingedampft. Es wurden 19 mg der Zielverbindung (54% d. Th.) erhalten.
LC-MS (Methode 1): Rₜ = 0.68 min
MS (ESpos): m/z = 522 (M+H)⁺
¹H-NMR (400 MHz, DMSO-*d*₆) δ = 1.01 (s, 3 H), 1.48 - 1.69 (m, 4 H), 2.21 - 2.48 (m, 2 H), 2.77 (s, 3 H), 3.26 - 3.42 (m, 2 H; überlagert mit Lösungsmittelpeak), 5.33 (s, 2 H), 7.00 - 7.09 (m, 1 H), 7.10 - 7.16 (m, 1 H), 7.19 - 7.28 (m, 2 H), 7.55 - 7.64 (m, 1 H), 7.96 - 8.08 (m, 1 H), 8.58 (d, 1 H), 9.61 und 9.76 (je d, zusammen 1 H).

### Beispiel 13

### 8-[(2,6-Difluorbenzyl)oxy]-2,6-dimethyl-3-[2-(piperazin-1-yl)pyridin-4-yl]imidazo[1,2-a]pyridin

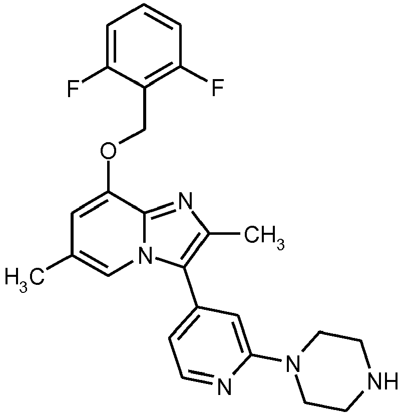

60 mg (0.16 mmol) 3-Brom-8-[(2,6-difluorbenzyl)oxy]-2,6-dimethylimidazo[1,2-a]pyridin (Beispiel 21A) wurden in einem Gemisch aus 1.2 ml Ethanol, 0.6 ml Wasser und 0.6 ml Toluol unter Argon nacheinander mit 71 mg (0.25 mmol) 1-[4-(4,4,5,5-Tetramethyl-1,3,2-dioxaborolan-2-yl)pyridin-2-yl]piperazin, 104 mg (0.49 mmol) Kaliumphosphat und 8 mg (0.016 mmol) Bis(tri-tert-butyl-phosphin)palladium(0) versetzt. Die Suspension wurde mit Argon entgast und dann 40 min bei 120°C gerührt. Nach beendeter Reaktion wurde das Reaktionsgemisch eingeengt, der Rückstand in Essigsäureethylester/Wasser aufgenommen und extrahiert. Die wässrige Phase zweimal mit Essigsäureethylester extrahiert. Die vereinten organischen Phasen wurden über Natriumsulfat getrocknet, filtriert, einrotiert und im Hochvakuum getrocknet. Der Rückstand wurde mittels präparativer HPLC gereinigt (RP18 Säule, Laufmittel: Acetonitril/Wasser-Gradient unter Zusatz von 0.1% TFA). Die Produktfraktionen wurden vereinigt und einrotiert. Der Rückstand wurde in Dichlormethan und wenig Methanol gelöst und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingedampft. Es wurden 31 mg der Zielverbindung (41% d. Th.) erhalten.
LC-MS (Methode 1): Rₜ = 0.54 min
MS (ESpos): m/z = 450 (M+H)⁺

### Vergleichsbeispiel Beispiel 14

### 8-[(2,6-Difluorbenzyl)oxy]-2,6-dimethyl-3-[3-(pyrrolidin-1-yl)phenyl]imidazo[1,2-a]pyridin

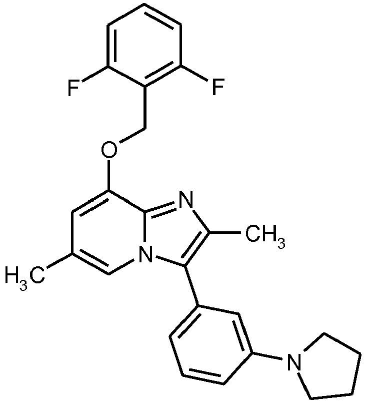

60 mg (0.16 mmol) 3-Brom-8-[(2,6-difluorbenzyl)oxy]-2,6-dimethylimidazo[1,2-a]pyridin (Beispiel 21A) wurden in einem Gemisch aus 1.2 ml Ethanol, 0.6 ml Wasser und 0.6 ml Toluol unter Argon nacheinander mit 78 mg (0.41 mmol) [3-(Pyrrolidin-1-yl)phenyl]borsäure, 104 mg (0.49 mmol) Kaliumphosphat und 8 mg (0.016 mmol) Bis(tri-tert-butyl-phosphin)palladium(0) versetzt. Die Suspension wurde mit Argon entgast und dann 30 min bei 120°C gerührt. Nach beendeter Reaktion wurde das Reaktionsgemisch eingeengt, der Rückstand in Essigsäureethylester/Wasser aufgenommen und extrahiert. Die wässrige Phase wurde zweimal mit Essigsäureethylester extrahiert. Die vereinten organischen Phasen wurden über Natriumsulfat getrocknet, filtriert, einrotiert und im Hochvakuum getrocknet. Der Rückstand wurde mittels präparativer HPLC gereinigt (RP18 Säule, Laufmittel: Acetonitril/Wasser-Gradient unter Zusatz von 0.1% TFA). Die Produktfraktionen wurden vereinigt und einrotiert. Der Rückstand wurde in Dichlormethan gelöst und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingedampft. Es wurden 32 mg der Zielverbindung (44% d. Th.) erhalten.
LC-MS (Methode 1): Rₜ = 1.00 min
MS (ESpos): m/z = 434 (M+H)⁺
¹H-NMR (500 MHz, DMSO-*d*₆) δ = 1.93 - 2.02 (m, 4 H), 2.24 (s, 3 H), 2.37 (s, 3 H), 3.23 - 3.32 (m, 4 H; überlagert mit Lösungsmittelpeak), 5.29 (s, 2 H), 6.54 (s, 1 H), 6.62 (d, 1 H), 6.66 (d, 1 H), 6.72 (s, 1 H), 7.19 - 7.28 (m, 2 H), 7.32 (t, 1 H), 7.55 - 7.63 (m, 1 H), 7.71 (s, 1 H).

### Vergleichsbeispiel Beispiel 15

### N-(3-{8-[(2,6-Difluorbenzyl)oxy]-2,6-dimethylimidazo[1,2-a]pyridin-3-yl}phenyl)acetamid

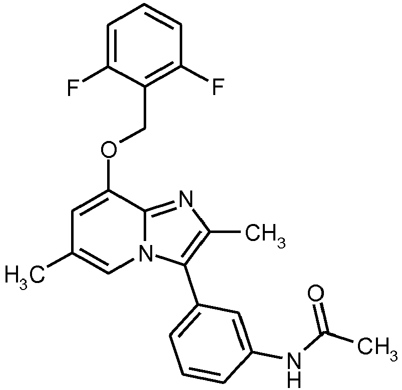

60 mg (0.16 mmol) 3-Brom-8-[(2,6-difluorbenzyl)oxy]-2,6-dimethylimidazo[1,2-a]pyridin (Beispiel 21A) wurden in einem Gemisch aus 1.2 ml Ethanol, 0.6 ml Wasser und 0.6 ml Toluol unter Argon nacheinander mit 44 mg (0.25 mmol) (3-Acetamidophenyl)borsäure, 104 mg (0.49 mmol) Kaliumphosphat und 8 mg (0.016 mmol) Bis(tri-tert-butyl-phosphin)palladium(0) versetzt. Die Suspension wurde mit Argon entgast und dann 30 min bei 120°C gerührt. Nach beendeter Reaktion wurde das Reaktionsgemisch eingeengt, der Rückstand in Essigsäureethylester/Wasser aufgenommen und extrahiert. Die wässrige Phase wurde zweimal mit Essigsäureethylester extrahiert. Die vereinten organischen Phasen wurden über Natriumsulfat getrocknet, filtriert, einrotiert und im Hochvakuum getrocknet. Der Rückstand wurde mittels präparativer HPLC gereinigt (RP18 Säule, Laufmittel: Acetonitril/Wasser-Gradient unter Zusatz von 0.1% TFA). Die Produktfraktionen wurden vereinigt und einrotiert. Der Rückstand wurde in Dichlormethan gelöst und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingedampft. Es wurden 35 mg der Zielverbindung (48% d. Th.) erhalten.
LC-MS (Methode 1): Rₜ = 0.76 min
MS (ESpos): m/z = 422 (M+H)⁺

### Beispiel 16

### 2,6-Dimethyl-3-[4-(pyrrolidin-1-yl)pyrimidin-2-yl]-8-[(2,3,6-trifluorbenzyl)oxy]imidazo[1,2-a]pyridin Trifluoracetat

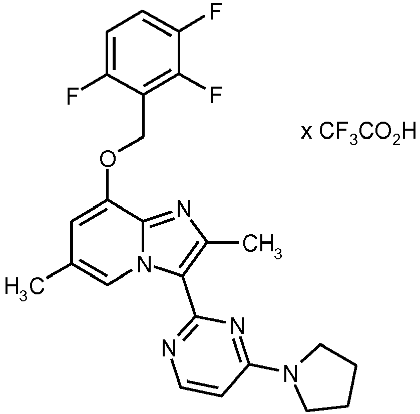

80 mg (0.26 mmol) 2,6-Dimethyl-8-[(2,3,6-trifluorbenzyl)oxy]imidazo[1,2-a]pyridin (Beispiel 22A), 39 mg (0.21 mmol) 2-Chlor-4-(pyrrolidin-1-yl)pyrimidin und 26 mg (0.26 mmol) Kaliumacetat wurden in 0.5 ml NMP vorgelegt. Es wurde 5 min Argon durch die Reaktionsmischung geleitet. Anschließend wurden 15 mg (0.01 mmol) Tetrakis-(triphenylphosphin)palladium(0) hinzugegeben und das Gemisch wurde bei 150°C für 12 h in der Mikrowelle gerührt. Das Gemisch wurde erneut mit Argon begast, mit 15 mg (0.01 mmol) Tetrakis(triphenylphosphin)palladium(0) versetzt und 4.5 h bei 150°C in der Mikrowelle gerührt. Der Reaktionsgemisch wurde abgekühlt, mit Wasser/TFA versetzt und mittels präparativer HPLC gereinigt (RP18 Säule, Laufmittel: Acetonitril/Wasser-Gradient unter Zusatz von 0.1% TFA). Es wurden 5.5 mg der Zielverbindung (3.4% d. Th., Reinheit 92%) erhalten.
LC-MS (Methode 1): Rₜ = 0.98 min
MS (ESpos): m/z = 454 (M+H)⁺

### Beispiel 17

### 2-{2,6-Dimethyl-8-[(2,3,6-trifluorbenzyl)oxy]imidazo[1,2-a]pyridin-3-yl}-N,N-dimethylpyrimidin-4-amin

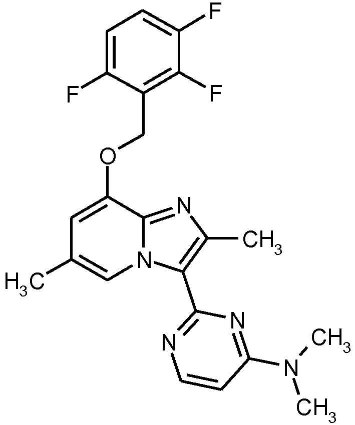

140 mg (0.46 mmol) 2,6-Dimethyl-8-[(2,3,6-trifluorbenzyl)oxy]imidazo[1,2-a]pyridin (Beispiel 22A), 58 mg (0.37 mmol) 2-Chlor-N,N-dimethylpyrimidin-4-amin und 45 mg (0.46 mmol) Kaliumacetat wurden in 0.7 ml NMP vorgelegt. Es wurde 5 min Argon durch die Reaktionsmischung geleitet. Anschließend wurden 26 mg (0.02 mmol) Tetrakis(triphenylphosphin)palladium(0) hinzugegeben und das Gemisch wurde bei 150°C für 12 h in der Mikrowelle gerührt. Der Reaktionsgemisch wurde abgekühlt, mit Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden eingedampft und mittels Kieselgelchromatographie (Laufmittel: Dichlormethan-Methanol-Gradient) gereinigt. Die Produktfraktionen wurden eingedampft und nochmals mittels Dickschichtchromatographie (Laufmittel: Dichlormethan/Methanol/Essigsäureethylester = 10/1/2) gereinigt. Es wurde 1 mg der Zielverbindung (0.3% d. Th., Reinheit 55%) erhalten.
LC-MS (Methode 1): Rₜ = 0.75 min
MS (ESpos): m/z = 428 (M+H)⁺

### Beispiel 18

### N-Benzyl-2-{2,6-dimethyl-8-[(2,3,6-trifluorbenzyl)oxy]imidazo[1,2-a]pyridin-3-yl}pyrimidin-4-amin

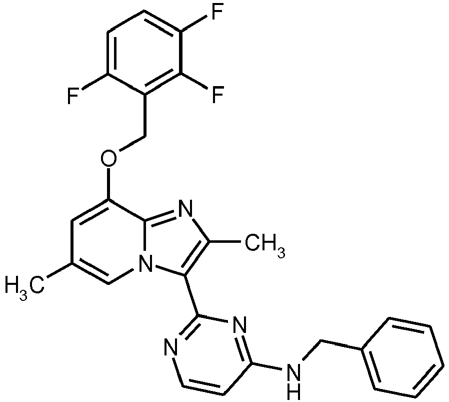

100 mg (0.33 mmol) 2,6-Dimethyl-8-[(2,3,6-trifluorbenzyl)oxy]imidazo[1,2-a]pyridin (Beispiel 22A), 57 mg (0.26 mmol) N-Benzyl-2-chlorpyrimidin-4-amin und 32 mg (0.33 mmol) Kaliumacetat wurden in 0.95 ml NMP vorgelegt. Es wurde 5 min Argon durch die Reaktionsmischung geleitet. Anschließend wurden 38 mg (0.03 mmol) Tetrakis-(triphenylphosphin)palladium(0) hinzugegeben und das Gemisch wurde bei 150°C für 8 h in der Mikrowelle gerührt. Der Reaktionsgemisch wurde abgekühlt, mit Acetonitril/Wasser/TFA versetzt und mittels präparativer HPLC gereinigt (RP18 Säule, Laufmittel: Acetonitril/Wasser-Gradient unter Zusatz von 0.1% TFA). Die Produktfraktionen wurden eingedampft und mittels Dickschichtchromatographie gereinigt (Laufmittel: Dichlormethan/Methanol/Essigsäureethylester = 10/1/2). Die Produktfraktionen wurden eingedampft und nochmals mittels präparativer HPLC gereinigt (Kinetix, 5µ, C18-Säule, Laufmittel: Acetonitril/Wasser (50/50) unter Zusatz von 0.2% TFA). Es wurden 1.5 mg der Zielverbindung (0.6% d. Th., Reinheit 60%) erhalten.
LC-MS (Methode 1): Rₜ = 0.92 min
MS (ESpos): m/z = 490 (M+H)⁺

### Vergleichsbeispiel Beispiel 19

### N-{3-[8-(Cyclohexylmethoxy)-2-methylimidazo[1,2-a]pyridin-3-yl]phenyl} acetamid

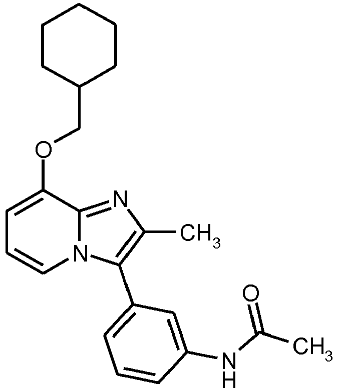

60 mg (0.17 mmol; Reinheit 93%) 3-Brom-8-(cyclohexylmethoxy)-2-methylimidazo[1,2-a]pyridin (Beispiel 37A) wurden in einem Gemisch aus 1.2 ml Ethanol, 0.6 ml Wasser und 0.6 ml Toluol unter Argon nacheinander mit 46 mg (0.26 mmol) (3-Acetamidophenyl)borsäure, 110 mg (0.52 mmol) Kaliumphosphat und 9 mg (0.017 mmol) Bis(tri-tert-butyl-phosphin)palladium(0) versetzt. Die Suspension wurde mit Argon entgast und dann 30 min bei 120°C gerührt. Nach beendeter Reaktion wurde das Reaktionsgemisch eingeengt, der Rückstand in Essigsäureethylester/Wasser aufgenommen und extrahiert. Die wässrige Phase wurde zweimal mit Essigsäureethylester extrahiert. Die vereinten organischen Phasen wurden über Natriumsulfat getrocknet, filtriert, einrotiert und im Hochvakuum getrocknet. Der Rückstand wurde mittels präparativer HPLC gereinigt (RP18 Säule, Laufmittel: Acetonitril/Wasser-Gradient unter Zusatz von 0.1% TFA). Die Produktfraktionen wurden vereinigt und einrotiert. Der Rückstand wurde in Dichlormethan und wenig Methanol gelöst und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingedampft. Die Produktfraktionen wurde nochmals mittels Dickschichtchromatographie gereinigt (Laufmittel: Dichlormethan/Methanol = 15/1). Es wurden 34 mg der Zielverbindung (51% d. Th.) erhalten.
LC-MS (Methode 1): Rₜ = 0.78 min
MS (ESpos): m/z = 378 (M+H)⁺

### Vergleichsbeispiel Beispiel 20

### N-(3-{6-Chlor-8-[(3-fluorpyridin-2-yl)methoxy]-2-methylimidazo[1,2-a]pyridin-3-yl}phenyl)acetamid

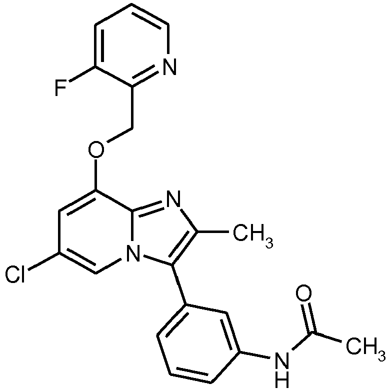

40 mg (0.10 mmol) 3-Brom-6-chlor-8-[(3-fluorpyridin-2-yl)methoxy]-2-methylimidazo[1,2-a]pyridin (Beispiel 43A) wurden in einem Gemisch aus 0.75 ml Ethanol, 0.37 ml Wasser und 0.37 ml Toluol unter Argon nacheinander mit 17 mg (0.09 mmol) (3-Acetamidophenyl)borsäure, 65 mg (0.31 mmol) Kaliumphosphat und 5.2 mg (0.01 mmol) Bis(tri-tert-butyl-phosphin)palladium(0) versetzt. Die Suspension wurde mit Argon entgast und dann 30 min bei 100°C gerührt. Nach beendeter Reaktion wurde das Reaktionsgemisch in Essigsäureethylester/Wasser aufgenommen und extrahiert. Die wässrige Phase wurde zweimal mit Essigsäureethylester extrahiert. Die vereinten organischen Phasen wurden über Natriumsulfat getrocknet, filtriert und einrotiert. Der Rückstand wurde mittels präparativer HPLC gereinigt (RP18 Säule, Laufmittel: Acetonitril/Wasser-Gradient unter Zusatz von 0.1% TFA). Die Produktfraktionen wurden eingedampft und mittels Dickschichtchromatographie gereinigt (Laufmittel: Dichlormethan /Methanol = 10/1). Die Produktfraktionen wurden in Dichlormethan gelöst und zweimal mit gesättigter, wässriger Natriumhydrogencarbonatlösung gewaschen. Die vereinigten wässrigen Phasen wurden zweimal mit Dichlormethan extrahiert. Die vereinigten organischen Phasen wurden über Natriumsulfat getrocknet, filtriert und eingedampft. Es wurden 3 mg der Zielverbindung (6% d. Th.) erhalten.
LC-MS (Methode 1): Rₜ = 0.71 min
MS (ESpos): m/z = 425 (M+H)⁺

### B. Bewertung der pharmakologischen Wirksamkeit

Es werden die folgenden Abkürzungen verwendet:
- ATP: Adenosintriphosphat
- Brij35: Polyoxyethylen(23)laurylether
- BSA: Rinderserumalbumin
- DTT: Dithiothreitol
- TEA: Triethanolamin

Die pharmakologische Wirkung der erfindungsgemäßen Verbindungen kann in folgenden Assays gezeigt werden:

### B-1. Vermessung von sGC Enzymaktivität mittels PPi Nachweis

Lösliche Guanylylcyclase (sGC) setzt unter Stimulation GTP zu cGMP und Pyrophosphat (PPi) um. PPi wird mit Hilfe des in WO 2008/061626 beschriebenen Verfahrens nachgewiesen. Das im Test entstehende Signal nimmt mit fortschreitender Umsetzung zu und dient als Maß für die sGC-Enzymaktivität. Mit Hilfe einer PPi Referenzkurve kann das Enzym in bekannter Weise charakterisiert werden, z.B. hinsichtlich Umsatzrate, Stimulierbarkeit oder Michaelis Konstante.

### Durchführung des Tests

Zur Durchführung des Tests wurden 29 µL Enzymlösung (0-10 nM lösliche Guanylylcyclase (hergestellt nach Hönicka et al., Journal of Molecular Medicine 77(1999)14-23), in 50 mM TEA, 2 mM Magnesiumchlorid, 0.1% BSA (FraktionV), 0.005% Brij 35, pH 7.5) in die Mikroplatte vorgelegt und 1 µL der Stimulatorlösung (0-10 µM 3-Morpholinosydnonimine, SIN-1, Merck in DMSO) hinzugegeben. Es wurde 10 min bei RT inkubiert. Anschließend wurden 20 µl Detektionsmix (1,2 nM Firefly Luciferase (*Photinus pyralis* Luziferase, Promega), 29 µM Dehydro-Luziferin (hergestellt nach Bitler & McElroy, Arch. Biochem. Biophys. 72 (1957) 358), 122 µM Luziferin (Promega), 153 µM ATP (Sigma) und 0,4 mM DTT (Sigma) in 50 mM TEA, 2 mM Magnesiumchlorid, 0.1% BSA (Fraktion V), 0.005% Brij 35, pH 7,5) zugegeben. Die Enzymreaktion wurde durch Zugabe von 20 µl Substratlösung (1.25 mM Guanosin-5'-triphosphat (Sigma) in 50 mM TEA, 2 mM Magnesiumchlorid, 0.1% BSA (Fraktion V), 0.005% Brij 35, pH 7.5) gestartet und kontinuierlich luminometrisch vermessen.

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative MEC-Werte (MEC = minimal effektive Konzentration) für die erfindungsgemäßen Verbindungen sind in der nachstehenden Tabelle wiedergegeben (zum Teil als Mittelwerte aus Einzelbestimmungen):

**Tabelle A:**

| Beispiel Nr. | MEC [µM] |
|---|---|
| 1 | 0.1 |
| 2 | 0.1 |
| 3 | 0.2 |
| 4 | 1.0 |
| 5 | 3.0 |
| 6 | 10 |
| 7 | 0.3 |
| 8 | 0.3 |
| 9 | 0.3 |
| 10 | 0.1 |
| 11 | 0.3 |
| 12 | 1.0 |
| 13 | 0.2 |
| 14 | 6.5 |
| 15 | 0.02 |
| 16 | 3.0 |
| 19 | 0.2 |
| 20 | 0.3 |

### B-3. Gefäßrelaxierende Wirkung in vitro

Kaninchen werden durch Nackenschlag betäubt und entblutet. Die Aorta wird entnommen, von anhaftendem Gewebe befreit, in 1.5 mm breite Ringe geteilt und einzeln unter einer Vorspannung in 5 ml-Organbäder mit 37°C warmer, Carbogen-begaster Krebs-Henseleit-Lösung folgender Zusammensetzung gebracht (jeweils mM): Natriumchlorid: 119; Kaliumchlorid: 4.8; Calciumchlorid-Dihydrat: 1; Magnesiumsulfat-Heptahydrat: 1.4; Kaliumdihydrogenphosphat: 1.2; Natriumhydrogencarbonat: 25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung zugesetzt und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀-Wert). Das Standardapplikationsvolumen beträgt 5 µl, der DMSO-Anteil in der Badlösung entspricht 0.1%.

### B-4. Blutdruckmessung an narkotisierten Ratten

Männliche Wistar-Ratten mit einem Körpergewicht von 300 - 350 g werden mit Thiopental (100 mg/kg i.p.) anästhesiert. Nach der Tracheotomie wird in die Femoralarterie ein Katheter zur Blutdruckmessung eingeführt. Die zu prüfenden Substanzen werden als Lösungen entweder oral mittels Schlundsonde oder über die Femoralvene intravenös verabreicht (Stasch et al. Br. J. Pharmacol. 2002; 135: 344-355).

### B-5. Radiotelemetrische Blutdruckmessung an wachen, spontan hypertensiven Ratten

Für die im Folgenden beschriebene Blutdruckmessung an wachen Ratten wird ein im Handel erhältliches Telemetriesystem der Firma DATA SCIENCES INTERNATIONAL DSI, USA eingesetzt.

Das System besteht aus 3 Hauptkomponenten:
Implantierbare Sender (Physiotel® Telemetrietransmitter)
Empfänger (Physiotel® Receiver), die über einen Multiplexer (DSI Data Exchange Matrix) mit einem
Datenakquisitionscomputer verbunden sind.

Die Telemetrieanlage ermöglicht eine kontinuierliche Erfassung von Blutdruck Herzfrequenz und Körperbewegung an wachen Tieren in ihrem gewohnten Lebensraum.

### Tiermaterial

Die Untersuchungen werden an ausgewachsenen weiblichen spontan hypertensiven Ratten (SHR Okamoto) mit einem Körpergewicht von >200 g durchgeführt. SHR/NCrl von Okamoto Kyoto School of Medicine, 1963 wurden aus männlichen Wistar Kyoto Ratten mit stark erhöhtem Blutdruck und weiblichen mit leicht erhöhtem Blutdruck gekreuzt und in der F13 an die U.S. National Institutes of Health abgegeben.

Die Versuchstiere werden nach Senderimplantation einzeln in Makrolon - Käfigen Typ 3 gehalten. Sie haben freien Zugang zu Standardfutter und Wasser.

Der Tag - Nacht - Rhythmus im Versuchslabor wird per Raumbeleuchtung um 6:00 Uhr morgens und um 19:00 Uhr abends gewechselt.

### Senderimplantation

Die eingesetzten Telemetriesender TA11 PA - C40 werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobabital (Nembutal, Sanofi: 50mg/kg i.p.) narkotisiert und an der Bauchseite weiträumig rasiert und desinfiziert. Nach Eröffnung des Bauchraumes entlang der Linea alba wird der flüssigkeitsgefüllte Meßkatheter des Systems oberhalb der Bifurcation nach cranial in die Aorta descendens eingesetzt und mit Gewebekleber (VetBonD TM, 3M) befestigt. Das Sendergehäuse wird intraperitoneal an der Bauchwandmuskulatur fixiert und die Wunde wird schichtweise verschlossen.

Postoperativ wird zur Infektionsprophylaxe ein Antibiotikum verabreicht (Tardomyocel COMP Bayer 1ml/kg s.c.)

### Substanzen und Lösungen

Wenn nicht anders beschrieben werden die zu untersuchenden Substanzen jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5% iger Tylose suspendiert.

Eine Lösungsmittel- behandelte Gruppe von Tieren wird als Kontrolle eingesetzt.

### Versuchsablauf

Die vorhandene Telemetrie - Meßeinrichtung ist für 24 Tiere konfiguriert. Jeder Versuch wird unter einer Versuchsnummer registiert (VJahr Monat Tag).

Den in der Anlage lebenden instrumentierten Ratten ist jeweils eine eigene Empfangsantenne zugeordnet (1010 Receiver, DSI).

Die implantierten Sender sind über einen eingebauten Magnetschalter von außen aktivierbar. Sie werden bei Versuchsvorlauf auf Sendung geschaltet. Die ausgestrahlten Signale können durch ein Datenakquisitionssystem (Dataquest TM A.R.T. for WINDOWS, DSI) online erfasst und entsprechend aufgearbeitet werden. Die Ablage der Daten erfolgt jeweils in einem hierfür eröffneten Ordner der die Versuchsnummer trägt.
Im Standardablauf werden über je 10 Sekunden Dauer gemessen
Systolischer Blutdruck (SBP)
Diastolischer Blutdruck (DBP)
Arterieller Mitteldruck (MAP)
Herzfrequenz (HR)
Aktivität (ACT)

Die Messwerterfassung wird rechnergesteuert in 5 Minuten Abständen wiederholt. Die als Absolutwert erhobenen Quelldaten werden im Diagramm mit dem aktuell gemessenen Barometerdruck (Ambient Pressure Reference Monitor; APR-1) korrigiert und in Einzeldaten abgelegt. Weitere technische Details sind der umfangreichen Dokumentation der Herstellerfirma (DSI) zu entnehmen.

Wenn nicht anders beschrieben erfolgt die Verabreichung der Prüfsubstanzen am Versuchstag um 9.00 Uhr. Im Anschluss an die Applikation werden die oben beschriebenen Parameter 24 Stunden gemessen.

### Auswertung

Nach Versuchsende werden die erhobenen Einzeldaten mit der Analysis-Software (DATAQUEST TM A. R.T. TM ANALYSIS) sortiert. Als Leerwert werden hier 2 Stunden vor Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten Average) und als Textdatei auf einen Datenträger übertragen. Die so vorsortierten und komprimierten Messwerte werden in Excel-Vorlagen übertragen und tabellarisch dargestellt. Die Ablage der erhobenen Daten erfolgt pro Versuchstag in einem eigenen Ordner, der die Versuchsnummer trägt. Ergebnisse und Versuchsprotokolle werden in Papierform nach Nummern sortiert in Ordnern abgelegt.

### Literatur:

Klaus Witte, Kai Hu, Johanna Swiatek, Claudia Müssig, Georg Ertl and Björn Lemmer: Experimental heart failure in rats: effects on cardiovascular circadian rhythms and on myocardial β-adrenergic signaling. Cardiovasc Res 47 (2): 203-405, 2000; Kozo Okamoto: Spontaneous hypertension in rats. Int Rev Exp Pathol 7: 227- 270, 1969; Maarten van den Buuse: Circadian Rhythms of Blood Pressure, Heart Rate, and Locomotor Activity in Spontaneously Hypertensive Rats as Measured With Radio-Telemetry. Physiology & Behavior 55(4): 783-787, 1994.

### B-6. Bestimmung pharmakokinetischer Kenngrößen nach intravenöser und oraler Gabe

Die pharmakokinetischen Parameter der erfindungsgemäßen Verbindungen werden in männlichen CD-1-Mäusen, männlichen Wistar-Ratten und weiblichen Beagle-Hunden bestimmt. Die intravenöse Gabe erfolgt bei Mäusen und Ratten mittels einer speziesspezifischen Plasma/DMSO-Formulierung und bei Hunden mittels einer Wasser/PEG400/Ethanol-Formulierung. Die orale Gabe der gelösten Substanz mittels Schlundsonde wird in allen Spezies basierend auf einer Wasser/PEG400/Ethanol-Formulierung durchgeführt. Den Ratten wird zur vereinfachten Blutabnahme vor der Substanzgabe ein Silikonkatheter in die rechte *Vena jugularis externa* gelegt. Die Operation erfolgt mindestens einen Tag vor dem Versuch unter Isofluran-Narkose und unter Gabe eines Analgetikums (Atropin/Rimadyl (3/1) 0.1 mL s.c.). Die Blutabnahme (in der Regel mehr als 10 Zeitpunkte) erfolgt in einem Zeitfenster, welches terminale Zeitpunkte von mindestens 24 bis maximal 72 Stunden nach Substanzgabe beinhaltet. Das Blut wird bei der Entnahme in heparinisierte Röhrchen geleitet. So dann wird mittels Zentrifugation das Blutplasma gewonnen und gegebenenfalls bis zur weiteren Bearbeitung bei -20°C gelagert.

Den Proben der erfindungsgemäßen Verbindungen, Kalibrierproben und Qualifier wird ein interner Standard zugesetzt (dies kann auch eine chemisch nicht verwandte Substanz sein) und es folgt eine Proteinfällung mittels Acetonitril im Überschuss. Nach Zugabe einer Puffer-Lösung, die an die LC-Bedingungen angepasst ist, und folgendem Vortexen wird bei 1000 g zentrifugiert. Der Überstand wird mittels LC-MS/MS unter Verwendung von C18-reversed-phase-Säulen und variablen Eluenten-Gemischen vermessen. Die Quantifizierung der Substanzen erfolgt anhand der Peakhöhen oder -flächen aus extrahierten Ionenchromatogrammen spezifischer selected ion monitoring-Experimente.

Aus den ermittelten Plasmakonzentration-Zeit-Verläufen werden die pharmakokinetischen Kenngrößen wie AUC, Cₘₐₓ, t_{1/2} (terminale Halbwertszeit), F (Bioverfügbarkeit), MRT (Mean Residence Time) und CL (Clearance) mittels eines validierten pharmakokinetischen Rechenprogramms berechnet.

Da die Substanzquantifizierung in Plasma durchgeführt wird, muss die Blut/Plasma-Verteilung der Substanz bestimmt werden, um die pharmakokinetischen Parameter entsprechend anpassen zu können. Dazu wird eine definierte Menge Substanz in heparinisiertem Vollblut der entsprechenden Spezies für 20 min im Taumelrollenmischer inkubiert. Nach Zentrifugation bei 1000g wird die Konzentration im Plasma gemessen (mittels LC-MS/MS; s.o.) und durch Quotientenbildung der C_{Blut}/C_{Plasma}-Wert ermittelt.

### B-7. Metabolismus-Untersuchung

Zur Bestimmung des Metabolismus-Profils der erfindungsgemäßen Verbindungen werden diese mit rekombinanten humanen Cytochrom P450 (CYP) Enzymen, Lebermikrosomen oder mit primären frischen Hepatozyten verschiedener Tierspezies (z.B. Ratte, Hund) als auch humanen Ursprungs inkubiert, um Informationen über einen möglichst kompletten hepatischen Phase I- und Phase II-Metabolismus sowie über die am Metabolismus beteiligten Enzyme zu erhalten und zu vergleichen.

Die erfindungsgemäßen Verbindungen wurden mit einer Konzentration von etwa 0.1-10 µM inkubiert. Dazu wurden Stammlösungen der erfindungsgemäßen Verbindungen mit einer Konzentration von 0.01-1 mM in Acetonitril hergestellt, und dann mit einer 1:100 Verdünnung in den Inkubationsansatz pipettiert. Die Lebermikrosomen und rekombinanten Enzyme wurden in 50 mM Kaliumphosphatpuffer pH 7.4 mit und ohne NADPH-generierendem System, bestehend aus 1 mM NADP⁺, 10 mM Glucose-6-phosphat und 1 Unit Glucose-6-phosphat Dehydrogenase, bei 37°C inkubiert. Primäre Hepatozyten wurden in Suspension in Williams E Medium ebenfalls bei 37°C inkubiert. Nach einer Inkubationszeit von 0 - 4h wurden die Inkubationsansätze mit Acetonitril abgestoppt (Endkonzentration ca. 30%) und das Protein bei ca. 15000 x g abzentrifugiert. Die so abgestoppten Proben wurden entweder direkt analysiert oder bis zur Analyse bei -20°C gelagert.

Die Analyse erfolgt mittels Hochleistungsflüssigkeits-Chromatographie mit Ultraviolett- und massenspektrometrischer Detektion (HPLC-UV-MS/MS). Dazu werden die Überstände der Inkubationsproben mit geeigneten C18-reversed-phase-Säulen und variablen Eluenten-Gemischen aus Acetonitril und 10 mM wässriger Ammoniumformiat-Lösung oder 0.05 % Ameisensäure chromatographiert. Die UV-Chromatogramme in Verbindung mit massenspektrometrischen Daten dienen zur Identifizierung, Strukturaufklärung und quantitativen Abschätzung der Metabolite, und der quantitativen metabolischen Abnahme der erfindungsgemäßen Verbindung in den Inkubationsansätzen.

### B-8. Caco-2 Permeabilitäts-Test

Die Permeabilität einer Testsubstanz wurde mit Hilfe der Caco-2 Zelllinie, einem etablierten *in vitro* Modell für Permeabilitätsvorhersagen an der gastrointestinalen Barriere, bestimmt (Artursson, P. and Karlsson, J. (1991). Correlation between oral drug absorption in humans and apparent drug permeability coefficients in human intestinal epithelial (Caco-2) cells. Biochem. Biophys.175 (3), 880-885). Die Caco-2 Zellen (ACC No. 169, DSMZ, Deutsche Sammlung von Mikroorganismen und Zellkulturen, Braunschweig, Deutschland) wurden in 24-Well Platen mit Einsatz ausgesät und 14 bis 16 Tage kultiviert. Für die Permeabilitätsstudien wurde die Testsubstanz in DMSO gelöst und mit Transportpuffer (Hanks Buffered Salt Solution, Gibco/Invitrogen, mit 19.9 mM Glukose und 9.8 mM HEPES) auf die finale Testkonzentration verdünnt. Um die Permeabilität von apikal nach basolateral (PₐₚₚA-B) der Testsubstanz zu bestimmen, wurde die Lösung mit der Testsubstanz auf die apikale Seite des Caco-2 Zellmonolayers gegeben und Transportpuffer auf die basolaterale Seite. Um die Permeabilität von basolateral nach apikal (PₐₚₚB-A) der Testsubstanz zu bestimmen, wurde die Lösung mit der Testsubstanz auf die basolaterale Seite des Caco-2 Zellmonolayers gegeben und Transportpuffer auf die apikale Seite. Zu Beginn des Experiments wurden Proben aus dem jeweiligen Donor-Kompartiment genommen, um die Massenbilanz sicher zu stellen. Nach einer Inkubation von zwei Stunden bei 37° C wurden Proben aus beiden Kompartimenten genommen. Die Proben wurden mittels LC-MS/MS analysiert und die apparenten Permeabilitätskoeffizienten (Pₐₚₚ) berechnet. Die Permeabilität von Lucifer Yellow wurde für jeden Zellmonolayer bestimmt, um die Integrität der Zellschicht sicher zu stellen. Die Permeabilität von Atenolol (Marker für niedrige Permeabilität) und Sulfasalazin (Marker für aktive Exkretion) wurde in jedem Testlauf als Qualitätskontrolle mitbestimmt.

### B-9. hERG Kaliumstrom Assay.

Der sogenannte hERG (human ether-a-go-go related gene) Kaliumstrom trägt wesentlich zur Repolarisierung des humanen kardialen Aktionspotentials bei (Scheel et al., 2011). Eine Inhibition dieses Stroms durch Pharmaka kann in seltenen Fällen potentiell letale Herzrhythmusstörungen zur Folge haben, und wird deshalb frühzeitig während der Arzneimittelentwicklung untersucht.

Der hier verwendete funktionelle hERG Assay basiert auf einer recombinanten HEK293 ZellLinie, die das KCNH2(HERG)-Gen stabil exprimiert (Zhou et al., 1998). Diese Zellen werden mittels der "whole-cell voltage-clamp" Technik (Hamill et al., 1981) in einem automatisierten System (Patchliner™; Nanion, München, D) untersucht, welches die Membranspannung kontrolliert und den hERG Kalium-Strom bei Zimmertemperatur misst. Die PatchControlHT™ Software (Nanion) steuert Patchliner System, Datenerfassung und Datenanalyse. Die Spannungskontrolle erfolgt durch 2 EPC-10 quadro Verstärker unter Kontrolle der PatchMasterPro™ Software (beide: HEKA Elektronik, Lambrecht, D). NPC-16 Chips mit mittlerem Widerstand (∼2 MΩ; Nanion) dienen als planares Substrat für die Voltage-Clamp Experimente.

NPC-16 Chips werden mit intra- und extrazellulärer Lösung (vgl. Himmel, 2007) sowie mit Zellsuspension befüllt. Nach Bildung eines Giga-Ohm-Seals und Herstellen des Ganzzell-Modus (einschliesslich mehrerer automatisierter Qualitätskontrollschritte) wird die Zellmembran auf das Haltepotential -80 mV geklemmt. Das nachfolgende Spannungsklemm-Protokoll ändert die Kommandospannung auf +20 mV (Dauer 1000 ms), -120 mV (Dauer 500 ms), und zurück zum Haltepotential -80 mV; dies wird alle 12 s wiederholt. Nach einer initialen Stabilisierungsphase (ca 5-6 Minuten) wird Testsubstanzlösung in aufsteigenden Konzentrationen (z.B. 0.1, 1, und 10 µmol/L) zupipettiert (Exposition ca 5-6 Minuten pro Konzentration), gefolgt von mehreren Auswaschschritten.

Die Amplitude des einwärtsgerichteten "Tail"-Stroms, der durch eine Potentialänderung von +20 mV auf -120 mV erzeugt wird, dient zur Quantifizierung des hERG Kaliumstroms, und wird als Funktion der Zeit dargestellt (IgorPro™ Software). Die Stromamplitude am Ende verschiedener Zeitabschnitte (z.B. Stabilisierungsphase vor Testsubstanz, erste/zweite/dritte Konzentration Testsubstanz) dient zur Erstellung einer Konzentrations-Wirkungs-Kurve, aus der die halbmaximale Hemmkonzentration IC₅₀ der Testsubstanz errechnet wird.
Hamill OP, Marty A, Neher E, Sakmann B, Sigworth FJ. Improved patch-clamp techniques for high-resolution current recording from cells and cell-free membrane patches. Pfluegers Arch 1981; 391:85-100.
Himmel HM. Suitability of commonly used excipients for electrophysiological in-vitro safety pharmacology assessment of effects on hERG potassium current and on rabbit Purkinje fiber action potential. J Pharmacol Toxicol Methods 2007;56:145-158.
Scheel O, Himmel H, Rascher-Eggstein G, Knott T. Introduction of a modular automated voltage-clamp platform and its correlation with manual human ether-a-go-go related gene voltage-clamp data. Assay Drug Dev Technol 2011;9:600-607.
Zhou ZF, Gong Q, Ye B, Fan Z, Makielski JC, Robertson GA, January CT. Properties of hERG channels stably expressed in HEK293 cells studied at physiological temperature. Biophys J 1998;74:230-241.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel® (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die erhaltene Lösung wird steril filtriert und in sterile und pyrogenfreie Injektionsbehältnisse abgefüllt.

## Patentansprüche

1. Verbindung der Formel (I) 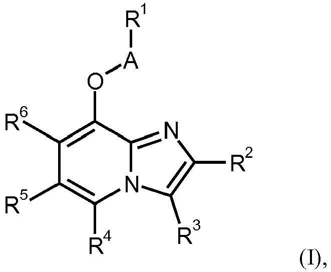 in welcher
A für CH₂ steht,
R¹ für Cyclohexyl steht,
oder
für eine Phenyl-Gruppe der Formel 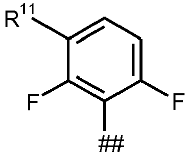 steht, wobei
## für die Anknüpfstelle an A steht,
und
R¹¹ für Wasserstoff oder Fluor steht,
oder
für eine Pyridyl-Gruppe der Formel 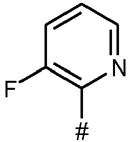 steht, wobei
# für die Anknüpfstelle an A steht,
R² für Methyl steht,
R³ für 3-Pyridyl, 4-Pyridyl, 2-Pyrimidyl, 5-Pyrimidyl oder 1,3,5-Triazinyl steht,
wobei 3-Pyridyl in 4-Position mit -NR⁹R¹⁰ substituiert ist,
wobei 4-Pyridyl in 3-Position mit -NR⁹R¹⁰ substituiert ist,
wobei 2-Pyrimidyl in 4-Position mit -NR⁹R¹⁰ substituiert ist,
wobei 5-Pyrimidyl in 2-Position mit -NR⁹R¹⁰ substituiert ist,
worin jeweils
R⁹ für Wasserstoff oder Methyl steht,
R¹⁰ für (C₁-C₆)-Alkyl oder Phenyl steht,
worin (C₁-C₆)-Alkyl mit 1 oder 2 Substituenten unabhängig voneinander ausgewählt aus der Gruppe (C₁-C₄)-Alkoxy, Amino und Phenyl, sowie bis zu dreifach mit Fluor substituiert sein kann,
oder
R⁹ und R¹⁰ zusammen mit dem Stickstoffatom, an das sie gebunden sind, einen Pyrrolidinyl-, Morpholinyl- oder Piperazinyl-Ring bilden,
worin der Pyrrolidinyl-, Morpholinyl- oder Piperazinyl-Ring mit 1 oder 2 Substituenten Methyl substituiert sein kann,
wobei 4-Pyridyl in 2-Position mit Fluor substituiert sein kann,
wobei 1,3,5-Triazinyl bis zu zweifach mit -NR^{9A}R^{10A} substituiert ist,
worin
R^{9A} für Wasserstoff oder Methyl steht,
R^{10A} für Wasserstoff, (C₁-C₆)-Alkyl oder Phenyl steht,
worin (C₁-C₆)-Alkyl mit Amino sowie bis zu dreifach mit Fluor substituiert sein kann,
wobei 1,3,5-Triazinyl mit Chlor substituiert sein kann,
R⁴ für Wasserstoff steht,
R⁵ für Wasserstoff, Chlor oder Methyl steht,
R⁶ für Wasserstoff steht,
sowie ihre *N*-Oxide, Salze, Solvate, Salze der *N*-Oxide und Solvate der *N*-Oxide und Salze.

2. Verfahren zur Herstellung von Verbindungen der Formel (I), wie in Anspruch 1 definiert, **dadurch gekennzeichnet, dass** man
[A] eine Verbindung der Formel (II) 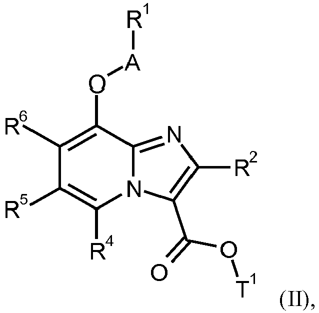 in welcher A, R¹, R², R⁴, R⁵ und R⁶ jeweils die oben angegebenen Bedeutungen haben und
T¹ für (C₁-C₄)-Alkyl oder Benzyl steht,
in einem inerten Lösungsmittel in Gegenwart einer geeigneten Base oder Säure zu einer Carbonsäure der Formel (III) 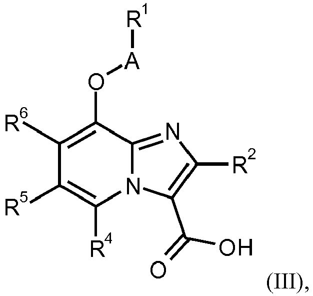 in welcher A, R¹, R², R⁴, R⁵ und R⁶ jeweils die oben angegebenen Bedeutungen haben, umsetzt,
und diese in der Folge in Gegenwart einer geeigneten Säure zu einem Imidazo[1,2-a]-pyridin der Formel (IV) 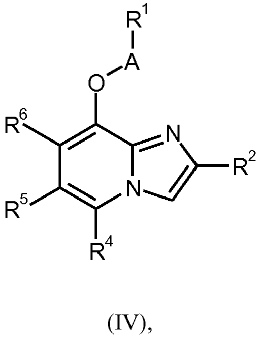 in welcher A, R¹, R², R⁴, R⁵ und R⁶ jeweils die oben angegebenen Bedeutungen haben, umsetzt, und dieses dann mit einem Halogen-Äquivalent in eine Verbindung der Formel (V) 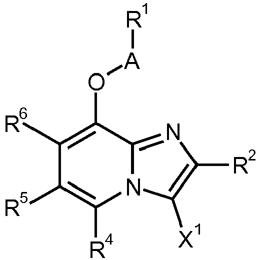 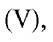 in welcher A, R¹, R², R⁴, R⁵ und R⁶ jeweils die oben angegebenen Bedeutungen haben und
X¹ für Chlor, Brom oder Iod steht,
überführt, und diese im Anschluß in einem inerten Lösungsmittel in Gegenwart eines geeigneten Übergangsmetallkatalysators mit einer Verbindung der Formel (VI), 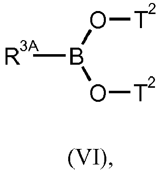 in welcher
R^{3A} die oben für R³ angegebenen Bedeutungen hat und
T² für Wasserstoff oder (C₁-C₄)-Alkyl steht, oder beide Reste T² zusammen eine -C(CH₃)₂-C(CH₃)₂-Brücke bilden,
umsetzt, anschliessend gegebenenfalls vorhandene Schutzgruppen abspaltet, und die resultierenden Verbindungen der Formel (I) gegebenenfalls mit den entsprechenden (i) Lösungsmitteln und/oder (ii) Säuren oder Basen in ihre Solvate, Salze und/oder Solvate der Salze überführt,

3. Verbindung der Formel (I), wie in Anspruch 1 definiert, zur Verwendung zur Behandlung und/oder Prophylaxe von Krankheiten.

4. Verwendung einer Verbindung der Formel (I), wie in Anspruch 1 definiert, zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen und Arteriosklerose.

5. Arzneimittel enthaltend eine Verbindung der Formel (I), wie Anspruch 1 definiert, in Kombination mit einem inerten, nicht-toxischen, pharmazeutisch geeigneten Hilfsstoff.

6. Arzneimittel enthaltend eine Verbindung der Formel (I), wie in Anspruch 1 definiert, in Kombination mit einem weiteren Wirkstoff ausgewählt aus der Gruppe bestehend aus organischen Nitraten, NO-Donatoren, cGMP-PDE-Inhibitoren, antithrombotisch wirkenden Mitteln, den Blutdruck senkenden Mitteln sowie den Fettstoffwechsel verändernden Mitteln.

7. Arzneimittel nach Anspruch 5 oder 6 zur Verwendung zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen und Arteriosklerose.

## Claims

1. Compound of the formula (I) 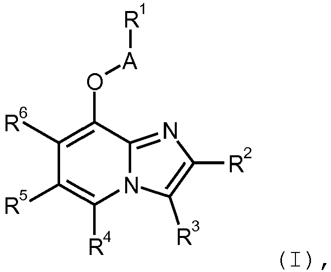 in which
A represents CH₂,
R¹ represents cyclohexyl,
or
represents a phenyl group of the formula 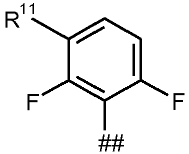 where
## represents the point of attachment to A,
and
R¹¹ represents hydrogen or fluorine,
or
represents a pyridyl group of the formula 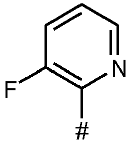 where
# represents the point of attachment to A,
R² represents methyl,
R³ represents 3-pyridyl, 4-pyridyl, 2-pyrimidyl, 5-pyrimidyl or 1,3,5-triazinyl,
where 3-pyridyl is substituted in the 4-position by -NR⁹R¹⁰,
where 4-pyridyl is substituted in the 3-position by -NR⁹R¹⁰,
where 2-pyrimidyl is substituted in the 4-position by -NR⁹R¹⁰,
where 5-pyrimidyl is substituted in the 2-position by -NR⁹R¹⁰,
in which in each case
R⁹ represents hydrogen or methyl,
R¹⁰ represents (C₁-C₆) -alkyl or phenyl, in which (C₁-C₆) -alkyl may be substituted by 1 or 2 substituents independently of one another selected from the group consisting of (C₁-C₄)-alkoxy, amino and phenyl, and up to trisubstituted by fluorine,
or
R⁹ and R¹⁰ together with the nitrogen atom to which they are attached form a pyrrolidinyl, morpholinyl or piperazinyl ring,
in which the pyrrolidinyl, morpholinyl or piperazinyl ring may be substituted by 1 or 2 methyl substituents,
where 4-pyridyl may be substituted in the 2-position by fluorine,
where 1,3,5-triazinyl is up to disubstituted by -NR^{9A}R^{10A},
in which
R^{9A} represents hydrogen or methyl,
R^{10A} represents hydrogen, (C₁-C₆) -alkyl or phenyl,
in which (C₁-C₆) -alkyl may be substituted by amino and up to three times by fluorine,
where 1,3,5-triazinyl may be substituted by chlorine,
R⁴ represents hydrogen,
R⁵ represents hydrogen, chlorine or methyl,
R⁶ represents hydrogen,
and the N-oxides, salts, solvates, salts of the N-oxides and solvates of the N-oxides and salts thereof.

2. Process for preparing compounds of the formula (I) as defined in Claim 1, **characterized in that**
[A] a compound of the formula (II) 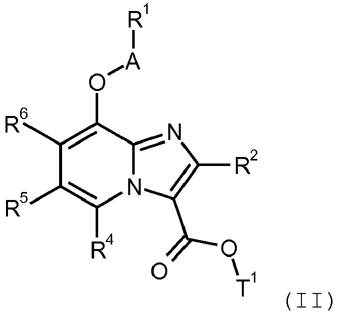 in which A, R¹, R², R⁴, R⁵ and R⁶ are each as defined above and
T¹ represents (C₁-C₄)-alkyl or benzyl,
is reacted in an inert solvent in the presence of a suitable base or acid to give a carboxylic acid of the formula (III) 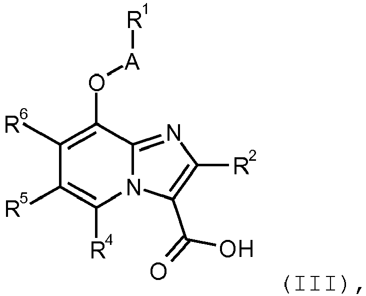 in which A, R¹, R², R⁴, R⁵ and R⁶ each have the meanings given above,
and these are subsequently reacted in the presence of a suitable acid to give an imidazo[1,2-a]-pyridine of the formula (IV) 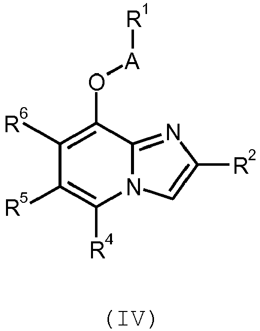 in which A, R¹, R², R⁴, R⁵ and R⁶ each have the meanings given above,
and this is then converted with a halogen equivalent into a compound of the formula (V) 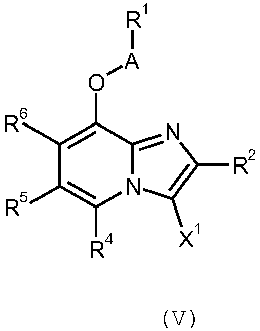 in which A, R¹, R², R⁴, R⁵ and R⁶ are each as defined above and
X¹ represents chlorine, bromine or iodine,
and this is subsequently reacted in an inert solvent, in the presence of a suitable transition metal catalyst, with a compound of the formula (VI) 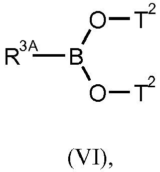 in which
R^{3A} has the meanings given above for R³ and
T² represents hydrogen or (C₁-C₄) -alkyl, or the two T² radicals together form a -C(CH₃)₂-C(CH₃)₂- bridge,
any protective groups present are subsequently detached, and the resulting compounds of the formula (I) are optionally converted with the appropriate (i) solvents and/or (ii) acids or bases to the solvates, salts and/or solvates of the salts thereof.

3. Compound of the formula (I) as defined in Claim 1 for use for the treatment and/or prophylaxis of diseases.

4. Use of a compound of the formula (I) as defined in Claim 1 for producing of a medicament for the treatment and/or prophylaxis of heart failure, angina pectoris, hypertension, pulmonary hypertension, ischaemias, vascular disorders, renal insufficiency, thromboembolic disorders and arteriosclerosis.

5. Medicament comprising a compound of the formula (I) as defined Claim 1 in combination with an inert, non-toxic, pharmaceutically suitable excipient.

6. Medicament comprising a compound of the formula (I) as defined in Claim 1 in combination with a further active compound selected from the group consisting of organic nitrates, NO donors, cGMP-PDE inhibitors, antithrombotic agents, hypotensive agents and lipid metabolism modifiers.

7. Medicament according to Claim 5 or 6 for use for the treatment and/or prophylaxis of heart failure, angina pectoris, hypertension, pulmonary hypertension, ischaemias, vascular disorders, kidney failure, thromboembolic disorders and arteriosclerosis.

## Revendications

1. Composé de formule (I) 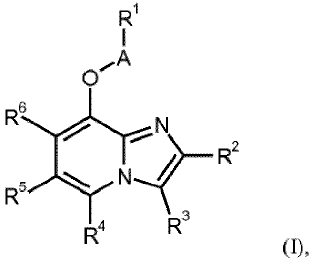 dans laquelle
A représente CH₂,
R¹ représente cyclohexyle,
ou
représente un groupe phényle de formule : 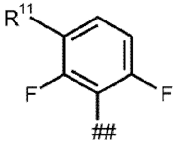 dans laquelle
## représente l'emplacement de liaison à A,
et
R¹¹ représente hydrogène ou fluor,
ou représente un groupe pyridyle de formule : 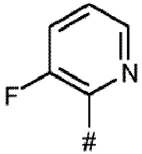 dans laquelle
# représente l'emplacement de liaison à A,
R² représente méthyle,
R³ représente 3-pyridyle, 4-pyridyle, 2-pyrimidyle, 5-pyrimidyle ou 1,3,5-triazinyle,
le 3-pyridyle étant substitué en position 4 avec - NR⁹R¹⁰,
le 4-pyridyle étant substitué en position 3 avec - NR⁹R¹⁰,
le 2-pyrimidyle étant substitué en position 4 avec -NR⁹R¹⁰, le 5-pyrimidyle étant substitué en position 2 avec -NR⁹R¹⁰,
avec à chaque fois
R⁹ représentant hydrogène ou méthyle,
R¹⁰ représentant alkyle en (C₁-C₆) ou phényle, l'alkyle en (C₁-C₆) pouvant être substitué avec 1 ou 2 substituants choisis indépendamment l'un de l'autre dans le groupe constitué par alcoxy en (C₁-C₄), amino et phényle, ainsi que jusqu'à trois fois avec fluor,
ou
R⁹ et R¹⁰ formant ensemble avec l'atome d'azote auquel ils sont reliés un cycle pyrrolidinyle, morpholinyle ou pipérazinyle,
le cycle pyrrolidinyle, morpholinyle ou pipérazinyle pouvant être substitué avec 1 ou 2 substituants méthyle,
le 4-pyridyle pouvant être substitué en position 2 avec fluor,
le 1,3,5-triazinyle étant substitué jusqu'à deux fois avec -NR^{9A}R^{10A},
R^{9A} représentant hydrogène ou méthyle,
R^{10A} représentant hydrogène, alkyle en (C₁-C₆) ou phényle,
l'alkyle en (C₁-C₆) pouvant être substitué avec amino, ainsi que jusqu'à trois fois avec fluor,
le 1,3,5-triazinyle pouvant être substitué avec chlore,
R⁴ représente hydrogène,
R⁵ représente hydrogène, chlore ou méthyle,
R⁶ représente hydrogène,
ainsi que leurs N-oxydes, sels, solvates, sels des N-oxydes et solvates des N-oxydes et des sels.

2. Procédé de fabrication de composés de formule (I), tels que définis dans la revendication 1, **caractérisé en ce que**
[A] un composé de formule (II) 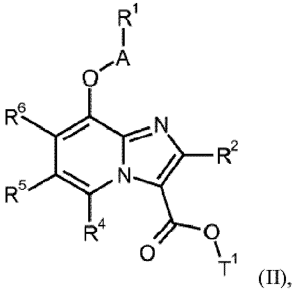 dans laquelle A, R¹, R², R⁴, R⁵ et R⁶ ont chacun les significations indiquées précédemment, et
T¹ représente alkyle en (C₁-C₄) ou benzyle,
est mis en réaction dans un solvant inerte en présence d'une base ou d'un acide approprié pour former un acide carboxylique de formule (III) : 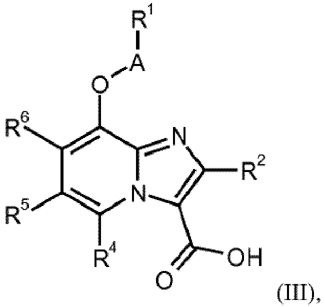 dans laquelle A, R¹, R², R⁴, R⁵ et R⁶ ont chacun les significations indiquées précédemment,
puis celui-ci est mis en réaction en présence d'un acide approprié pour former une imidazo[1,2-a]-pyridine de formule (IV) : 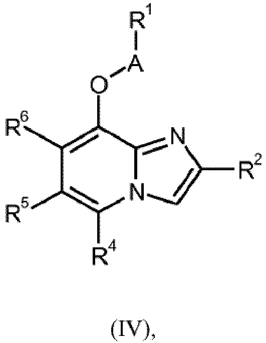 dans laquelle A, R¹, R², R⁴, R⁵ et R⁶ ont chacun les significations indiquées précédemment,
puis celle-ci est transformée avec un équivalent d'halogène en un composé de formule (V) : 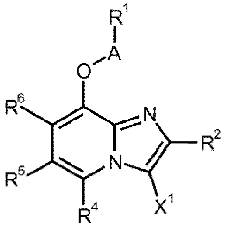 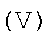 dans laquelle A, R¹, R², R⁴, R⁵ et R⁶ ont chacun les significations indiquées précédemment, et
X¹ représente chlore, brome ou iode,
puis celui-ci est mis en réaction dans un solvant inerte en présence d'un catalyseur de métal de transition approprié avec un composé de formule (VI) : 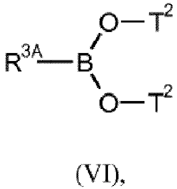 dans laquelle
R^{3A} a les significations indiquées précédemment pour R³, et
T² représente hydrogène ou alkyle en (C₁-C₄), ou les deux radicaux T² forment ensemble un pont -C(CH₃)₂-C(CH₃)₂-,
puis des groupes protecteurs éventuellement présents sont clivés, et les composés de formule (I) résultants sont éventuellement transformés avec (i) les solvants et/ou (ii) les acides ou les bases appropriés en leurs solvates, sels et/ou solvates des sels.

3. Composé de formule (I), tel que défini dans la revendication 1, destiné à une utilisation pour le traitement et/ou la prophylaxie de maladies.

4. Utilisation d'un composé de formule (I), tel que défini dans la revendication 1, pour la fabrication d'un médicament pour le traitement et/ou la prophylaxie de l'insuffisance cardiaque, de l'angine de poitrine, de l'hypertension, de l'hypertension pulmonaire, des ischémies, des maladies vasculaires, de l'insuffisance rénale, des maladies thromboemboliques et de l'artériosclérose.

5. Médicament contenant un composé de formule (I), tel que défini dans la revendication 1, en combinaison avec un adjuvant inerte, non toxique, pharmaceutiquement approprié.

6. Médicament contenant un composé de formule (I), tel que défini dans la revendication 1, en combinaison avec un agent actif supplémentaire choisi dans le groupe constitué par les nitrates organiques, les donneurs de NO, les inhibiteurs de cGMP-PDE, les agents à activité antithrombotique, les agents abaissant la tension artérielle, ainsi que les agents modifiant le métabolisme lipidique.

7. Médicament selon la revendication 5 ou 6, destiné à une utilisation pour le traitement et/ou la prophylaxie de l'insuffisance cardiaque, de l'angine de poitrine, de l'hypertension, de l'hypertension pulmonaire, des ischémies, des maladies vasculaires, de l'insuffisance rénale, des maladies thromboemboliques et de l'artériosclérose.
